# FASCICULE DE BREVET EUROPEEN

(11) **EP 0 748 314 B1**
(45) Date de publication et mention de la délivrance du brevet: **15.05.2002**
(21) Numéro de dépôt: 95910602.2
(22) Date de dépôt: 27.02.1995
(51) Int. Cl.: C07D 233/84, C07D 403/12, C07D 233/90, C07D 413/04, C07D 233/68, A61K 31/415

(54) **DERIVES TETRASUBSTITUES DE L'IMIDAZOLE, LEUR PROCEDE DE PREPARATION, LEUR APPLICATION A TITRE DE MEDICAMENTS ET LES COMPOSITIONS PHARMACEUTIQUES LES RENFERMANT**
TETRASUBSTITUIERTE IMIDAZOLDERIVATE, VERFAHREN ZU IHRER HERSTELLUNG, IHRE ANWENDUNG ALS MEDIKAMENTE UND DIESE ENTHALTENDE PHARMAZEUTISCHEN ZUSAMMENSETZUNGEN
TETRASUBSTITUTED IMIDAZOLE DERIVATIVES, METHOD FOR PREPARING THE SAME, USE THEREOF AS DRUGS, AND PHARMACEUTICAL COMPOSITIONS CONTAINING THE SAME

(30) Priorité: 04.03.1994 FR 9402517
(43) Date de publication de la demande: 18.12.1996
(73) Titulaire: Aventis Pharma S.A., 92160 Antony (FR)
(72) Inventeur: CORBIER, Alain, F-91370 Verrières-le-Buisson (FR); DEPREZ, Pierre, F-94320 Thiais (FR); FORTIN, Michel, F-75018 Paris (FR); GUILLAUME, Jacques, F-93190 Livry-Gargan (FR); HECKMANN, Bertrand, F-94230 Cachan (FR)
(86) Numéro de dépôt international: FR9500227
(87) Numéro de publication internationale: WO9523791

(56) Documents cités:
- EP-A- 0 503 162
- EP-A- 0 560 177
- EP-A- 0 577 023
- EP-A- 0 577 025
- WO-A-95/23792

## Description

La présente invention concerne de nouveaux dérivés tétrasubstitués de l'imidazole, leur procédé de préparation, les nouveaux intermédiaires obtenus, leur application à titre de médicaments et les compositions pharmaceutiques les renfermant.

Les documents EP-A-503162, EP-A-560177, EP-A-577025 et EP-A-577023 décrivent des dérivés de l'imidazole comme antagonistes de l'angiotensine II.

La présente invention a pour objet les produits de formule (I) : 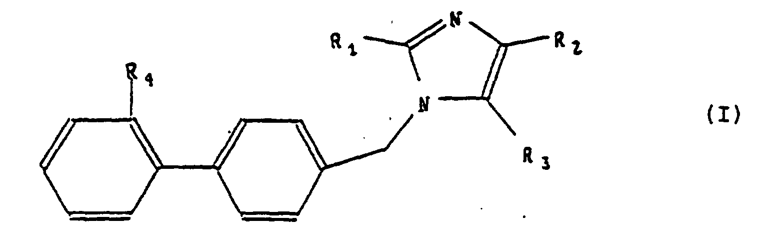 dans laquelle
R₁ représente un radical alkyle ou alkylthio, linéaire ou ramifié renfermant au plus 4 atomes de carbone ,
R₂ représente soit un radical alkylthio linéaire ou ramifié renfermant au plus 6 atomes de carbone et éventuellement substitué par un ou plusieurs atomes d'halogène, soit un radical 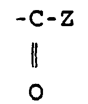 dans
lequel Z représente un radical carboxy libre, salifié par une base minérale ou organique ou estérifié par un radical alkyle linéaire ou ramifié renfermant au plus 6 atomes de carbone, R₃ représente le radical 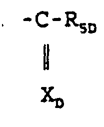 dans lequel
X_{D} représente un atome d'oxygène ou un radical 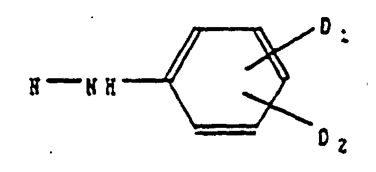 dans lequel D₁ et D₂, identiques ou différents, représentent un atome d'halogène ou un radical nitro,
et R_{5D} représente
i) le radical -CHY₁Y₂ dans lequel
   Y₁ représente un atome d'hydrogène ou d'halogène
   et Y₂ représente un radical carboxy libre, salifié par une base minérale ou organique, ou estérifié par un radical alkyle linéaire ou ramifié renfermant au plus 6 atomes de carbone ; les radicaux alkylthio, alkylsulfonyle et alkylsulfinyle, linéaires ou ramifiés renfermant au plus 6 atomes de carbone, ou les radicaux phénylthio, phénylsulfonyle ou phénylsulfinyle éventuellement substitués par un ou plusieurs atomes d'halogène ou radicaux alkyle ou alcoxy linéaires ou ramifiés renfermant au plus 6 atomes de carbone,
ii) le radical 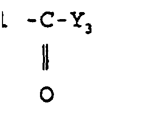 dans lequel Y₃ représente un radical hydroxyle, éventuellement salifié, alcoxy ou alkylthio, linéaire ou ramifié renfermant au plus 6 atomes de carbone,
iii) lorsque X_{D} représente un atome d'oxygène, R_{5D} représente un radical amino éventuellement substitué par un radical tétrazolyle ou par un ou deux radicaux alkyle, linéaires ou ramifiés renfermant au plus 6 atomes de carbone, eux-mêmes éventuellement substitués par un ou plusieurs radicaux phényle ou cyclohexyle,
   R₄ représente le radical -SO₂-NH₂,
   -SO₂-N=CH-N(CH₃)₂, 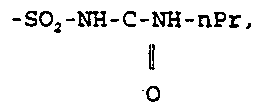 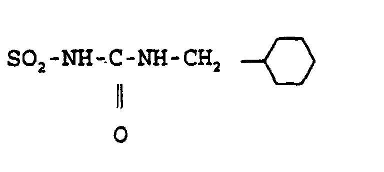 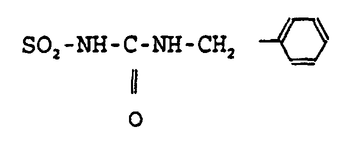 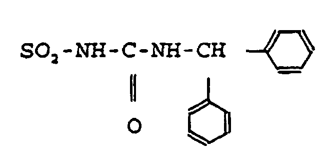 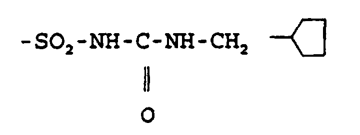 -SO₂-NH-CO₂Et, -SO₂-NH-CO₂H, SO₂-NH-CO₂Pr, 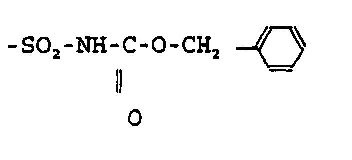 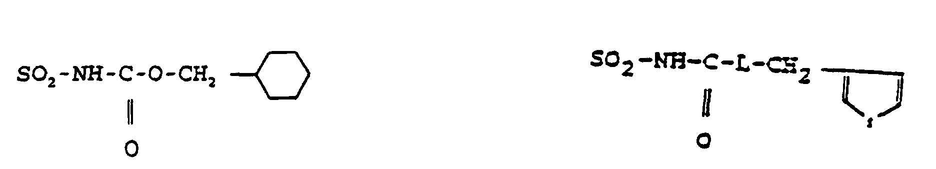 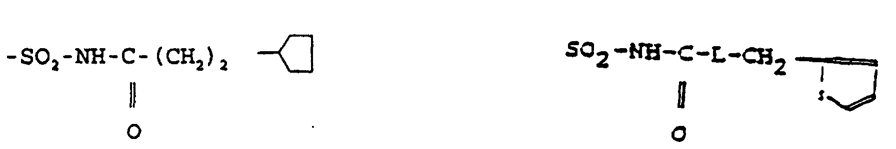 avec L représente -O- ou -NH-
   étant entendu que les atomes de soufre dans les produits de formule (I) peuvent éventuellement être oxydés sous forme de sulfone ou de sulfoxyde,
   lesdits produits de formule (I) étant sous toutes les formes isomères racémiques, énantiomères et diastéréoisomères possibles ainsi que les sels d'addition avec les acides minéraux et organiques ou avec les bases minérales et organiques desdits produits de formule (I).

Dans les produits de formule (I) et dans ce qui suit :
- le terme radical alkyle linéaire ou ramifié désigne de préférence les radicaux méthyle, éthyle, propyle, isopropyle, butyle, isobutyle, sec-butyle et tert-butyle mais peut également représenter un radical pentyle ou hexyle et particulièrement isopentyle et isohexyle,
- le terme atome d'halogène désigne de préférence l'atome de chlore, mais peut aussi représenter un atome de fluor, de brome ou d'iode,
- le terme radical alcoxy linéaire ou ramifié désigne de préférence les radicaux méthoxy, éthoxy, propoxy ou isopropoxy, mais peut aussi représenter un radical butoxy linéaire, secondaire ou tertiaire,
- le terme radical acyle désigne de préférence un radical ayant de 1 à 6 atomes de carbone tel que par exemple le radical formyle, acétyle, propionyle, butyryle ou benzoyle, mais également le radical pentanoyle, hexanoyle, acryloyle, crotonbyle ou carbamoyle,
- le terme radical acyloxy désigne par exemple un radical
dans lequel le radical acyle a les valeurs indiquées ci-dessus et désigne de préférence un radical formyloxy, acétyloxy, propionyloxy, butyryloxy ou benzoyloxy,
- le terme radical cycloalkyle désigne de préférence les radicaux cyclopropyle, cyclobutyle et tout particulièrement les radicaux cyclopentyle et cyclohexyle,
- le terme radical alkylthio désigne de préférence les radicaux dans lesquels le radical alkyle est tel que défini ci-dessus comme par exemple dans méthylthio ou éthylthio,
- le terme radical haloalkylthio désigne de préférence les radicaux dans lesquels le radical alkyle est tel que défini ci-dessus et est substitué par un ou plusieurs atomes d'halogène tel que défini ci-dessus comme par exemple dans bromoéthylthio, trifluorométhylthio, trifluoroéthylthio ou encore pentafluoroéthylthio,
- le terme radical haloalcoxy désigne de préférence les radicaux dans lesquels le radical alcoxy est tel que défini ci-dessus et est substitué par un ou plusieurs atomes d'halogène tel que défini ci-dessus comme par exemple dans bromoéthoxy, trifluorométhoxy, trifluoroéthoxy ou encore pentafluoroéthoxy.

Dans tous les radicaux que peuvent représenter R₁, R₂, R₃ et R₄, tels que définis ci-dessus, les atomes de soufre peuvent ne pas être oxydés comme dans les radicaux alkylthio, ou au contraire être oxydés pour donner les radicaux alkylsulfinyle, ou alkylsulfonyle :
- les termes radical alkylthio, alkylsulfinyle et alkylsulfonyle désignent les radicaux dans lesquels le radical alkyle linéaire ou ramifié peut représenter, par exemple, les valeurs indiquées ci-dessus pour le radical alkyle ; ces radicaux représentent ainsi de préférence les radicaux méthylthio, hydroxyméthylthio, éthylthio, aminoéthylthio, méthylsulfinyle, éthylsulfinyle, méthylsulfonyle, éthylsulfonyle mais peut aussi représenter un radical propylthio, isopropylthio, butylthio, sec-butylthio, tert-butylthio, isopentylthio ou isohexylthio ou ces radicaux dans lesquels le radical thio est oxydé en radical sulfinyle ou sulfonyle.

Parmi les substituants des radicaux alkylthio et alcoxy, éventuellement oxydés, on peut citer par exemple les radicaux hydroxyle, alcoxy, carboxy libre, salifié ou estérifié, acyle, acyloxy, alkyle, phényle, les atomes d'halogène.

Les radicaux carbamoyle et amino que peuvent représenter ou porter l'un ou plusieurs des éventuels substituants des radicaux définis dans les produits de formule
(I) et dans ce qui suit, désignent des radicaux dans lesquels à l'atome d'azote sont liés deux radicaux, identiques ou différents, choisis parmi l'atome d'hydrogène pour donner le radical amino ; les radicaux alkyle tels que définis ci-dessus pour donner les radicaux monoalkyl- ou dialkylamino dans lesquels les radicaux alkyles linéaires ou ramifiés renferment de 1 à 6 atomes de carbone, tous ces radicaux étant éventuellement substitués ainsi qu'il est indiqué ci-dessus et ci-après.

Les radicaux carbamoyle substitué et amino substitué désignent respectivement les radicaux dans lesquels l'atome d'azote peut être substitué par un ou deux radicaux choisis parmi les radicaux tels que définis précédemment notamment le ou les radicaux alkyle choisis parmi les radicaux alkyle tels que définis ci-dessus comme par exemple pour monoalkylamino dans méthylamino, éthylamino ou isopropylamino ou par exemple pour dialkylamino dans diméthylamino, diéthylamino ou encore méthyléthylamino, ces radicaux alkyles étant éventuellement substitués ainsi qu'il est indiqué ci-dessus, comme par exemple les radicaux méthoxyméthyle, méthoxyéthyle, éthoxyéthyle.

A titre d'exemple et de façon non exhaustive, le terme radical carbamoyle désigne les radicaux carbamoyle substitué sur l'atome d'azote par un ou deux radicaux alkyle éventuellement substitués ainsi qu'il est défini ci-dessus, pour former notamment un groupe N-monoalkyl carbamoyle tel que N-méthylcarbamoyle, N-éthylcarbamoyle ou un groupe N,N-dialkyl carbamoyle, tel que N,N-diméthylcarbamoyle, N,N-diéthylcarbamoyle ; un groupe N-(hydroxyalkyl) carbamoyle, tel que N-(hydroxyméthyl) carbamoyle, N-(hydroxyéthyl) carbamoyle, phénylcarbamoyle ; pyridylcarbamoyle ; benzylcarbamoyle ; N-méthyl N-phénylcarbamoyle ; pyridylméthylcarbamoyle. Par ailleurs, parmi les radicaux alkyles substitués, on peut citer également les radicaux alkyles substitués par un radical carbamoyle tel que défini ci-dessus, pour former un groupe carbamoylalkyle tel que carbamoylméthyle ou carbamoyléthyle.

Le radical amino peut être un radical alcoxycarbonylamino, ce radical étant alors de préférence le radical tert-butyloxycarbonylamino ou le radical benzyloxycarbonylamino.

Les radicaux amino et carbamoyle peuvent encore notamment être substitués par un ou deux acides aminés choisis parmi les 20 acides aminés naturels tels que notamment la proline ou par exemple la glycine, l'alanine, la leucine, l'isoleucine, la valine ou la phénylalanine ou l'un des autres acides aminés naturels connus de l'homme de métier.

Le ou les radicaux carboxy des produits de formule (I) peuvent être salifiés ou estérifiés par les groupements divers connus de l'homme du métier parmi lesquels on peut citer, par exemple :
- parmi les composés de salification, des bases minérales telles que, par exemple, un équivalent de sodium, de potassium, de lithium, de calcium, de magnésium ou d'ammonium ou des bases organiques telles que, par exemple, la méthylamine, la propylamine, la triméthylamine, la diéthylamine, la triéthylamine, la N,N-diméthyléthanolamine, le tris (hydroxyméthyl) amino méthane, l'éthanolamine, la pyridine, la picoline, la dicyclohexylamine, la morpholine, la benzylamine, la procaïne, la lysine, l'arginine, l'histidine, la N-méthylglucamine,
- parmi les composés d'estérification, les radicaux alkyle pour former des groupes alcoxy carbonyle tel que, par exemple, méthoxycarbonyle, éthoxycarbonyle, tert-butoxycarbonyle ou benzyloxycarbonyle, ces radicaux alkyles pouvant être substitués par des radicaux choisis par exemple parmi les atomes d'halogène, les radicaux hydroxyle, alcoxy, acyle, acyloxy, alkylthio, amino ou aryle comme, par exemple, dans les groupements chlorométhyle, hydroxypropyle, méthoxyméthyle, propionyloxyméthyle, méthylthiométhyle, diméthylaminoéthyle, benzyle ou phénéthyle.

Les sels d'addition avec les acides minéraux ou organiques des produits de formule (I) peuvent être, par exemple, les sels formés avec les acides chlorhydrique, bromhydrique, iodhydrique, nitrique, sulfurique, phosphorique, propionique, acétique, formique, benzoïque, maléique, fumarique, succinique, tartrique, citrique, oxalique, glyoxylique, aspartique, ascorbique, les acides alcoylmonosulfoniques tels que par exemple l'acide méthanesulfonique, l'acide éthanesulfonique, l'acide propanesulfonique, les acides alcoyldisulfoniques tels que par exemple l'acide méthanedisulfonique, l'acide alpha, bêta-éthanedisulfonique, les acides arylmonosulfoniques tels que l'acide benzènesulfonique et les acides aryldisulfoniques.

Parmi les valeurs que peut prendre R₄ tel que défini ci-dessus, on peut citer par exemple tout particulièrement et de façon non exhaustive les radicaux :
-SO₂-NH₂,
-SO₂-N=CH-N(CH₃)₂, 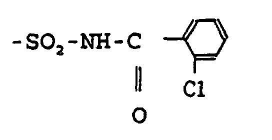 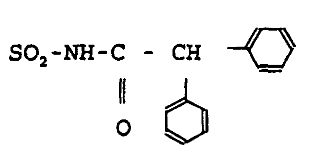 -SO₂-NH-CO₂Et, -SO₂-NH-CO₂Pr, -SO₂-NH-CO₂Bu, -SO₂-NH-CO₂H, 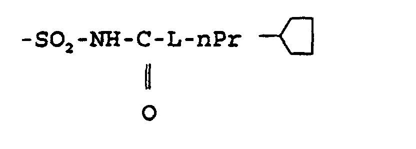 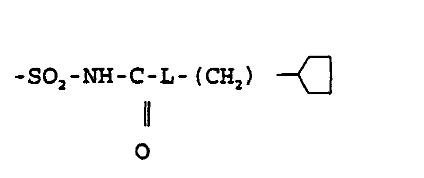 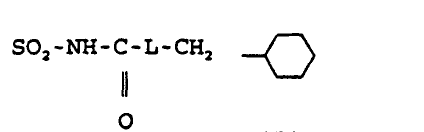 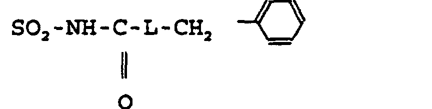 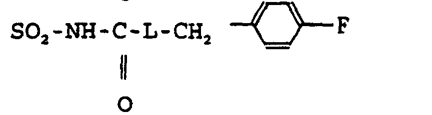 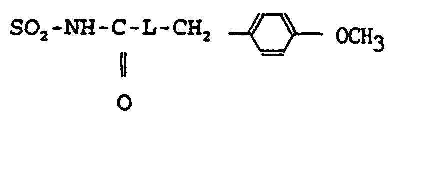 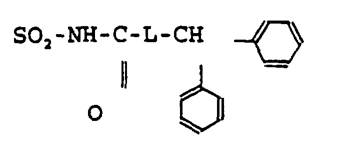 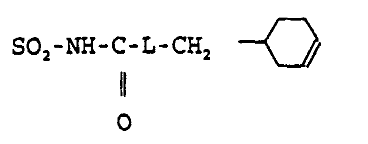 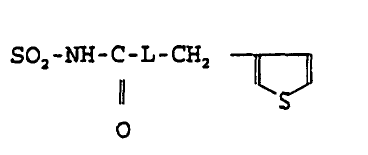 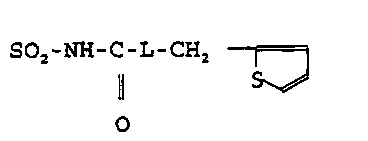 avec L représente -O- ou -NH- ,
étant entendu que les atomes de soufre dans les produits de formule (I) peuvent éventuellement être oxydés sous forme de sulfone ou de sulfoxyde, lesdits produits de formule (I) étant sous toutes les formes isomères racémiques, énantiomères et diastéréoisomères possibles ainsi que les sels d'addition avec les acides minéraux et organiques ou avec les bases minérales et organiques desdits produits de formule (I).

Parmi les produits objet de l'invention, peuvent être cités tout particulièrement les produits de formule (I) répondant aux formules suivantes :
- acide 2-butyl 1-((2'-(((((cyclohexylméthyl) amino) carbonyl) amino) sulfonyl) (1,1'-biphényl) 4-yl) méthyl) 4-méthylthio alpha-oxo-1H-imidazole 5-acétique,
- 2-butyl 4-(méthylthio) béta-oxo-1-((2'-(((((propylamino) carbonyl) amino) sulfonyl) (1,1'-biphényl) 4-yl) méthyl) 1H-imidazole 5-propanoate d'éthyle,
- 2-butyl 4-(méthylthio) 1-((2'-(((((propylamino) carbonyl) amino) sulfonyl) (1,1'-biphényl) 4-yl) méthyl) N-(1H-tétrazol-5-yl) 1H-imidazole 5-carboxamide,
- acide 2-butyl 4-(méthylthio) alpha-oxo-1-((2'-(((((phénylméthyl) amino) carbonyl) amino) sulfonyl) (1,1'-biphényl) 4-yl) méthyl) 1H-imidazole 5-acétique,
- 4'-((2-butyl 5-(2-(méthylsulfinyl) acétyl) 4-(méthylthio) 1H-imidazol-1-yl) méthyl) N-(((phénylméthyl) amino) carbonyl) (1,1'-biphényl) 2-sulfonamide,
- 4-(méthylthio) 1-((2'-(((((phénylméthyl) amino) carbonyl) amino) sulfonyl) (1,1'-biphényl) 4-yl) méthyl) 2-propyl-N-(2H-tétrazol 5-yl) 1H-imidazole 5-carboxamide,
- 4'-((4-(méthylthio) 5-(2-(phénylsulfinyl) acétyl) 2-propyl 1H-imidazol-1-yl) méthyl N-(((phénylméthyl) amino) carbonyl) (1,1'-biphényl) 2-sulfonamide,
- 2-butyl 4-(méthylthio) beta-oxo 1-((2'-(((((phénylméthyl) amino) carbonyl) amino) sulfonyl) (1,1'-biphényl) 4-yl) méthyl) 1H-imidazol-5-propanoate d'éthyle,
- 4'-((2-butyl 5-(2-((4-fluorophényl) sulfonyl) acétyl) 4-(méthylthio) 1H-imidazol 1-yl) méthyl) N-(((phénylméthyl) amino) carbonyl) (1,1'-biphényl) 2-sulfonamide,
- 4'-((4-(méthylthio) 5-((phénylsulfinyl) acétyl) 2-propyl 1H-imidazol-1-yl) méthyl) N-(((2-thiénylméthyl) amino) carbonyl) (1,1'-biphényl) 2-sulfonamide,
- (±) N-(((cyclopentylméthyl) amino) carbonyl) 4'-((4-(méthylthio) 5-((phénylsulfinyl) acétyl) 2-propyl 1H-imidazol-1-yl) méthyl) (1,1'-biphényl) 2-sulfonamide,
- (±) N-(4'-((4-(méthylthio) 5-((phénylsulfinyl) acétyl) 2-propyl 1H-imidazol 1-yl) méthyl) (1,1'-biphényl-2-yl) sulfonyl) cyclopentanepropanamide.

L'invention a également pour objet un procédé de préparation des produits de formule (I), telle que définie ci-dessus, caractérisé en ce que :
soit l'on soumet un composé de formule (II): 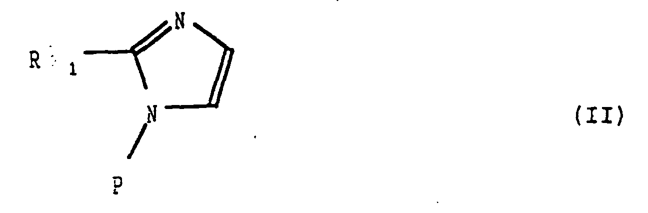 dans laquelle R₁ a la signification indiquée ci-dessus et P représente un groupement protecteur de l'atome d'azote, à une réaction d'halogénation pour obtenir un composé de formule (III) : 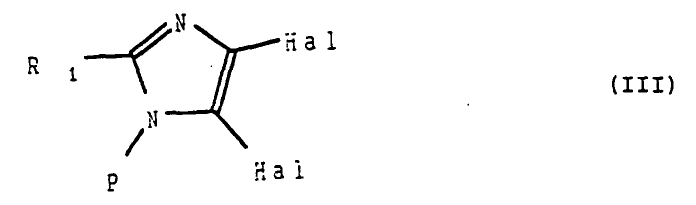 dans laquelle R₁ et P ont les significations indiquées ci-dessus et Hal représente un atome d'halogène, que l'on soumet à une réaction d'échange halogène-métal sur l'un des atomes d'halogène puis à une réaction avec un composé de formule (IV_{c}), (IV_{d}) ou (IVₑ) : 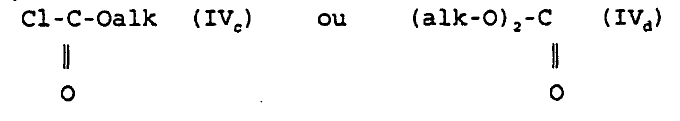 ou 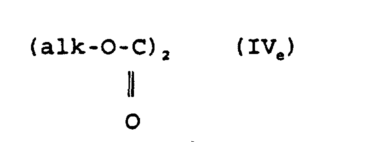 dans lesquelles alk représente un radical alkyle renfermant au plus 4 atomes de carbone,
pour obtenir le composé de formule (V) : 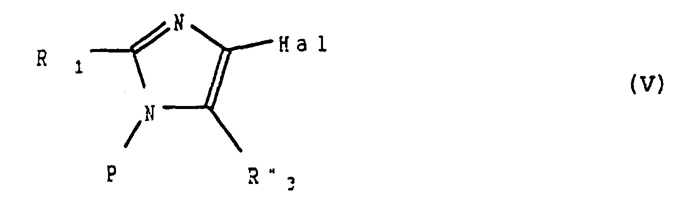 dans laquelle R ₁, P et Hal ont les significations indiquées ci-dessus et R"₃ représente K-O-alk avec alk tel que défini ci-dessus et K représente le radical 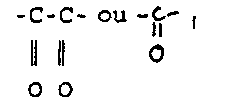 composé de formule (V) que l'on peut soumettre à une réaction d'échange halogène-métal sur l'atome d'halogène puis à une réaction avec un composé de formule (IV'ₐ), (IV'_{b}), (IV'_{c}), (IV'_{d}) ou (IV'ₑ) :

(-S-R")₂ (IV'ₐ) ou MeSO₂SR" (IV'_{b})

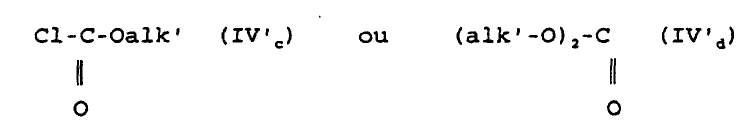

ou 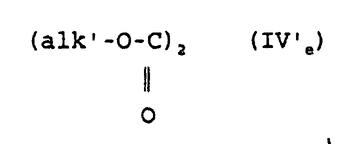 dans lesquelles R" représente un radical alkyle linéaire au ramifié renfermant au plus 6 atomes de carbone et alk' identique ou différent de alk, représente un radical alkyle renfermant au plus 4 atomes de carbone, pour obtenir le composé de formule (VII) : 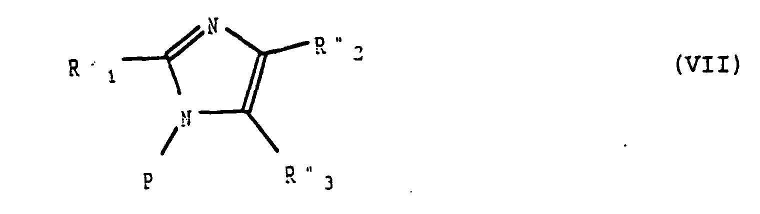 dans laquelle R₁, R"₃ et P ont les significations indiquées ci-dessus, et R"₂ représente -S-R" ou -K-Oalk' dans lesquelles R", alk' et K ont les significations indiquées ci-dessus,
produit de formule (VII) dont on libère la fonction amine bloquée par P tel que défini ci-dessus, puis fait réagir avec un composé de formule (VIII) : 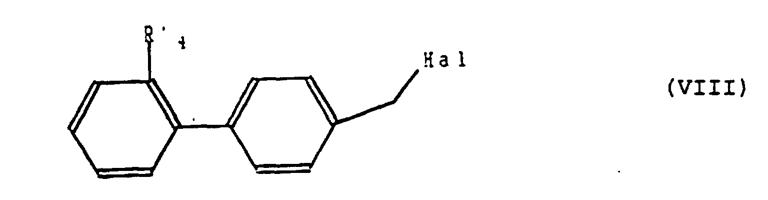 dans laquelle R'₄ a la signification indiquée ci-dessus pour R₄, dans laquelle les éventuelles fonctions réactives sont éventuellement protégées par des groupements protecteurs et Hal représente un atome d'halogène pour obtenir un produit de formule (I₁) : 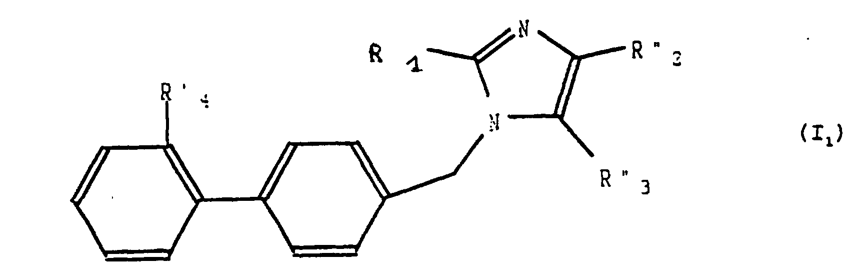 dans laquelle R₁, R"₂, R"₃ et R'₄ ont les significations indiquées ci-dessus,
soit l'on soumet un composé de formule (IX) : 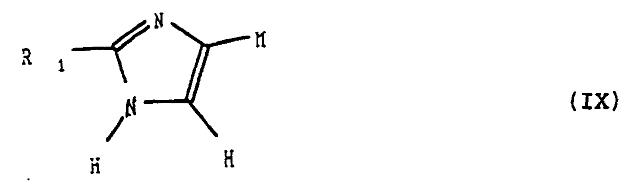 dans laquelle R₁ a la signification indiquée ci-dessus et M représente un atome d'hydrogène ou le radical R'₂ qui a la signification indiquée ci-dessus pour R₂, dans laquelle les éventuelles fonctions réactives sont éventuellement protégées par des groupements protecteurs,
à une réaction avec le composé de formule (VIII) telle que définie ci-dessus, pour obtenir un produit de formule (X) : 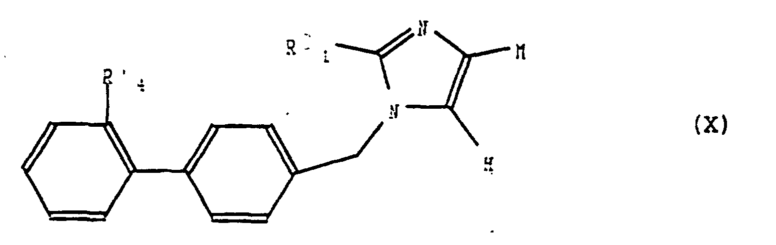 dans laquelle R₁, M et R'₄ ont les significations indiquées ci-dessus,
produit de formule (X) que lorsque M représente un atome d'hydrogène, l'on peut soumettre à une réaction d'halogénation pour obbtenir le produit de formule (XIV) : 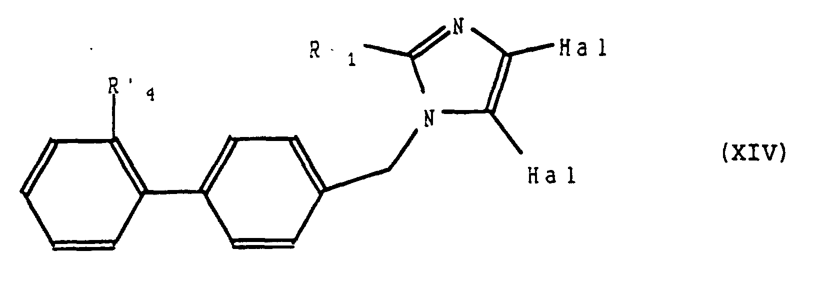 dans laquelle R ₁, R'₄ et Hal ont les significations indiquées ci-dessus, que l'on peut soumettre à une réaction d'échange halogéne métal puis à l'action d'un composé de formule (IV_{c}), (IV_{d}) ou (IVₑ) telle que définie ci-dessus pour obtenir le produit de formule (XXI) : 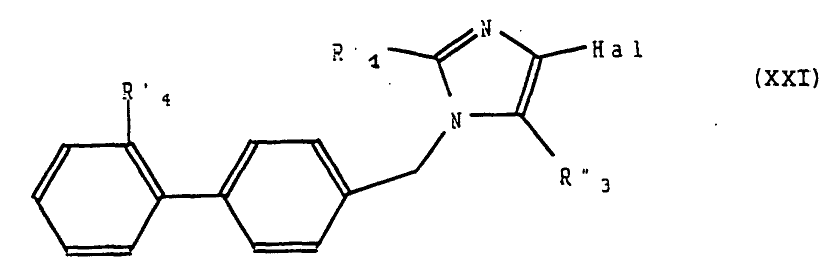 dans laquelle R₁, R'₄, Hal et R"₃ ont les significations indiquées ci-dessus, produit de formule (I₇) que l'on peut soumettre à une réaction d'échange halogène métal puis à l'action d'un composé de formule (IV'ₐ), (IV'_{b}), (IV'_{c}), (IV'_{d}) ou (IV'ₑ), telle que définie ci-dessus, pour obtenir un produit de formule (I) : 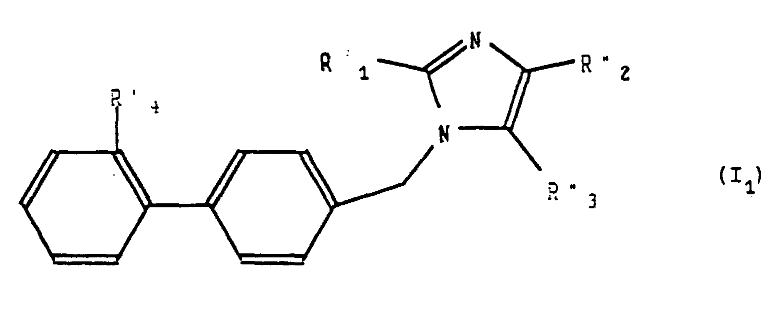 dans laquelle R₁, R'₄, R"₂ et R"₃ ont les significations indiquées ci-dessus,
soit l'on soumet un composé de formule (XX) : 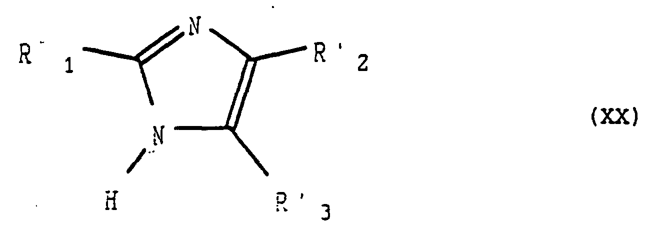 dans laquelle R₁ et R'₂ ont les significations indiquées ci-dessus et R'₃ a la signification indiquée ci-dessus pour R₃ dans laquelle les éventuelles fonctions réactives sont éventuellement protégées par des groupements protecteurs, à une réaction avec le composé de formule (VIII) telle que définie ci-dessus, pour obtenir un produit de formule (I') : 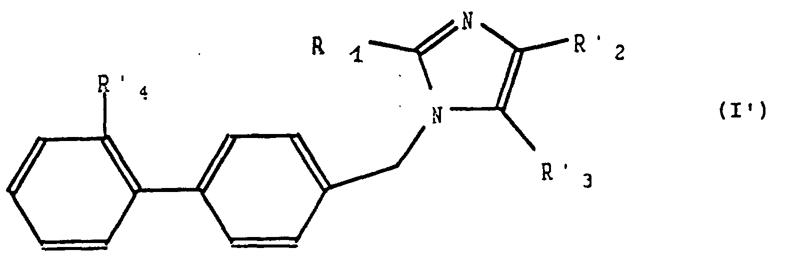 dans laquelle R₁, R'₂, R'₃ et R'₄ ont les significations indiquées ci-dessus,
produits de formules (I₁) et (I') qui peuvent être des produits de formule (I) et que, pour obtenir des ou d'autres produits de formule (I), l'on peut soumettre, si désiré et si nécessaire, à l'une ou plusieurs des réactions de transformations suivantes, dans un ordre quelconque :
a) estérification de fonction acide,
b) saponification de fonction ester en fonction acide,
c) transformation de fonction ester en fonction acyle,
d) transformation de la fonction cyano en fonction acide,
e) transformation de fonction acide en fonction amide,
f) réduction de la fonction carboxy en fonction alcool,
g) transformation de fonction alcoxy en fonction hydroxyle, ou encore de fonction hydroxyle en fonction alcoxy,
h) oxydation de fonction alcool en fonction aldéhyde, acide ou cétone,
i) transformation du radical formyle en radical carbamoyle,
j) transformation du radical carbamoyle en radical nitrile,
k) transformation de radical nitrile en tétrazolyle,
l) oxydation de groupement alkylthio ou arylthio en sulfoxyde ou sulfone correspondant,
r) transformation d'un carbamate en urée et notamment d'un sulfonylcarbamate en sulfonylurée,
s) élimination des groupements protecteurs que peuvent porter les fonctions réactives protégées,
t) salification par un acide minéral ou organique ou par une base pour obtenir le sel correspondant,
u) dédoublement des formes racémiques en produits dédoublés, lesdits produits de formule (I) ainsi obtenus étant sous toutes les formes isomères possibles racémiques, énantiomères et diastéréoisomères.

Dans des conditions préférentielles de mise en oeuvre de l'invention, la réaction d'halogénation des composés de formule (II) et (X) telles que définies ci-dessus respectivement en composés de formule (III) et (XIV) telles que définies ci-dessus, peut être réalisée dans les conditions usuelles de l'homme du métier et notamment par bromation à l'aide de N-bromosuccinimide dans le dichlorométhane ou encore de brome dans l'acide acétique.

L'obtention du composé de formule (V) correspondant peut être réalisée par réaction du composé de formule (III) telle que définie ci-dessus avec un composé organo métallique tel que le n-butyllithium dans un solvant tel que le tétrahydrofuranne à une température d'environ -78°C suivie de l'action d'un composé de formule (IV_{c}), (IV_{d}) ou (IVₑ).

La réaction du produit de formule (V) telle que définie ci-dessus avec le composé, de formule (IV'ₐ), (IV'_{b}) ou (IV'_{c}) (IV'_{d}) ou (IV'ₑ), telle que définie ci-dessus, pour obtenir un composé de formule (VII) telle que définie ci-dessus peut être réalisée d'une manière identique en utilisant le n-butyllithium comme agent de métallation.

La fonction amine du composé de formule (VII) telle que définie ci-dessus, protégée par P tel que défini ci-dessus, peut être libérée dans les conditions usuelles connues de l'homme du métier et notamment lorsque P représente le radical -CH₂-O-(CH₂)₂-Si(CH₃)₃, par action de l'acide trifluoroacétique ou encore en présence d'ion fluorure.

Dans le produit de formule (VIII), Hal représente de préférence un atome de brome mais peut également représenter un atome de chlore ou d'iode.

La réaction du produit de formule (VIII) sur le produit de formule (VII) ou (IX) ou (XX), peut être réalisée dans un solvant tel que par exemple le diméthylformamide ou encore le diméthylacétamide, le tétrahydrofuranne, le diméthoxyéthane ou le diméthylsulfoxyde au reflux du solvant ou à la température ambiante, de préférence sous agitation ; la réaction est réalisée de préférence en présence d'une base telle que par exemple l'hydrure de sodium ou de potassium ou encore du carbonate de sodium ou de potassium, du méthylate ou éthylate ou tert-butylate de sodium ou de potassium.

La réaction de transformation du produit de formule (XIV) telle que définie ci-dessus, en produit de formule (XXI) telle que définie ci-dessus puis en produit de formule (I₁), peut être réalisée dans les mêmes conditions que celles définies pour l'obtention des produits de formules (V) et (VII) telles que définies ci-dessus, à partir du produit de formule (III) telle que définie ci-dessus.

Selon les valeurs de R₁, R'₂, R"₂, R'₃, R"₃ et R'₄, les produits de formules (I₁) et (I') constituent ou non des produits de formule (I) et peuvent donner des produits de formule (I), ou être transformés en d'autres produits de formule (I) en étant soumis à une ou plusieurs des réactions a) à u) indiquées ci-dessus.

Ainsi les diverses fonctions réactives que peuvent porter certains composés des réactions définies ci-dessus peuvent, si nécessaire, être protégées : il s'agit par exemple des radicaux hydroxyle, acyle, carboxy libres ou encore amino et monoalkylamino qui peuvent être protégés par les groupements protecteurs appropriés.

La liste suivante, non exhaustive, d'exemples de protection de fonctions réactives peut être citée :
- les groupements hydroxyle peuvent être protégés par exemple par les radicaux alkyle tels que tert-butyle, triméthylsilyle, tert-butyldiméthylsilyle, méthoxyméthyle, tétrahydropyrannyle, benzyle ou acétyle,
- les groupements amino peuvent être protégés par exemple par les radicaux acétyle, trityle, benzyle, tert-butoxycarbonyle, phtalimido ou d'autres radicaux connus dans la chimie des peptides,
- les groupements acyles tel que le groupement formyle peuvent être protégés par exemple sous forme de cétals ou de thiocétals cycliques ou non cycliques tels que le diméthyl ou diéthylcétal ou l'éthylène dioxycétal, ou le diéthylthiocétal ou l'éthylènedithiocétal,
- les fonctions acide des produits décrits ci-dessus peuvent être, si désiré, amidifiées par une amine primaire ou secondaire par exemple dans du chlorure de méthylène en présence, par exemple, de chlorhydrate de 1-éthyl-3-(diméthylaminopropyl) carbodiimide à la température ambiante :
- les fonctions acide peuvent être protégées par exemple sous forme d'esters formés avec les esters facilement clivables tels que les esters benzyliques ou ter butyliques ou des esters connus dans la chimie des peptides.

Les réactions auxquelles les produits de formules (I₁) et (I'), telles que définies ci-dessus peuvent être soumis, si désiré ou si nécessaire, peuvent être réalisées, par exemple, comme indiqué ci-après.
a) Les produits décrits ci-dessus peuvent, si désiré, faire l'objet, sur les éventuelles fonctions carboxy, de réactions d'estérification qui peuvent être réalisées selon les méthodes usuelles connues de l'homme du métier.
b) Les éventuelles transformations de fonctions ester en fonction acide des produits décrits ci-dessus peuvent être, si désiré, réalisées dans les conditions usuelles connues de l'homme du métier notamment par hydrolyse ou alcaline par exemple par de la soude ou de la potasse en milieu alcoolique tel que, par exemple, dans du méthanol ou encore par hydrolyse acide au moyen d'acide chlorhydrique ou sulfurique.
c) la ruction d'addition sur la fonction ester 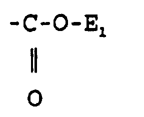 dans laquelle E₁ peut représenter un radical alkyle ou aryle éventuellement substitué et éventuellement protégé en fonction acyle 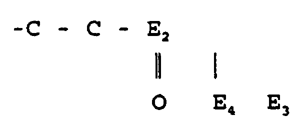 peut être réalisée notamment par action de l'anion carboné 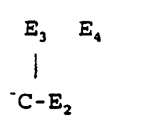 dans lequel E₂, E₃ et E₄, identiques ou différents sont choisis parmi l'atome d'hydrogène, les radicaux alkyl, alkylthioaryle, alkylsulfoxyde, arylsulfoxyde, alkylsulfone, arylsulfone, acyle, carboxy libre, salifié, estérifié ou amidifié, les radicaux alkyle, alkylthio et aryle étant éventuellement substitués et éventuellement protégés ainsi qu'il est indiqué ci-dessus.
   Une telle réaction est réalisée notamment ainsi qu'il est décrit dans la partie expérimentale, ou selon les méthodes usuelles connues de l'homme du métier.
d) Les éventuelles fonctions cyano des produits décrits ci-dessus peuvent être, si désiré, transformées en fonction acide dans les conditions usuelles connues de l'homme du métier par exemple par une double hydrolyse réalisée en milieu acide tel que par exemple dans un mélange d'acide sulfurique, d'acide acétique glacial et d'eau, ces trois composés étant de préférence en proportions égales, ou encore dans un mélange de soude, d'éthanol et d'eau au reflux.
e) la réaction de transformation de fonction acide en fonction amide peut notamment être réalisée par formation d'abord d'un chlorure d'acide selon les conditions usuelles connues de l'homme du métier et par exemple par action de SOCl₂ puis amidification comme indiqué ci-dessus, ou encore par amidification directe de l'acide ci-dessus.
   Notamment on peut obtenir le radical 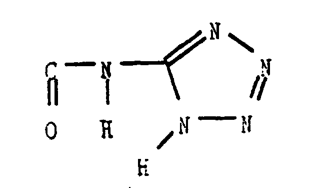 en transformant la fonction acide en chlorure d'acide, notamment par action de SOCl₂ dans un solvant tel que par exemple le toluène, ou le benzène,
   puis en faisant agir l'amine 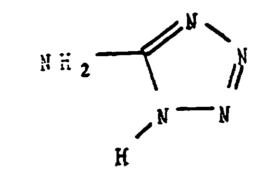
f) Les éventuelles fonctions carboxy libre ou estérifié des produits décrits ci-dessus peuvent être, si désiré, réduites en fonction alcool par les méthodes connues de l'homme de métier : les éventuelles fonctions carboxy estérifié peuvent être, si désiré, réduites en fonction alcool par les méthodes connues de l'homme du métier et notamment par de l'hydrure de lithium et d'aluminium dans un solvant tel que par exemple le tétrahydrofuranne ou encore le dioxane ou l'éther éthylique.
   Les éventuelles fonctions carboxy libre des produits décrits ci-dessus peuvent être, si désiré, réduites en fonction alcool notamment par de l'hydrure de bore.
g) Les éventuelles fonctions alcoxy telles que notamment méthoxy des produits décrits ci-dessus peuvent être, si désiré, transformées en fonction hydroxyle dans les conditions usuelles connues de l'homme du métier par exemple par du tribromure de bore dans un solvant tel que par exemple le chlorure de méthylène, par du bromhydrate ou chlorhydrate de pyridine ou encore par de l'acide bromhydrique ou chlorhydrique dans de l'eau ou de l'acide trifluoro acétique au reflux.
h) Les éventuelles fonctions alcool des produits décrits ci-dessus peuvent être, si désiré, transformées en fonction aldéhyde ou acide par oxydation dans les conditions usuelles connues de l'homme du métier telles que par exemple par action de l'oxyde de manganèse pour obtenir les aldéhydes ou du réactif de Jones pour accéder aux acides.
i) j) Les réactions de transformation de radical formyle en radical carbamoyle et de radical carbamoyle en radical nitrile, sont réalisées notamment pour R₃ et R₄ selon les conditions usuelles connues de l'homme du métier, telles que par exemple passage par le céto nitrile et déplacement par une amine (Chem. Comm. 1971, p. 733).
k) Les éventuelles fonctions nitrile des produits décrits ci-dessus peuvent être, si désiré, transformées en tétrazolyle dans les conditions usuelles connues de l'homme du métier telles que par exemple par cycloaddition d'un azidure métallique tel que par exemple l'azidure de sodium ou un azidure de trialkylétain sur la fonction nitrile ainsi qu'il est indiqué dans la méthode décrite dans l'article référencé comme suit : J. Organometallic Chemistry., 33, 337 (1971) KOZIMA S.& coll.
l) Les éventuels groupements alkylthio ou arylthio des produits décrits ci-dessus peuvent être, si désiré, transformés en les fonctions sulfoxyde ou sulfone correspondantes dans les conditions usuelles connues de l'homme du métier telles que par exemple par les peracides comme par exemple l'acide peracétique ou l'acide métachloroperbenzoïque ou encore par l'ozone, l'oxone, le périodate de sodium dans un solvant tel que par exemple le chlorure de méthylène ou le dioxanne à la température ambiante.
   L'obtention de la fonction sulfoxyde peut être favorisée par un mélange équimolaire du produit renfermant un groupement alkylthio ou arylthio et du réactif tel que notamment un peracide.
   L'obtention de la fonction sulfone peut être favorisée par un mélange du produit renfermant un groupement alkylthio ou arylthio avec un excès du réactif tel que notamment un peracide.
r) la réaction de transformation d'un carbamate en urée et notamment d'un sulfonylcarbamate en sulfonylurée, peut être réalisée par exemple au reflux d'un solvant comme par exemple le toluène en présence de l'amine adéquate.
s) L'élimination de groupements protecteurs tels que par exemple ceux indiqués ci-dessus peut être effectuée dans les conditions usuelles connues de l'homme de métier notamment par une hydrolyse acide effectuée avec un acide tel que l'acide chlorhydrique, benzène sulfonique ou para-toluène sulfonique, formique ou trifluoroacétique ou encore par une hydrogénation catalytique.
   Le groupement phtalimido peut être éliminé par l'hydrazine.
   On trouvera une liste de différents groupements protecteurs utilisables par exemple dans le brevet BF 2 499 995.
t) Les produits décrits ci-dessus peuvent, si désiré, faire l'objet de réactions de salification par exemple par un acide minéral ou organique ou par une base minérale ou organique selon les méthodes usuelles connues de l'homme du métier.
u) Les éventuelles formes optiquement actives des produits décrits ci-dessus peuvent être préparées par dédoublement des racémiques selon les méthodes usuelles connues de l'homme du métier.

Des illustrations de telles réactions définies ci-dessus sont données dans la préparation des exemples décrits ci-après.

Les composés de formule (I) tels que définis ci-dessus ainsi que leurs sels d'addition avec les acides présentent d'intéressantes propriétés pharmacologiques.

Les produits de formule (I) tels que définis ci-dessus, sont doués de propriétés antagonistes pour les récepteurs à l'angiotensine II et sont ainsi notamment inhibiteurs des effets de l'angiotensine II, en particulier de l'effet vasoconstricteur et également de l'effet trophique au niveau des myocytes.

Ces propriétés justifient leur application en thérapeutique et l'invention a également pour objet à titre de médicaments, les produits tels que définis par la formule (I) ci-dessus, lesdits produits de formule (I) étant sous toutes les formes isoméres possibles racémiques ou optiquement actives, ainsi que les sels d'addition avec les acides minéraux ou organiques pharmaceutiquement acceptables desdits produits de formule (I).

L'invention a plus particulièrement pour objet, à titre de médicaments, les produits décrits ci-aprés dans les exemples et notamment les produits de formule (I) suivants :
- acide 2-butyl 1-((2'-(((((cyclohexylméthyl) amino) carbonyl) amino) sulfonyl) (1,1'-biphényl) 4-yl) méthyl) 4-méthylthio alpha-oxo-1H-imidazole 5-acétique,
- 2-butyl 4-(méthylthio) béta-oxo-1-((2'-(((((propylamino) carbonyl) amino) sulfonyl) (1,1'-biphényl) 4-yl) méthyl) 1H-imidazole 5-propanoate d'éthyle,
- 2-butyl 4-(méthylthio) 1-((2'-(((((propylamino) carbonyl) amino) sulfonyl) (1,1'-biphényl) 4-yl) méthyl) N-(1H-tétrazol-5-yl) 1H-imidazole 5-carboxamide,
- acide 2-butyl 4-(méthylthio) alpha-oxo-1'((2'-(((((phénylméthyl) amino) carbonyl) amino) sulfonyl) (1,1'-biphényl) 4-yl) méthyl) 1H-imidazole 5-acétique,
- 4'-((2-butyl 5-(2-(méthylsulfinyl) acétyl) 4-(méthylthio) 1H-imidazol-1-yl) méthyl) N-(((phénylméthyl) amino) carbonyl) (1,1'-biphényl) 2-sulfonamide,
- 4-(méthylthio) 1-((2'-(((((phénylméthyl) amino) carbonyl) amino) sulfonyl) (1,1'-biphényl) 4-yl) méthyl) 2-propyl-N-(2H-tétrazol 5-yl) 1H-imidazole 5-carboxamide,
- 4'-((4-(méthylthio) 5-(2-(phénylsulfinyl) acétyl) 2-propyl 1H-imidazol-1-yl) méthyl N-(((phénylméthyl) amino) carbonyl) (1,1'-biphényl) 2-sulfonamide,
- 2-butyl 4-(méthylthio) beta-oxo 1-((2'-(((((phénylméthyl) amino) carbonyl) amino) sulfonyl) (1,1'-biphényl) 4-yl) méthyl) 1H-imidazol-5-propanoate d'éthyle,
- 4'-((2-butyl 5-(2-((4-fluorophényl) sulfonyl) acétyl) 4-(méthylthio) 1H-imidazol 1-yl) méthyl) N-(((phénylméthyl) amino) carbonyl) (1,1'-biphényl) 2-sulfonamide,
- 4'-((4-(méthylthio) 5-((phénylsulfinyl) acétyl) 2-propyl 1H-imidazol-1-yl) méthyl) N-(((2-thiénylméthyl) amino) carbonyl) (1,1'-biphényl) 2-sulfonamide,
- (±) N-(((cyclopentylméthyl) amino) carbonyl) 4'-((4-(méthylthio) 5-((phénylsulfinyl) acétyl) 2-propyl 1H-imidazol-1-yl) méthyl) (1,1'-biphényl) 2-sulfonamide,
- (±) N-(4'-((4-(méthylthio) 5-((phénylsulfinyl) acétyl) 2-propyl 1H-imidazol 1-yl) méthyl) (1,1'-biphényl-2-yl) sulfonyl) cyclopentanepropanamide,
ainsi que leurs sels d'addition avec les acides minéraux ou organiques pharmaceutiquement acceptables.

Les médicaments, objet de l'invention, peuvent être utilisés dans le traitement des affections cardiovasculaires présentant des désordres de la vasomotricité et de la volémie : infarctus du myocarde et ses conséquences, insuffisance cardiaque, insuffisance rénale, angine de poitrine, hyperaldostéronisme, hypertension artérielle et ses conséquences. Ces médicaments, objet de l'invention, pourraient également être utilisés pour le traitement du glaucome, de l'athérosclérose et de différents types de spasmes viscéraux, ainsi qu'à titre de substances protectrices neuronales ou encore dans la prévention des resténoses post-angioplastie.

Notamment les médicaments, objet de l'invention peuvent être utilisés pour leurs effets anti-hypertrophique et anti-fibrotique aux niveaux cardiaque et vasculaire. Tout particulièrement, ils peuvent être utilisés pour le traitement et la prévention des désordres cardiovasculaires et en particulier microcirculatoires associés au diabète.

Ils peuvent également être utilisés dans le traitement de certains désordres gastro-intestinaux, gynécologiques et en particulier pour un effet relaxant au niveau de l'utérus.

Les médicaments, objet de l'invention peuvent également être utilisés dans le traitement des troubles de la mémoire et des fonctions cognitives, ainsi que de l'anxiété.

L'invention s'étend aux compositions pharmaceutiques contenant à titre de principe actif l'un au moins des médicaments tels que définis ci-dessus.

Ces compositions pharmaceutiques peuvent être administrées par voie buccale, rectale, par voie parentérale ou par voie locale en application topique sur la peau et les muqueuses ou par injection par voie intraveineuse ou intramusculaire.

Ces compositions peuvent être solides ou liquides et se présenter sous toutes les formes pharmaceutiques couramment utilisées en médecine humaine comme, par exemple, les comprimés simples ou dragéifiés, les gélules, les granulés, les suppositoires, les préparations injectables, les pommades, les crèmes, les gels et les préparations en aérosols ; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie usuelle, variable selon le produit utilisé, le sujet traité et l'affection en cause, peut être, par exemple, de 1 à 100 mg par jour chez l'adulte, par voie orale.

Certains produits de départ de formules (II), (IX) et (XX) sont connus et peuvent être préparés par exemple comme indiqué dans le brevet européen EP 168 950.

D'autres produits de départ de formules (II), (IX) et (XX) peuvent en particulier être préparés comme indiqué dans le brevet européen EP 0465368 ou dans les préparations 1 à 6 décrites ci-après.

Certains produits de départ de formules (II), (IX) et (XX) sont commerciaux tels que par exemple :
les produits de formule (II) suivants :
- le 2-phénylimidazole
- le 2-méthoxyméthylimidazole
- le 2-propylimidazole
- le 2-isopropylimidazole
- le 2-éthylimidazole
- le 2-méthylimidazole
les produits de formule (IX) suivants :
- le 4-méthyl 2-phénylimidazole
- le 2,4-diméthylimidazole
- le 2-éthyl 4-méthylimidazole

Dès exemples de produits commerciaux de formule (XX) sont donnés dans les brevets EP 0465368 ou EP 0503162.

On peut encore notamment préparer certains produits de formules (II), (IX) et (XX) à partir d'autres produits de formule (II) ou (IX) par exemple en les soumettant à une ou plusieurs des réactions décrites aux points a) à u) ci-dessus réalisées comme indiqué ci-dessus.

Certains produits de formules (IX) et (XX) peuvent également être obtenus par monohalogénation du produit de formule (II) tel que défini ci-dessus en produit de formule (P₁) : 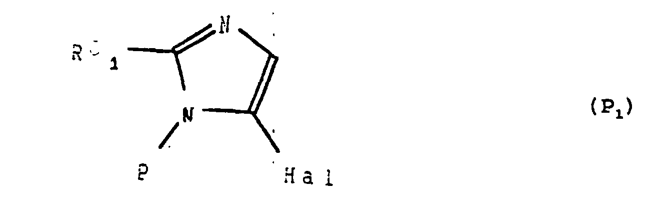 dans lequel R ₁ et P ont les significations indiquées ci-dessus pour le produit de formule (II), produit de formule (P₁) que l'on peut faire réagir après échange suivant la réaction halogène métal connue de l'homme du métier avec l'électrophile adapté, suivant les méthodes connues de l'homme du métier et notamment ainsi qu'il a été décrit ci-dessus pour passer du produit de formule (III) au produit de formule (V). Par le même processus, certains produits de formules (IX) et (XX) peuvent également être obtenus à partir du produit de formule (III) telle que définie ci-dessus. On peut encore notér que le produit de formule : 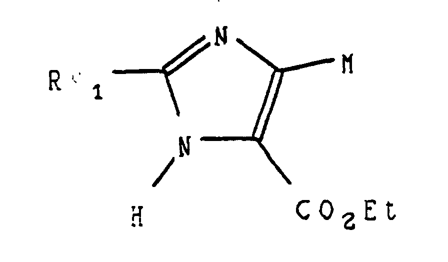 dans laquelle R₁ et M ont les significations indiquées ci-dessus, décrit dans EP 0465368, peut être soumis à une réaction de saponification thermique puis à une décarboxylation pour obtenir un produit de formule (IX) telle que définie ci-dessus.

Une telle illustration est donnée dans la partie expérimentale décrite ci-après.

Les produits de formule (III) dans lesquels R ₁ représente un radical alkylthio peuvent être obtenus soit à partir du produit de formule (II) tel que défini ci-dessus ainsi qu'il est décrit ci-dessus, soit à partir de produits commerciaux tels que notamment le 2,4,5-tribromoimidazole, le 4,5-dibromo 2-phénylimidazole, ainsi qu'il a été décrit ci-dessus pour passer du produit de formule (III) au produit de formule (V).

Les composés de départ de formule (VIII) peuvent être disponibles dans le commerce ou peuvent être préparés selon les méthodes usuelles connues de l'homme du métier.

Un procédé de préparation de certains produits de formule (VIII) est notamment décrit dans le brevet européen EP 0465368.

Des exemples de préparation de composés de formule (VIII) sont également décrits dans la littérature et des exemples en sont donnés notamment dans le brevet US 4,880,804 ou par exemple dans la référence Chemistry and Industry 7 september 1987 HOWARD and COLQUHOUN pp. 612-617.

Il a récemment été mis en évidence qu'il existe en fait deux sous-types de récepteurs à l'Angiotensine II : le récepteur AT₁ et le récepteur AT₂. Certains produits de formule (I) de la présente invention ont une affinité non seulement pour le récepteur AT₁ mais également pour le récepteur AT₂.

L'invention a ainsi particulièrement pour objet l'utilisation des produits de formule (I) telle que définie ci-dessus, pour la préparation de compositions pharmaceutiques destinées au traitement d'affections résultant d'une stimulation anormale des récepteurs AT₁ et AT₂ de l'Angiotensine II.

L'invention a plus particulièrement pour objet l'utilisation des produits de formule (I) telle que définie ci-dessus, pour la préparation de compositions pharmaceutiques destinées au traitement de l'hypertension artérielle, du post-infarctus et de l'insuffisance cardiaque et des resténoses post-angioplastie.

L'invention a tout particulièrement pour objet l'utilisation des produits de formule (I) telle que définie ci-dessus, pour la préparation de compositions pharmaceutiques destinées au traitement de l'insuffisance rénale.

L'invention a également pour objet l'utilisation des produits de formule (I) telle que définie ci-dessus, pour la préparation de compositions pharmaceutiques destinées au traitement et à la prévention des désordres cardiovasculaires et en particulier microcirculatoire, associés au diabète.

Les exemples suivants illustrent l'invention sans toutefois la limiter.

### PREPARATION 1 : 2-butyl α-hydroxy α-méthyl 1-[(2'-((((diméthylamino) méthylène) amino) sulfonyl) (1,1'-biphényl) 4-yl) méthyl] 4-(méthylthio) 1H-imidazole 5-acétate d'éthyle

### Stade A : 4'-[(2-butyl 4-(méthylthio) 1H-imidazol 1-yl) méthyl] N-[(diméthylamino) méthylène] (1,1'-biphényl) 2-sulfonamide

### a) 2-butyl 4-(méthylthio) 1H-imidazole

On introduit 760 mg de 2-n-butyl 4-méthylthio imidazole 5-carboxylate d'éthyle, préparé ainsi qu'il est décrit dans la demande de brevet européen EP 0465368, dans 15 cm³ de NaOH(2N). On porte à reflux et agite durant 24 heures. Après refroidissement, on dilue par 50 cm³ H₂O, extrait par 3 x 20 cm³ CH₂Cl₂, lave par 20 cm³ H₂O et sèche. On obtient 535 mg de produit attendu.
PF = 64°C.

### Spectre IR (CHCl₃)

| | |
|---|---|
| Absence de C=0 | |
| =C-NH | 3452 cm⁻¹ |
| Système conjugué | 1596 - 1502 cm⁻¹ |

### b) 4'-[(2-butyl 4-(méthylthio) 1H-imidazol 1-yl) méthyl] N-[(diméthylamino) méthylène] (1,1'-biphényl) 2-sulfonamide

On dissout 5 g de 2-butyl 4-(méthylthio) 1H-imidazole dans 120 cm³ de THF. Puis on ajoute lentement à la solution orangée obtenue 1,55 g d'hydrure de sodium à 50 % en dispersion dans l'huile. La température s'élève jusqu'à 25° C. On agite 30 mn à cette température puis on introduit 14 g de 4'-bromométhyl N-[(diméthylamino) méthylène] (1,1'-biphényl) 2-sulfonamide. On agite à température ambiante jusqu'à fin d'évolution, soit pendant environ 3 heures. On reprend à l'eau, extrait à l'acétate d'éthyle, sépare par chromatographie sur silice en éluant à l'acétate d'éthyle puis empâte à l'éther iso, filtre et sèche. On obtient ainsi 9,35 g du )produit attendu (cristaux incolores). PF = 148° C.

### Spectre IR : CHCl₃

| | |
|---|---|
| Absence de =C-NH- | |
| -SO₂-N=C-N〈 | 1627 cm⁻¹ |
| aromatique, hétéroaromatique | 1593-1564-1516-1500 cm⁻¹ |

### Stade B : 4'-[(5-bromo 2-butyl 4-(méthylthio) 1H-imidazol 1-yl) méthyl] N-[(diméthylamino) méthylène] (1,1'-biphényl) 2-sulfonamide

On dissout 10,4 g du produit obtenu au stade A, ci-dessus, dans 450 cm³ de CH₂Cl₂ et ajoute 3,9 g de N-bromosuccinimide.

On agite environ 15 mn à température ambiante, lave à l'eau et à l'eau salée, décante, sèche, filtre et chasse le solvant sous vide à 50°C.

On empâte à l'éther iso, filtre et sèche. On obtient 11,8 g de produit attendu (cristaux incolores) PF = 158°C.

### Spectre IR : CHCl₃

| | |
|---|---|
| -N=CH-N〈 | 1628 cm-1 |
| aromatique et hétéroaromatique | 1592-1568 cm⁻¹ |

| Microanalyse : | |
|---|---|
| | Br |
| % calculé | 14,54 |
| % trouvé | 14,4-14,7 |

### Stade C : 2-butyl α-hydroxy α-méthyl 1-[(2'-((((diméthylamino) méthylène) amino) sulfonyl) (1,1'-biphényl) 4-yl) méthyl] 4-(méthylthio) 1H-imidazole 5-acétate d'éthyle

On dissout 11,8 g du produit obtenu au stade B ci-dessus dans 160 cm³ de THF. Puis on ajoute sand dépasser 25°C, 17,5 cm³ d'une solution 1M de chlorure d'isopropyl magnésium en solution dans l'éther. Après environ 30 mn d'agitation à température ambiante, on ajoute lentement 4 cm³ de pyruvate d'éthyle. On agite environ 1 heure à température ambiante, ajoute 1 cm³ de pyruvate d'éthyle et poursuit l'agitation pendant encore environ 1 heure.

On reprend par 200 cm³ de NH₄Cl en solution à 10 % et extrait à l'acétate d'éthyle. On sèche, concentre jusqu'à - 200 cm³, filtre et sèche les cristaux obtenus.

On obtient 6 g de produit attendu (cristaux incolores) PF = 208-210°C.

### Spectre IR : CHCl₃

| | |
|---|---|
| OH complexe | ~ 3530 cm⁻¹ |
| C=O | 1722 cm⁻¹ |
| C=N | 1626 cm⁻¹ |
| Hétérocycle + aromatique | 1565, 1518 cm⁻¹ |

### Spectre UV :

| 1) Dans EtOH | |
|---|---|
| infl. 274 nm | ∈ = 3100 |
| infl. 231 nm | ∈ = 23000 |

| 2) Dans EtOH - HCl N/10 | |
|---|---|
| infl. 228 nm | ∈ = 30000 |
| infl. 273 nm | ∈ = 3400 |

### PREPARATION 2 : 2-méthyl 4-(méthylthio) 1-[(2'-((((diméthylamino) méthylène) amino) sulfonyl) (1,1'-biphényl) 4-yl) méthyl] α-oxo 1H-imidazole 5-acétate d'éthyle

### Stade A : 4,5-dibromo 2-méthyl 1-[(2-(triméthylsilyl) éthoxy) méthyl] 1H-imidazole

Le produit est obtenu par 2 réactions consécutives.

### 1) Etape de protection de la 2-méthyl imidazole

On dissout 6,8 g de 2-méthyl imidazole dans 250 cm³ de THF et on ajoute par petites fractions 4 g d'hydrure de sodium à 50 % dans l'huile. La réaction étant exothermique, on maintient la température du milieu à température ambiante durant environ 30 mn.

On ajoute alors goutte à goutte dans le milieu réactionnel 17,5 cm³ de chlorure de SEM. Après environ 20 mn d'agitation à 20°C, on hydrolyse l'excès d'hydrure de sodium par addition de THF à 20 % H₂O.

La solution obtenue est amenée à sec, reprise par de l'acétate d'éthyle et lavée par de l'eau. La phase organique ainsi recueillie est séchée. On obtient une huile jaune qui est purifiée par chromatographie sur silice avec pour éluant : CH₂Cl₂-méthanol (90-10).

On obtient ainsi 14 g de produit protégé.

### 2) Bromation

Le produit protégé obtenu ci-dessus est dissout dans 250 cm³ de CH₂Cl₂. On ajoute ensuite à cette solution 25 g de N-bromo succinimide par petites fractions.

L'agitation est maintenue environ 30 mn à température ambiante. La phase organique est lavée par une solution de )bicarbonate de sodium, puis abondamment à l'eau. Après séchage et évaporation, on récupère 13,4 g de produit attendu (huile jaune homogène).

### Spectre IR : CHCl₃

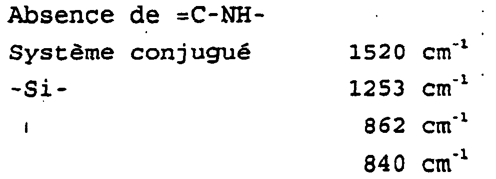

### Stade B : 2-méthyl 1-[(2-(triméthylsilyl) éthoxy) méthyl] 5-(méthylthio) α-oxo 1H-imidazole 4-acétate d'éthyle

On dissout, sous atmosphère anhydre, 3 g du produit obtenu au stade A ci-dessus, dans 20 cm³ de THF anhydre. Cette solution est alors refroidie à -78°C et on y ajoute, tout en maintenant la température à -78°C, 5,6 cm³ de n-butyl lithium 1,5 molaire dans l'hexane. L'agitation est maintenue à -78°C pendant environ 10 mn.

On introduit ensuite 0,76 cm³ de diméthyl disulfure, puis laisse remonter lentement la température jusqu'à 20°C et agite pendant environ 30 mn.

Le milieu réactionnel est de nouveau refroidi à -78°C, et on y ajoute comme précédemment 5,6 cm³ de n-butyl lithium 1,5 M dans l'hexane. Après environ 10 mn d'agitation à -78°C, on introduit en une fois 5,5 cm³ d'oxalate de diéthyle. L'agitation est maintenue à température ambiante pendant environ 30 mn. Le milieu réactionnel est alors versé sur de l'eau glacée. On extrait par de l'acétate d'éthyle, lave la phase organique par une solution de bicarbonate de sodium puis par de l'eau et sèche. On récupère une huile brune, que l'on purifie par chromatographie sur silice avec pour éluant : acétate d'éthyle-cyclohexane (50-50). On obtient 1,63 g de produit attendu (huile).

### Spectre IR : CHCl₃

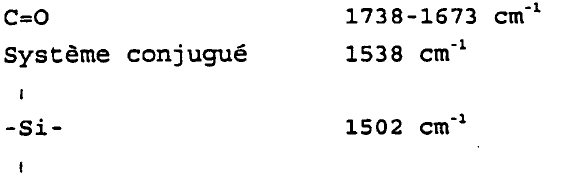

### Stade C : 2-méthyl 5-(méthylthio) α-oxo 1H-imidazole 4-acétate d'éthyle

On dissout 1,6 g du produit obtenu au stade B ci-dessus dans 30 cm³ de CH₂Cl₂, et ajoute 10 cm³ d'acide trifluoroacétique. Le milieu réactionnel est alors porté au reflux pendant environ 10 h. La solution est ensuite amenée à sec, et le résidu repris par de l'eau. On alcaline la phase aqueuse par addition de bicarbonate de sodium, extrait par de l'acétate d'éthyle, lave à l'eau puis sèche et récupère 920 mg de produit attendu (huile jaune) utilisé tel quel dans la suite de la synthèse.

### Spectre IR : CHCl₃

| | |
|---|---|
| =C-NH | 3415 cm⁻¹ |
| C=O | 1716-1633 cm⁻¹ |
| Système conjugué | 1530-1502 cm⁻¹ |

### Stade D : 2-méthyl 4-(méthylthio) 1-[(2'-((((diméthylamino) méthylène) amino) sulfonyl) (1,1'-biphényl) 4-yl) méthyl] α-oxo 1H-imidazole 5-acétate d'éthyle

On dissout 880 mg du produit obtenu au stade C ci-dessus dans 10 cm³ de DMF/anhydre et ajoute successivement 800 mg de carbonate de potassium puis 2,2 g de 4'-bromométhyl N-[(diméthylamino) méthylène] (1,1'-biphényl) 2-sulfonamide. L'agitation est maintenue pendant environ 3 H à température ambiante. La suspension jaune ainsi obtenue est versée sur de l'eau. On extrait par de l'acétate d'éthyle, lave à l'eau puis sèche et récupère une résine jaune que l'on purifie sur silice avec pour éluant l'acétate d'éthyle.

On obtient ainsi 1,17 g de produit attendu.

### Spectre IR : CHCl₃

| | |
|---|---|
| Absence de =C-NH | |
| C=O ester | 1735 cm⁻¹ |
| Autre C=O | 1629 cm⁻¹ (F) |
| C=N | |
| Aromatique | 1570 cm⁻¹ |
| hétéroatome | 1516 cm⁻¹ |

### PREPARATION 3 : 1-[(2'-(aminosulfonyl) (1,1'-biphényl) 4-yl) méthyl] 4-(méthylthio) α-oxo 2-propyl 1H-imidazole 5-acétate d'éthyle

### Stade A : 4,5-dibromo 2-n-propyl 1-[(2-(triméthylsilyl) éthoxy) méthyl] 1H-imidazole

On procède comme au stade A de la préparation 2, en remplaçant 2-méthyl 1H-imidazole par le 2-propyl 1H-imidazole.

Le produit est obtenu par 2 réactions consécutives.

### 1) Etape de protection de la 2-propyl imidazole

On dissout 30 g de 2-propyl imidazole dans 500 cm³ de THF et on ajoute par petites fractions 12,5 g d'hydrure de sodium à 50 % dans l'huile.

On ajoute alors dans le milieu réactionnel 53 cm³ de chlorure de SEM, on hydrolyse par du THF à 20 % H₂O.

On obtient ainsi 57,3 g de produit protégé.

### 2) Bromation

Le produit protégé obtenu ci-dessus est dissous dans 500 cm³ de CH₂Cl₂. On ajoute 93,5 g de N-bromo succinimide et obtient 93,7 g de produit attendu.

### Spectre IR : CHCl₃

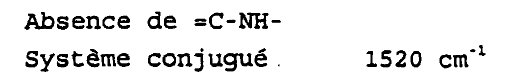

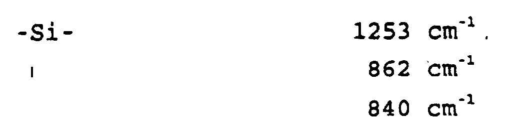

### Stade B : 2-n-propyl 1-[(2-(triméthylsilyl) éthoxy) méthyl] 5-(méthylthio) α-oxo 1H-imidazole 4-acétate d'éthyle

On introduit, sous atmosphère anhydre, 36,7 g du produit obtenu au stade A ci-dessus, dans 200 cm³ de THF, on ajoute à -78°C 63,7 cm³ de n-butyl lithium 1,5 Molaire dans l'hexane puis 8,62 cm³ de diméthyl disulfure, on laisse remonter à température ambiante. Puis on ajoute comme précédemment à -78°C 63,7 cm³ de n-butyl lithium 1,5 Molaire dans l'hexane suivi de 55 cm³ d'oxalate d'éthyle. On obtient 11,75 g de produit attendu.

### Spectre IR : CHCl₃

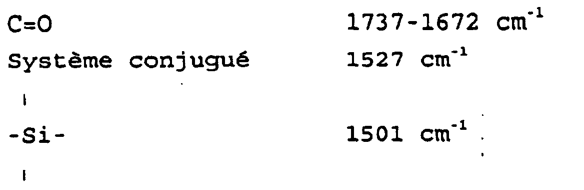

### Stade C : 2-n-propyl 5-(méthylthio) α-oxo 1H-imidazole 4-acétate d'éthyle

On procède comme au stade C de la préparation 2 à partir de 11 g du produit obtenu au stade B ci-dessus dans 200 cm³ de CH₂Cl₂, et 40 cm³ d'acide trifluoroacétique. On obtient 7,15 g de produit attendu.

### Spectre IR : CHCl₃

| | |
|---|---|
| =C-NH | 3413 cm⁻¹ |
| C=O | 1714-1633 cm⁻¹ |
| Système conjugué | 1524-1492 cm⁻¹ |

### Stade D : 2-n-propyl 4-(méthylthio) 1-[(2'-((((diméthylamino) méthylène) amino) sulfonyl) (1,1'-biphényl) 4-yl) méthyl] α-oxo 1H-imidazole 5-acétate d'éthyle

On procède comme au stade D de la préparation 2 à partir de 7 g du produit obtenu au stade C ci-dessus dans 100 cm³ de DMF et 7,5 g de carbonate de potassium et 15,6 g de 4'-(bromométhyl) N-[(diméthylamino) méthylène] (1,1'-biphényl) 2-sulfonamide. On obtient ainsi 7,83 g de produit attendu.

### Spectre IR : CHCl₃

| | |
|---|---|
| Absence de =C-NH | |
| C=O ester | 1735 cm⁻¹ |
| Autre C=O | 1630 cm⁻¹ (F) |
| C=N | |

### Stade E : 1-[(2'-(aminosulfonyl) (1,1'-biphényl) 4-yl) méthyl] 2-n-propyl 4-(méthylthio) α-oxo 1H-imidazole 5-acétate d'éthyle

On procède comme au stade A de l'exemple 2 à partir de 7,8 g du produit obtenu au stade D ci-dessus, dans 100 cm³ d'éthanol et 30 cm³ de HCl concentré et obtient ainsi 3,6 g de produit attendu.

### Spectre IR CHCl₃

| | |
|---|---|
| -NH₂ | 3443-3343 cm⁻¹ |
| C=O | 1734-1627 cm⁻¹ |
| Aromatique | 1593 cm⁻¹ |
| Hétéroatome | 1565 cm⁻¹ |
| NH₂ def. | 1542 cm⁻¹ |

### PREPARATION 4 : 1-[(2'-(aminosulfonyl) (1,1'-biphényl) 4-yl) méthyl] 2-butyl 4-(méthylthio) α-oxo 1H-imidazole 5-éthane thioate de S-méthyle

### Stade A : 4'-[(2-butyl 5-((méthylsulfinyl) acétyl) 4-(méthylthio) 1H-imidazol 1-yl) méthyl] (1,1'-biphényl) 2-sulfonamide

On prépare d'abord l'anion du DMSO en introduisant 3,36 g d'hydrure de sodium à 50 % dans l'huile. Le NaH est débarrassé de son huile par 3 lavages successifs au pentane. Puis on sèche et on ajoute 70 cm³ de diméthylsulfoxyde anhydre et le mélange est porté à 75°C, pendant environ 1 H. On baisse alors la température à 0°C et ajoute à l'anion du DMSO ainsi formé 70 cm³ de THF anhydre et 9,5 g de 2-butyl 1-[(2'-((((diméthylamino) méthylène) amino) sulfonyl) (1,1'-biphényl) 4-yl) méthyl] 4-(méthylthio) 1H-imidazole 5-carboxylate d'éthyle, préparé ainsi qu'il est indiqué dans la demande de brevet européen EP 0503162, dissous dans 70 cm³ de THF anhydre. Puis on laisse remonter à température ambiante et agite pendant environ 1/2 H. Le mélange réactionnel est alors versé dans 400 cm³ H₂O.

La solution est acidifiée jusqu'à pH 2 avec HCl 2N. On extrait par 4 x 200 cm³ de chlorure de méthylène et lave la phase organique par 4 x 100 cm³ d'H₂O distillée. On sèche la phase organique, filtre et évapore.

On purifie sur silice avec pour éluant CH₂Cl₂-méthanol (9-1) et récupère 7,5 g de produit attendu.

### Spectre IR dans CHCl₃

| | |
|---|---|
| Absence de SO₂N=-N〈 | |
| NH₂ | 3440-3340 cm⁻¹ |
| C=O | 1628 cm⁻¹ |
| Aromatique + Hétérocycle | 1545 - 1525 - 1495 cm⁻¹ |
| SO₂ | 1345-1165 cm⁻¹ |
| SO | 1050 cm⁻¹ |

### Stade B : 4'-[[5-[bromo (méthylsulfinyl) acétyl] 2-butyl 4-(méthylthio) 1H-imidazol 1-yl] méthyl] (1,1'-biphényl) 2-sulfonamide

On introduit 1 g du produit obtenu au stade A, ci-dessus et 530 mg de K₂CO₃. Puis on ajoute 10 cm³ de CH₂Cl₂ anhydre, porte la température du milieu à 0°C et ajoute goutte à goutte 342 mg de N-bromo succinimide, dissout dans le minimum de CH₂Cl₂ anhydre. On ajoute alors 100 cm³ de CH₂Cl₂ et lave la phase organique par 3 x 200 cm³ d'eau distillée, et 1 x 100 cm³ de NaCl saturée. On sèche, filtre et concentre à sec.

On obtient 1,09 g de produit attendu.

### Spectre IR dans CHCl₃

| | |
|---|---|
| NH₂ | 3440-3344 cm⁻¹ |
| >=O | 1634 cm⁻¹ |
| Système conj. + Aromatique + NH₂ | 1542 - 1520 cm⁻¹ |

### Stade C : 1-[(2'-(aminosulfonyl) (1,1'-biphényl) 4-yl) méthyl] 2-butyl 4-(méthylthio) α-oxo 2-propyl 1H-imidazole 5-éthane thioate de S-méthyle

On dissout 6,8 g du produit obtenu au stade B ci-dessus, dans 60 cm³ d'un mélange 25 cm³ TFA-75 cm³ CH₂Cl₂ et porte à reflux de chlorure de méthylène, pendant environ 5 heures. On traite la réaction en neutralisant par une solution de NaHCO₃ saturée jusqu'à pH 5-6, extrait par 2 x 200 cm³ d'acétate d'éthyle, lave par 1 x 100 cm³ de NaCl saturée, sèche, filtre et concentre à sec. On purifie sur silice, avec pour éluant ACOEt-cyclohexane (5-5). On récupère ainsi 2,7 g de produit attendu.

### Spectre IR dans CHCl₃

| | |
|---|---|
| NH₂ | 3445-3350 cm⁻¹ |
| >=O | 1670-1614 cm⁻¹ |
| Aromatique + hétéroaromatique + NH₂ dif. | 1542 cm⁻¹ - 1518 cm⁻¹ |

### PREPARATION 5 : 1-[(2'-(aminosulfonyl) (1,1'-biphényl) 4-yl) méthyl] 4-(méthylthio) 2-propyl 1H-imidazole 5-carboxylate d'éthyle

### Stade A : cyano-[(1-oxobutyl) amino] acétate d'éthyle

On mélange 5 g d'éthyl (hydroxyimino) cyanoacétate, 40 cm³ de tétrahydrofuranne, 11,5 cm³ d'anhydride butyrique et 2,5 g de platine et agite en atmosphère d'hydrogène jusqu'à saturation. On filtre, rince par 5 x 15 cm³ d'éther éthylique, évapore l'éther, ajoute peu à peu 200 cm³ d'essence G, essore, lave avec 3 x 10 cm³ d'essence G et sèche à environ 75°C. On concentre à ~ 10 cm³, ajoute 50 cm³ d'essence G, laisse cristalliser 30 mn à température ambiante, essore, lave avec 3 x 3 cm³ d'essence G et sèche à environ 75°C. On obtient 5,73 g de produit. PF = 110°C.
Recristallisation pour analyses :

On dissout 540 mg du produit obtenu dans 50 cm³ d'éther isopropylique à reflux, filtre, concentre, laisse environ 1 h au repos à température ambiante, essore, lave à l'éther isopropylique et sèche. On obtient 440 mg de produit attendu.
PF = 110°C.

| Microanalyse pour C₉H₁₄N₂O₃ = 198,22 | | | | |
|---|---|---|---|---|
| | C | H | N | O |
| % calculé | 54,53 | 7,12 | 14,13 | 24,22 |
| % trouvé | 54,5 | 7,2 | 14,0 | |

### Spectre IR CHCl₃

| | |
|---|---|
| =C-NH | ~ 3430 cm⁻¹ |
| -C≡N | ~ 2245 cm⁻¹ |
| C=O | 1758 cm⁻¹ ester 1692 cm⁻¹ amide |
| Amide II | 1492 cm⁻¹ |

### Stade B : 3-amino 2-[(1-oxobutyl) amino] 3-(méthylthio) 2-propenoate d'éthyle

A une solution de 20 g de nitrile obtenu au stade A ci-dessus, dans 400 ml d'éthanol, on ajoute 1,4 ml de triéthylamine, on refroidit à environ -10°C et introduit environ 22 g de méthylmercaptan par barbotage. On agite environ 72 heures à 0°C. On élimine l'excès de méthanethiol chasse l'éthanol, empâte à l'essence G, filtre et sèche. On obtient 24,3 g de produit attendu (cristaux incolores).
F_{K115} = 120-124°C

| Microanalyse pour C₁₀H₁₈N₂O₃S = 246,33 | | | | | |
|---|---|---|---|---|---|
| | C | H | N | S | O |
| % calculé | 48,76 | 7,37 | 11,37 | 13,02 | 19,49 |
| % trouvé | 48,6 | 7,5 | 11,4 | 12,6 | - |

### Spectre IR CHCl₃

| | |
|---|---|
| =C-NH₂ | 3500, 3412 cm⁻¹ |
| =C-NH | 3365, 3275 cm⁻¹ |
| C=O complexe | 1665 cm⁻¹ |
| C=C et NH₂ déf. | 1592 cm⁻¹ |
| Amide II | 1488 cm⁻¹ |

### Spectre UV dans EtOH

| | |
|---|---|
| Max. 220 nm | ∈ = 5500 |
| Max. 291-292 | ∈ = 19400 |

### Stade C : 4-(méthylthio) 2-propyl 1H-imidazole 5-carboxylate d'éthyle

A 20,1 g de pentachlorure de phosphore dans 300 cm³ de chlorure de méthylène, refroidis à environ -70°C on ajoute une solution de 12,9 g de 4-diméthylaminopyridine dans 90 cm³ de chlorure de méthylène.

On maintient encore environ 15 mn à environ -70°C puis on introduit une solution de 12 g du produit obtenu au stade B ci-dessus dans 120 cm³ de chlorure de méthylène. On laisse revenir à température ambiante et maintient sous agitation pendant environ 22 heures.

On verse le mélange réactionnel dans 2,5 1 d'eau + glace et neutralise par addition d'environ 60 g de bicarbonate de sodium. On agite encore environ 30 mn, on décante et on extrait avec 500 cm³ de CH₂Cl₂. On lave à l'eau salée, sèche et chasse le solvant à environ 50°C. On purifie par chromato-graphie sur silice avec pour éluants CH₂Cl₂-ACOEt (90-10) puis CH₂Cl₂-ACOEt (80-20). On chasse les solvants à environ 50°C, empâte à l'essence G, filtre et sèche. On obtient 7,4 g de produit attendu (cristaux incolores). F_{K95} = 85°C.

### Microanalyse

| Concordance pour C₁₀H₁₆N₂O₂S = 228,32 | | | | | |
|---|---|---|---|---|---|
| | C | H | N | S | O |
| % calculé | 52,61 | 7,06 | 12,27 | 14,04 | 14,02 |
| % trouvé | 52,7 | 7,3 | 12,2 | 14,0 | |

### Spectre IR CHCl₃

| | |
|---|---|
| =C-NH | 3440 - 3260 cm⁻¹ |
| C=O Complexe max. | ~ 1672 cm⁻¹ |
| Hétérocycle | 1542 - 1498 cm⁻¹ |

### Spectre UV dans EtOH

| | |
|---|---|
| Max. 213-214 nm | ∈ = 14500 |
| Infl. 229 nm | ∈ = 7200 |
| Max. 286 nm | ∈ = 12200 |

### Spectre UV dans EtOH/HCl N/10

| | |
|---|---|
| Max. 238 nm | ∈ = 6800 |
| Max. 277 nm | ∈ = 9600 |
| par retour basique --> max. 296 nm. | |

### Stade D : 1-[(2'-((((diméthylamino) méthylène) amino) sulfonyl) (1,1'-biphényl) 4-yl) méthyl] 4-(méthylthio) 2-propyl 1H-imidazole 5-carboxylate d'éthyle

On dissout 8,1 g du produit obtenu au stade C ci-dessus, dans 80 cm³ de diméthylformamide et ajoute 16,1 g de 4'-(bromométhyl) N-[(diméthylamino) méthylène] (1,1'-biphényl) 2-sulfonamide et 4,9 g de carbonate de potassium. On agite à température ambiante pendant environ 24 heures. On chasse le DMF à 50°C, empâte à l'eau, filtre, lave à l'eau et sèche à environ 60°C.

On empâte dans 240 cm³ d'ACOEt, sous agitation pendant environ 30 mn à environ 50°C, ajoute 160 cm³ d'hexane, filtre et sèche. On obtient 14 g de produit attendu (cristaux incolores). F_{K163} : 182°C.

### Spectre IR CHCl₃

| | |
|---|---|
| Absence de =C-NH | |
| C=O | 1690 cm⁻¹ |
| C=N | 1628 cm⁻¹ |
| Hétérocycle | 1504 - 1565 cm⁻¹ |

### Spectre UV dans EtOH

| | |
|---|---|
| Infl. 230 nm | ∈ = 32000 |
| Max. 287 nm | ∈ = 15500 |

### Stade E : 1-[(2'-(aminosulfonyl) (1,1'-biphényl) 4-yl) méthyl] 4-(méthylthio) 2-propyl 1H-imidazole 5-carboxylate d'éthyle

On mélange 11,2 g du produit obtenu au stade D, ci-dessus avec 200 cm³ d'éthanol et 100 cm³ d'acide chlorhydrique concentré. On chauffe au reflux pendant environ 2 heures. On évapore l'éthanol, dilue par addition de 400 cm³ d'eau, alcalinise sous agitation par addition de lessive de soude et extrait à l'acétate d'éthyle. On lave à l'eau et à l'eau salée, sèche, filtre et chasse le solvant à environ 50°C.

On purifie par chromatographie sur silice avec pour éluant ACOEt 60 Hexane 40. On obtient 9,3 g de produit attendu (cristaux incolores). F_{K135} = 130-132°C.

### Spectre IR CHCl₃

| | |
|---|---|
| Absence de produit de départ | |
| C=O | 1689 cm⁻¹ |
| NH₂ | 3444 - 3340 cm⁻¹ |
| Système conjugué + aromatique NH₂ déf. | 1618 - 1590 - 1560 - 1540 - 1508 cm⁻¹ |

### Spectre UV dans EtOH

| | |
|---|---|
| Infl. 210 nm | ∈ = 45000 |
| Infl. 234 nm | ∈ = 17000 |
| Max. 286 nm | ∈ = 15000 |

### PREPARATION 6 :

### Stade A : 2,4,5-tribromo 1-[(2-(triméthylsilyl) éthoxy) méthyl] 1H-imidazole

On dissout 3,04 g de tribromoimidazole dans 20 cm³ de THF anhydre. On ajoute par petites portions 550 mg de NaH à 50 % dans l'huile. Après 20 minutes à température ambiante, on ajoute 1,95 ml de chlorure de 2-(triméthylsilyl) éthoxy méthyl. Après 30 minutes à température ambiante, on hydrolyse avec une solution saturée de NH₄Cl. On extrait à l'ACOEt, sèche, évapore à sec. On chromatographie sur silice avec pour éluant cyclohexane/ACOEt (9-1) et l'on récupère 4,2 g de produit attendu (huile).

### Stade B : 2n-propylthio 4,5-dibromo 1-[(2-(triméthylsilyl) éthoxy) méthyl] 1H-imidazole

On dissout 3,48 g de produit obtenu au stade A dans 25 cm³ d'éther. On ajoute 3 ml d'une solution d'EtMgBr 3M dans l'éther. Après 15 minutes à température ambiante, on ajoute 2,5 cm³ de disulfure de n-dipropyle puis 40 cm³ de THF anhydre. On distille l'éther et laisse 20 h à reflux de THF. On hydrolyse avec NH₄Cl saturé, extrait à l'ACOEt, sèche et évapore à sec.

Après chromatographie avec pour éluant (cyclohexane-ACOEt (9-1)), on obtient 2,7 g de produit attendu (huile).

### Stade C : 2n-propylthio 4-bromo 5-méthylthio 1-[(2-(triméthylsilyl) éthoxy) méthyl] 1H-imidazole

On dissout 2,4 g de produit attendu au stade B dans 25 cm³ de THF anhydre. On ajoute 3 ml d'une solution d'EtMgBr 3M dans l'éther. Après 1 h à température ambiante, on ajoute 1,08 cm³ de disulfure de diméthyle et on laisse 1 heure à la température ambiante puis on hydrolyse avec NH₄Cl saturé, extrait à l'ACOEt, sèche et évapore à sec.

Après chromatographie avec pour éluant cyclohexane-ACOEt (9-1), on obtient 1,83 g de produit attendu (huile).

### Stade D : 2n-propylthio 5-méthylthio 1-[(2-(triméthylsilyl) éthoxy) méthyl] 1H-imidazole

On introduit à température ambiante 7,94 g du produit obtenu au stade C ci-dessus, 80 cm³ de THF anhydre, refroidit à -78°C et ajoute 13,75 cm³ de n-butyllithium solution à 1,6 M dans l'hexane. Après 10 minutes, on ajoute 15 cm³ de chloroformiate d'éthyle, agite encore 15 mn à -78°C. Ajoute 60 cm³ d'eau, laisse revenir à température ambiante, évapore le THF, ajoute 10 cm³ de soude à 32 % pour détruire le chloroformiate d'éthyle en excès, extrait par 3 x 200 cm³ d'AcOEt lave par 2 x 80 cm³ d'eau, sèche, filtre et évapore.

Après chromatographie sur silice avec pour éluant AcOEt-cyclo (2-8), on récupère 4,34 g de produit attendu.

### Spectre IR : dans CHCl₃

| | |
|---|---|
| =O | 1708 cm⁻¹ |
| C=C | 1504 cm⁻¹ |
| C=N | |

### Stade E : 2n-propylthio 5-méthylthio imidazole 4-carboxylate d'éthyle

On introduit à température ambiante, 4,34 g de produit .0 obtenu au stade D ci-dessus, 40 cm³ d'EtOH, 28 cm³ d'eau, 28 cm³ d'HCl fumant. On chauffe à l'aide d'un bain à 70°C pendant deux heures. On évapore l'EtOH, passe en milieu alcalin avec environ 20 cm³ de soude à 32 %, revient à ph ∼ 6, avec 1 à 2 cm³ d'acide acétique, extrait par 3 x 300 cm³ d'AcOEt, lave par 2 x 70 cm³ d'eau, sèche, filtre et évapore. Après chromatographie sur silice avec pour éluant AcOEt-cyclo (3-7), et empâtage dans l'essence G, on sèche et obtient 3,72 g de produit, dont 200 mg sont recristallisés dans MeOH-eau (50-50). On obtient 149 mg de produit attendu.

### Spectre IR : dans CHCl₃

| | |
|---|---|
| =C-NH | 3430-3225 cm⁻¹ |
| =O | 1682 cm⁻¹ |
| Hétérocycle | 1522 cm⁻¹ |

### Stade F : 1-[(2'-((((diméthylamino) méthylène) amino) sulfonyl) (1,1'-biphényl) 4-yl) méthyl] 4-(méthylthio) 2n-propylthio 1H-imidazole 5-carboxylate d'éthyle

On introduit à température ambiante 1,04 g du produit obtenu au stade E ci-dessus, 10 cm³ de DMF, 0,55 g de K₂CO₃, 2,1 g de 4'-(bromométhyl) N-[(diméthylamino) méthylène] (1,1'-biphényl) 2-sulfonamide. On agite à température ambiante 2 h puis évapore le DMF, reprend par 3 x 150 cm³ de CH₂Cl₂, lave par 2 x 50 cm³ d'eau, par 50 cm³ de solution saturée en NaCl, sèche, filtre et évapore. On chromatographie sur silice avec pour éluant AcOEt-cyclo (6-4), puis met à cristalliser dans 5 l'éther et obtient 1,93 g de produit, dont 100 mg sont recristallisés dans l'éther isopropylique. On obtient 66 mg de produit attendu. F = 160°C.

### Spectre IR : dans CHCl₃

| | |
|---|---|
| =O | 1685 cm⁻¹ |
| SO₂N=CH-N〈 | 1624 cm⁻¹ |

### EXEMPLE 2 : 2-méthyl 4-(méthylthio) 1-[(2'-(((((phénylméthyl) amino) carbonyl) amino) sulfonyl) (1,1'-biphényl) 4-yl) méthyl] α-oxo 1H-imidazole 5-acétate d'éthyle

### Stade A : 1-[(2'-(aminosulfonyl) (1,1'-biphényl) 4-yl) méthyl] 2-méthyl 4-(méthylthio) α-oxo 1H-imidazole 5-acétate d'éthyle

On dissout 4,8 g du produit obtenu à la préparation 2, dans le mélange de 50 cm³ d'éthanol et 30 cm³ de HCl concentré. Le milieu réactionnel est porté au reflux pendant environ 5 h. La solution est alors concentrée sous vide puis reprise par de l'eau glacée. On alcalinise par addition de NH₄OH jusqu'à pH ~ 8 puis extrait par de l'acétate d'éthyle, lave à l'eau, sèche puis purifie par chromatographie sur silice avec pour éluant l'acétate d'éthyle.

On obtient ainsi 2,4 g de produit attendu.

### Spectre IR CHCl₃

| | |
|---|---|
| -NH₂ | 3443-3343 cm⁻¹ |
| C=O | 1734-1627 cm⁻¹ |
| Aromatique | 1593 cm⁻¹ |
| Hétéroatome | 1565 cm⁻¹ |
| NH₂ def. | 1542 cm⁻¹ |

### Stade B : 2-méthyl 4-(méthylthio) 1-[(2'-(((((phénylméthyl) amino) carbonyl) amino) sulfonyl) (1,1'-biphényl) 4-yl) méthyl] α-oxo 1H-imidazole 5-acétate d'éthyle

On dissout 0,8 g du produit obtenu au stade A ci-dessus dans 25 cm³ d'acétone et ajoute 465 mg de carbonate de potassium. Le milieu est alors porté au reflux, et on ajoute goutte à goutte 0,2 cm³ d'isocyanate de benzyle, l'agitation est maintenue dans ces conditions pendant environ 1 h. On évapore à sec, reprend le résidu par de l'eau puis on acidifie par addition d'hydrogénophosphate de sodium, le précipité est essoré, lavé abondamment à l'eau puis séché. On purifie par empâtage dans un mélange isopropanol 10 cm³, éther isopropylique 20 cm³, essore, lave par 2 x 25 cm³ d'éther isopropylique et obtient 480 mg de produit attendu (solide jaune).
PF = 130°C.

### Spectre IR : CHCl₃

| | |
|---|---|
| Absence de SO₂NH₂ | |
| -NH-C= complexe | 3395-3375 cm⁻¹ |
| C=O | 1732-1714-1624 cm⁻¹ |
| Aromatique | 1539-1497 cm⁻¹ |
| Hétéroatome | |
| Amide II | |

### EXEMPLE 3 : acide 2-méthyl 4-(méthylthio) alpha oxo-1-((2'-(((((phénylméthyl) amino) carbonyl) amino) sulfonyl) (1,1'-biphényl) 4-yl) méthyl) 1H-imidazole 5-acétique

On introduit 300 mg du produit obtenu à l'exemple 2 dans le mélange de 15 cm³ d'éthanol et 0,1 cm³ de KOH. L'agitation est maintenue pendant 1 nuit à température ambiante. La suspension est alors versée sur de l'eau, puis acidifiée jusqu'à pH 2 par addition d'acide chlorhydrique N, le solide blanc est finalement essoré, lavé abondamment à l'eau puis séché.

### Purification

Le produit brut est empâté dans 20 cm³ d'acétate d'éthyle, puis dans 10 cm³ d'éthanol bouillant. On essore, lave par 10 cm³ d'éthanol et on obtient 85 mg de produit attendu (solide blanc).

### Spectre IR : Nujol

| | |
|---|---|
| Absorption complexe région OH/NH | |
| C=O | 1720-1644-1630 cm⁻¹ ep |
| Aromatique | 1560 cm⁻¹ |
| Hétéroatome | 1546 cm⁻¹ |
| Amide II | 1520 - 1497 cm⁻¹ |

| Microanalyse | | | | |
|---|---|---|---|---|
| | C | H | N | S |
| % calculé | 58,11 | 4,52 | 9,68 | 11,08 |
| % trouvé | 57,9 | 4,5 | 9,5 | 10,9 |

### EXEMPLE 4 : 4-(méthylthio) α-oxo 1-[(2'-(((((phénylméthyl) amino) carbonyl) amino) sulfonyl) (1,1'-biphényl) 4-yl) méthyl] 2-propyl 1H-imidazole 5-acétate d'éthyle

On introduit 3,6 g du produit obtenu à la préparation 3 dans 50 cm³ d'acétone puis en une fois 2 g de carbonate de potassium.

Le milieu est alors porté au reflux et on y ajoute goutte à goutte 1 cm³ d'isocyanate de benzyle, le reflux est maintenu durant environ 2 H.

La suspension jaune obtenue est versée sur 500 cm³ d'eau glacée puis on acidifie le milieu par additon d'acide chlorhydrique N. On essore, lave abondamment à l'eau, puis sèche à 50°C. Le produit brut obtenu est purifié d'abord par empâtage dans 50 cm³ d'éther isopropylique.

On obtient 4,2 g de produit (Pf : 170°C) dont 500 mg sont recristallisés dans 25 cm³ d'éthanol. On essore, sèche et obtient ainsi 300 mg de produit attendu.
Pf : 188°C.

### Spectre IR CHCl₃

| | |
|---|---|
| =C-NH | 3375 cm⁻¹ |
| C=O | 1714 (F) 1621 cm⁻¹ |
| Système conjugué + aromatique + Amide II | 1537-1495 cm⁻¹ |

| Microanalyse | | | | |
|---|---|---|---|---|
| | C | H | N | S |
| % calculé | 60,54 | 5,39 | 8,82 | 10,1 |
| % trouvé | 60,5 | 5,3 | 8,7 | 10,0 |

### EXEMPLE 5 : acide 4-(méthylthio) 1-((2'-(((((phénylméthyl) amino) carbonyl) amino) sulfonyl) (1,1'-biphényl) 4-yl) méthyl) 2-propyl alpha oxo-1H-imidazole 5-acétique

On introduit 300 mg du produit obtenu à l'exemple 4, en suspension dans 5 cm³ d'éthanol puis ajoute 5 cm³ de soude 2N.

L'agitation est maintenue environ 1 h à température ambiante. Le milieu est acidifié par addition d'acide chlorhydrique 2N. On observe la précipitation d'un solide jaune, celui-ci est essoré, lavé abondamment à l'eau puis séché à 50°C.

Le produit brut obtenu est repris par 20 cm³ d'éthanol bouillant, le solide blanc est essoré à chaud puis lavé par 2 x 10 cm³ d'éthanol. Après séchage à 50°C, on obtient 170 g de produit attendu. PF = 215°C.

### Spectre IR : Nujol

| | |
|---|---|
| Absorption générale OH/OH + absorption max | 3360-3350-3190 cm⁻¹ |
| C=O | 1720-1640-1610 cm⁻¹ |
| Système conjugué + Amide II + Φ | 1558-1538-1510-1498 cm⁻¹ |

| Microanalyse | | | | |
|---|---|---|---|---|
| | C | H | N | S |
| % calculé | 59,39 | 4,98 | 9,25 | 10,56 |
| % trouvé | 59,2 | 4,9 | 9,4 | 10,4 |

### EXEMPLE 6 : 2-butyl 1-[(2'-(((((cyclohexylméthyl) amino) carbonyl) amino) sulfonyl) (1,1'-biphényl) 4-yl) méthyl] 4-(méthylthio) α-oxo 1H-imidazole 5-éthanethioate de S-méthyle

On introduit 3,20 mg du produit obtenu à la préparation 4 dans 100 ml d'acétone anhydre et 1,78 g de carbonate de potassium K₂CO₃. L'ensemble est porté au reflux et 1,1 cm³ de cyclohexylméthyle isocyanate sont introduits. Après environ 1 H d'agitation, la solution est refroidie à température ambiante, hydrolysée avec une solution aqueuse saturée de NH₄Cl (150 ml) amenée à pH ~ 4 à l'aide d'une solution HCl 1N puis extraite trois fois avec CH₂Cl₂, séchée, évaporée puis filtrée sur silice (éluant 50 CH₂Cl₂/50 AcOEt). On obtient ainsi 3,5 g de produit attendu (solide jaune). PF = 145°C.

### Spectre IR CHCl₃

| | |
|---|---|
| =C-NH | 3406-3375 cm⁻¹ |
| >=O | 1716-1670-1616 cm⁻¹ |
| Syst. conjugué + Aromatique + Amide II | 1598-1542-1492 cm⁻¹ |

### EXEMPLE 7 : acide 2-butyl 1-((2'-(((((cyclohexylméthyl) amino) carbonyl) amino) sulfonyl) (1,1'-biphényl) 4-yl) méthyl) 4-méthylthio alpha oxo-1H-imidazole 5-acétique

On introduit 2,8 g du produit obtenu à l'exemple 6, dans 100 ml d'éthanol. On ajoute ensuite à température ambiante 15 ml de NaOH 2N. L'éthanol est alors évaporé et 50 ml d'eau sont ajoutés. La phase aqueuse est extraite trois fois avec de l'éther, puis placée à 0°C où elle est acidifiée par HCl 1N. On filtre, sèche, recristallise dans EtOH anhydre à chaud et obtient 2 g de produit attendu.

| Microanalyse C₃₁H₃₈N₄O₆S₂ 1H₂O | | | | |
|---|---|---|---|---|
| | C | H | N | S |
| Calculé avec 1 molécule H₂O | 57,8 | 6,05 | 8,7 | 9,9 |
| Trouvé | 58,0 | 5,9 | 8,6 | 9,9 |
| Calculé sans molécule H₂O | 59,42 | 6,07 | 8,95 | 10,22 |

### EXEMPLE 8 : 4'-((2-butyl 5-((méthylsulfinyl) acétyl) 4-(méthylthio) 1H-imidazol 1-yl) méthyl) N-((propylamino) carbonyl) (1,1'-biphényl) 2-sulfonamide

On introduit 3,52 g de NaH à 50 % dans l'huile, on débarrasse le NaH de son huile par 3 lavages successifs au pentane. Puis on sèche. On ajoute 42 cm³ de DMSO anhydre, on porte le mélange à 75°C, pendant environ 1 heure, puis refroidit le mélange à 0°C et on ajoute à l'anion du DMSO formé 40 cm³ de THF anhydre et goutte à goutte 12 g de 2-butyl H- (méthylthio) 1-[[2'-((((propylamino) carbonyl) amino) sulfonyl) (1,1'-biphényl) 4-yl] méthyl] 1H-imidazole 5-carboxylate d'éthyle, dissous dans 80 cm³ de THF anhydre. On laisse remonter à la température ambiante et laisse sous agitation pendant environ 1 heure. Puis le mélange réactionnel est versé dans 400 cm³ d'eau distillée et acidifié jusqu'au pH 2, avec de l'acide chlorhydrique 2N. On extrait par 3 x 200 cm³ de chlorure de méthylène et lave la phase organique par 1 x 200 cm³ de solution saturée de NH₄Cl et 2 x 200 cm³ d'eau distillée.

La phase organique est séchée, filtrée et concentrée à sec. On empâte avec de l'éther isopropylique puis purifie sur silice avec pour éluant CH₂Cl₂-méthanol (95-5), empâte dans l'éther iso et obtient 8,93 g de produit attendu (poudre blanche).

| Microanalyse | | | | | |
|---|---|---|---|---|---|
| | C | H | N | S | O |
| % calculé | 55,61 | 5,99 | 9,27 | 15,9 | 13,23 |
| % trouvé | 55,5 | 5,9 | 9,2 | 16,0 | - |

### Spectre IR : CHCl₃

| | |
|---|---|
| NH | ~ 3370 cm⁻¹ associé |
| >=O | 1710-1635 cm⁻¹ |

### EXEMPLE 9 : 4'-((2-butyl 5-(2-(méthylsulfinyl) acétyl) 4-(méthylthio) 1H-imidazol 1-yl) méthyl) N-(((phénylméthyl) amino) carbonyl) (1,1'-biphényl) 2-sulfonamide

On introduït 400 mg du produit obtenu à la préparation 4 stade A, 162 mg de K₂CO₃ et 10 cm³ d'acétone anhydre. Le mélange est porté à reflux d'acétone et on y ajoute goutte à goutte 0,156 ml d'isocyanate de benzyle. On laisse sous agitation environ 1 h, évapore l'acétone et reprend le résidu par NH₄Cl jusqu'à pH 6-7. On extrait par 3 x 50 cm³ de CH₂Cl₂ et lave par 2 x 50 cm³ d'eau, puis sèche, filtre, et concentre à sec. On reprend dans du CH₂Cl₂ et purifie sur silice avec pour éluant CH₂Cl₂-méthanol (93-7). On récupère 240 mg de produit attendu.

### Spectre IR : CHCl₃

| | |
|---|---|
| =C-NH- | ~ 3375 cm⁻¹ |
| >=O | 1710-1637 cm⁻¹ |
| Aromatique + Amide II | 1541-1498 cm⁻¹ |
| >S-->O | 1044 cm⁻¹ |

### EXEMPLE 10 : acide 2-butyl 4-(méthylthio) alpha oxo 1-((2'-(((((phénylméthyl) amino) carbonyl) amino) sulfonyl) (1,1'-biphényl) 4-yl) méthyl) 1-H-imidazole 5-acétique

### Stade A : 2-butyl 4-(méthylthio) alpha oxo 1-[(2'-(((((phénylméthyl) amino) carbonyl) amino) sulfonyl) (1,1'-biphényl) 4-yl) methyl] 1-H-imidazole 5-étanethioate de 5-méthyle

On introduit 1,4 g du produit obtenu à la préparation 4, dans 560 mg de K₂CO₃, dissous dans 35 cm³ d'acétone, le mélange est porté à reflux d'acétone et on y ajoute 500 µl d'isocyanate de benzyle. On laisse agiter environ 1/2 heure, l'acétone est évaporée, on reprend le résidu par 30 cm³ d'eau que l'on amène à pH 6-7 par une solution de chlorure d'ammonium saturée. On extrait de la phase aqueuse par 3 x 50 cm³ de chlorure de méthylène, et la phase organique est lavée par 2 x 50 cm³ d'eau distillée puis séchée, filtrée et concentrée à sec. Après empâtement dans de l'éther éthylique, on obtient 1,67 g de poudre jaune que l'on recristallise par 50 cm³ d'acétate d'éthyle à chaud, filtre, concentre au minimum d'acétate d'éthyle et laisse cristalliser à température ambiante. On essore les cristaux et sèche. On obtient 1,02 g de produit attendu (cristaux jaune) PF = 145°C.

### Stade B : acide 2-butyl 4-(méthylthio) alpha oxo 1- ((2'-(((((phénylméthyl) amino) carbonyl) amino) sulfonyl) (1,1'-biphényl) 4-yl) méthyl) 1-H-imidazole 5-acétique

On introduit 990 mg du produit obtenu au stade A ci-dessus, dissous dans 20 cm³ d'éthanol et 20 cm³ de NaOH 2N. On laisse sous agitation à température ambiante environ 1/4 d'heure, puis évapore l'éthanol et reprend le résidu par 50 cm³ d'eau distillée, la solution est acidifiée jusqu'à pH 6-7 avec HCl 2N. On essore le précipité formé, lave avec de l'eau distillée puis sèche à 80°C. Pour purifier, on dissout à chaud dans le minimum d'éthanol, filtre et on laisse recristalliser à température ambiante. Les cristaux sont essorés et séchés et on obtient 473 mg de produit attendu.
PF = 208°C.

### Spectre IR : Nujol

| | |
|---|---|
| >=O | 1720-1620 cm⁻¹ |
| Aromatique Hétéroaromatique Amide II | 1542-1595-1495 cm⁻¹ |

### EXEMPLE 12 : 2-butyl 4-(méthylthio) béta oxo 1-((2'-(((((propylamino) carbonyl) amino) sulfonyl) (1,1'-biphényl) 4-yl) méthyl) 1-H-imidazole 5-propanoate d'éthyle

### Stade A: Préparation du malonate d'éthyle et de potassium

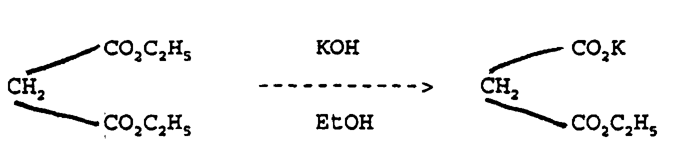

D'après la méthode de Strube R. E. (Organic Syntheses Coll. Vol IV 1963, 417) modifiée G. Bram et M. Viekas (Bull. Soc. Chim. 1964, 945), on dissout 80 g de malonate d'éthyle dans 50 ml d'éthanol 100 et refroidit. On ajoute ensuite goutte à goutte, en environ deux heures, une solution froide de 28 g de potasse dans 200 ml d'éthanol 100. Puis on laisse sous agitation pendant une nuit.

On essore ensuite le précipité et le rince à l'éther. On sèche et obtient 61 g du sel de potassium attendu (cristaux blancs).

### Stade B : Préparation du chlorure d'acide.

A une suspension de 1,088 g d'acide 2-butyl H-(méthylthio) 1- [[2'- ((((propylamino) carbonyl) amino) sulfonyl) (1,1'-biphényl) 4-yl] méthyl] 1H-imidazole 5-carboxylique, préparé ainsi qu'il est indiqué dans EP 0503162 dans 44 ml de toluène anhydre on ajoute 2,2 ml de chlorure de thionyle redistillé.

On agite environ une heure à température ambiante, puis une nuit à 55°C. On évapore ensuite le toluène et le chlorure de thionyle en excès, reprend avec 30 ml de toluène et évapore à nouveau (3 fois) puis on sèche pendant environ 2 heures à 50°C.

### Stade C : Condensation

On introduit 714 mg de produit obtenu au stade A ci-dessus dans 12 ml d'acétate d'éthyle. On refroidit à environ 4 ou 5°C et ajoute 1,062 g de triéthylamine puis 656 mg de chlorure de magnésium anhydre.

Le mélange est ensuite porté progressivement à 35°C (± 1°C) et maintenu à cette température pendant environ 6 h sous agitation.

On refroidit à nouveau à 2°C environ puis ajoute le chlorure d'acide obtenu au stade B ci-dessus, dans 20 ml de THF anhydre en environ 25 minutes. On rince avec 2 fois 3 ml de THF anhydre puis laisse revenir à température ambiante puis sous agitation pendant la nuit.

On refroidit à une température inférieure à 5°C puis on additionne goutte à goutte 14 ml d'HCl N. On décante, réextrait à l'acétate d'éthyle 3 fois, lave à l'eau, et à l'eau salée, sèche et évapore les solvants.

On purifie sur silice avec pour éluant CH₂Cl₂-Acétate d'éthyle (8-2) + 0,5 % méthanol et obtient 757 mg de produit attendu. PF 193-195°C.

### Spectre IR : CHCl₃

| | |
|---|---|
| =C-NH- | 3375 cm⁻¹ complexe |
| >=O | 1730 - 1706 cm⁻¹ 1632 cm⁻¹ (cétone conjugué) |
| | |
| Amide II | 1540 cm⁻¹ |

### EXEMPLE 13 : 2-butyl 4-(méthylthio) 1-((2'-(((((propylamino) carbonyl) amine) sulfonyl) (1,1'-biphényl) 4-yl) méthyl) N-(1H-tétrazol 5-yl) 1H-imidazole 5-carboxamide

On introduit 900 mg de l'acide 2-butyl H-(méthylthio) 1-[[2'-((((propylamino) carbonyl) amino) sulfonyl) (1,1'-biphényl) 4-yl] méthyl] 1H-imidazole 5-carboxylique, décrit dans EP 0503162, recristallisé dans l'isopropanol puis mis en suspension dans 10 ml de toluène. On ajoute 1 ml de chlorure de thionyle SOCl₂ et agite 1 h à température ambiante, puis 15 h à 55°C. Le solvant est alors évaporé. On reprend à plusieurs reprises au toluène pour éliminer tout le SOCl₂.

On met en suspension dans 3 ml de THF anhydre et ajoute une solution de 20 ml de THF anhydre contenant 510 mg d'aminotétrazole hydrate (commercial) et 210 µl de pyridine puis on agite pendant environ 20 h à température ambiante. La solution est alors hydrolysée par une solution saturée de NH₄Cl et extraite avec CH₂Cl₂. On sèche, évapore et purifie par chromatographie sur silice avec pour éluant CH₂Cl₂ 10 % MeOH, puis 50 AcOEt/45 Acétone/5 CH₂Cl₂ et ajout progressif de MeOH jusqu'à 10 % dans l'éluant.

On filtre, sèche et obtient 180 mg de produit attendu (solide blanc). PF = 130°C.

### Spectre IR : CHCl₃

| | |
|---|---|
| Région OH-NH absorption forte avec NH | |
| >=O | 1697-1640 cm⁻¹ |
| Amide II | 1570 cm⁻¹ |
| C=N | |
| NH₂ | |

### EXEMPLE 15 : 4-(méthylthio) 1-((2'-(((((phénylméthyl) amino) carbonyl) amino) sulfonyl) (1,1'-biphényl) 4-yl) méthyl) 2-propyl N-(2H-tétrazol 5-yl) 1H-imidazole 5-carboxamide

### Stade A : 4-(méthylthio) 1-[(2'-(((((phénylméthyl) amino) carbonyl) amino) sulfonyl) (1,1'-biphényl) 4-yl) méthyl] 2-propyl 1H-imidazole 5-carboxylate d'éthyle

On introduit 2 g du produit obtenu à la préparation 5, dissous dans 25 ml d'acétone anhydre et 1,2 g de carbonate de potassium. On porte au reflux et ajoute 740 µl de benzylisocyanate. Après environ 2 h d'agitation, on refroidit à température ambiante, hydrolyse par une solution aqueuse saturée de NH₄Cl puis extrait au chlorure de méthylène. On sèche, recristallise dans l'éther, filtre et obtient 1,9 g de produit attendu.

### Stade B : acide 4-(méthylthio) 1-[(2'-(((((phénylméthyl) amino) carbonyl) amino) sulfonyl) (1,1'-biphényl) 4-yl) méthyl] 2-propyl 1H-imidazole 5-carboxylique

On introduit 1,8 g du produit obtenu au stade A ci-dessus, dans 10 ml d'éthanol et 10 ml de soude 2N puis laisse sous agitation à température ambiante pendant environ 36 heures. L'éthanol est ensuite évaporé et, après ajoute de 25 ml d'eau, la phase aqueuse est extraite avec de l'éther, puis filtrée, placée à 0°C et acidifiée lentement jusqu'à pH 15 avec HCl 1N. On filtre et sèche et obtient 1,4 g de produit attendu.

### Spectre IR : CHCl₃

| | |
|---|---|
| Acide d'après la région OH | |
| =C-NH | 3480 cm⁻¹ |
| >=O | 1706 - 1690 cm⁻¹ complexe |
| Aromatique | 1539 - 1521 - 1500 cm⁻¹ |
| Hétéroaromatique | |
| Amide II | |

### Stade C : 4-(méthylthio) 1-((2'-(((((phénylméthyl) amino) carbonyl) amino) sulfonyl) (1,1'-biphényl) 4-yl) méthyl) 2-propyl N-(2H-tétrazol 5-yl) 1H-imidazole 5-carboxamide

On introduit 270 mg du produit obtenu au stade B ci-dessus dans 5 ml de toluène et 300 µl de SOCl₂ (chlorure de thionyle) puis met sous agitation pendant environ 1 h à température ambiante, puis environ 15 h à 55°C. Le solvant est alors évaporé, SOCl₂ est éliminé sous vide. Le chlorure d'acide obtenu est mis en suspension dans 3 ml de THF anhydre puis ajouté à une solution de THF anhydre 10 ml, contenant 150 mg d'aminotétrazole et 150 µl de pyridine. On agite pendant environ 20 h à température ambiante. La solution est ensuite hydrolysée par une solution aqueuse saturée de NH₄Cl et extraite 3 fois au CH₂Cl₂. Après séchage et évaporation du solvant, on effectue une séparation de l'amide recherchée de l'acide de départ par HPLC préparative en phase inverse avec pour éluant MeOH-H₂O (60-40) avec NH₄⁺ ACO⁻ 0,05 M. Le produit est recristallisé dans MeOH. On récupère ainsi 70 mg de produit attendu. Tf = 228°C.

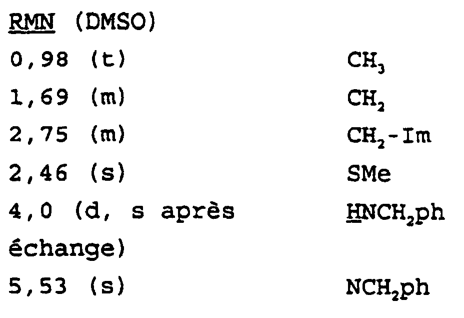

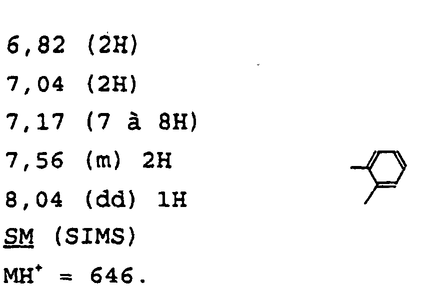

### EXEMPLE 16 : 2-butyl 1-((2'-(((((cyclohexylméthyl) amino) carbonyl) amino) sulfonyl) (1,1'-biphényl) 4-yl) méthyl) 4-(méthylthio) alpha-oxo 1H-imidazole 5-acétamide

On introduit 250 mg du produit de l'exemple 6 dans 5 ml d'EtOH anhydre et 90 mg d'acétate d'ammonium NH₄OAc. On porte au reflux une nuit, hydrolyse avec une solution NH₄Cl aqueuse et extrait avec AcOEt. Après séchage et évaporation, on purifie sur silice avec pour éluant AcOEt-CH₂Cl₂ (50-50) puis recristallise avec CH₂Cl₂ minimum éther iso et obtient 65 mg de produit attendu.

### Spectre IR : CHCl₃

| | |
|---|---|
| NH/NH₂ | 3510 - 3400 cm⁻¹ |
| >=O | 1708 - 1690 - 1622 cm⁻¹ |
| Système conjugué | 1560 - 1552 - 1490 cm⁻¹ |
| Aromatique | |
| NH, NH₂ | |

### EXEMPLE 17 : Acide 2-butyl 1-((2'-(((((cyclohexylméthyl) amine) carbonyl) amino) sulfonyl) (1,1'-biphényl) 4-yl) méthyl) alpha-((2,4-dinitrophényl) hydrazono) 4-(méthylthio) 1H-imidazole 5-acétique

On procède comme à l'exemple 16, en introduisant 250 mg du produit de l'exemple 7, dans 10 ml d'éthanol absolu et 150 mg de 2,4-dinitrophénylhydrazine. On porte au reflux pendant environ 5 heures sous agitation, et obtient 160 mg de produit attendu. Tf = 254°C.

### Spectre IR : Nujol

| | |
|---|---|
| Absorption générale OH/NH | |
| >=O | 1706 - 1668 - 1652 cm⁻¹ |
| C=N | |
| Système conjugué + Aromatique + Amide II + NO₂ | 1618 - 1590 - 1518 - 1501 cm⁻¹ |

### EXEMPLE 18 : Acide 2-butyl alpha-((2,4-dinitrophényl) hydrazono) 4-(méthylthio) 1-((2'-(((((phénylméthyl) amino) carbonyl) amino) sulfonyl) (1,1'-biphényl) 4-yl) méthyl) 1H-imidazole 5-acétique

On introduit 100 mg du produit de l'exemple 10 dans 5 ml d'EtOH et 48 mg de 2,4-dinitrophénylhydrazine. On porte au reflux environ 6 h sous agitation, puis refroidit, filtre et obtient 50 mg de produit attendu. Tf = 252°C.

| Microanalyse : C₃₇H₃₆N₈O₉S₂ M=543 | | | | |
|---|---|---|---|---|
| | C | H | N | S |
| % calculé | 55,5 | 4,5 | 14,0 | 8,0 |
| % trouvé | 55,3 | 4,3 | 13,7 | 8,3 |

### EXEMPLE 20 : 4'-((4-(méthylthio) 5-(2-(phénylsulfinyl) acétyl) 2-propyl 1H-imidazol-1-yl) méthyl) N-(((phénylméthyl) amino) carbonyl) (1,1'-biphenyl) 2-sulfonamide.

On refroidit à 0°C 203 mg de phénylméthylsulfoxyde et 440 mg d'ester obtenu comme à l'exemple 15 stade A. On ajoute 3,6 cm³ de lithium bis (triméthylsilyl) amide pendant 1 minute. On agite 15 minutes à 0°C, laisse revenir à température ambiante, maintient 15 minutes, ajoute 10 cm³ d'une solution aqueuse saturée en chlorure d'ammonium, ajoute de l'acide chlorhydrique N jusqu'à pH 2/3 puis extrait à l'acétate d'éthyle. On lave à l'eau les extraits organiques, les sèche et évapore le solvant. Après chromatographie du résidu sur silice (éluant : CH₂Cl₂-MeOH 90-10), on obtient 356 mg de produit brut que l'on solubilise dans le chlorure de méthylène puis ajoute de l'éther isopropylique, refroidit à -78°C, dissout le précipité dans l'acétonitrile chaud et recueille 250 mg de produit attendu cristallisé.

### Spectre IR : CHCl₃

| | |
|---|---|
| NH | 3300 cm⁻¹ |
| C=O | 1708 - 1636 cm⁻¹ |
| Système conjugué | |
| Aromatique | 1587 - 1540 - 1497 cm⁻¹ |
| amide II | |
| S - O | 1038 cm⁻¹ |

En opérant comme à l'exemple 15 stades B et C ou à l'exemple à partir du produit obtenu à l'exemple 15A (ou d'analogues préparés de manière identique) et des réactifs appropriés, on a préparé les produits suivants :

### EXEMPLE 21 : 2-butyl 4-(méthylthio) béta-oxo-1-((2'-(((((phenylméthyl) amino) carbonyl) amino) sulfonyl) (1,1'-biphényl) 4-yl) méthyl) 1H-imidazole 5-propanoate d'éthyle.

F = 173-175°C.
rf = 0,35 (CH₂Cl₂-AcOEt).

### EXEMPLE 22 : 2-butyl 4-(méthylthio) béta-oxo-1-((2'-(((((phénylméthyl) amino) carbonyl) amino) sulfonyl) (1,1'-biphényl) 4-yl) méthyl) 1H-imidazole 5-propanoate de méthyle.

F = 170°C.
rf # 0,85 (CH₂Cl₂-MeOH 90-10).

### EXEMPLE 23 : 2-butyl 1-((2'-(((((cyclohexylméthyl) amino) carbonyl) amino) sulfonyl) (1,1'-biphényl) 4-yl) méthyl) 4-(méhtylthio) bêta-oxo 1H-imidazole 5-propanoate de méthyle.

F ≈ 110°C.
rf # 0,67 (CH₂Cl₂-MeOH 90-10).

### EXEMPLE 25 : 4'-((4-((difluorométhyl) thio) 5-((phénylsulfiny) acétyl) 2-propyl 1H-imidazol 1-yl) méthyl) N-(((phénylméthyl) amino) carbonyl) (1,1'-biphényl) 2-sulfonamide.

F ≈ 103°C.
rf = 0,25 (AcOEt-cyclohexanne 8-2).

### EXEMPLE 26 : 4-(méthylthio) 1-((2'-(((((phénylméthyl) amino) carbonyl) amino) sulfonyl) (1,1'-biphényl) 4-yl) méthyl) 2-propyl 1H-imidazole 5-carboxamide.

F ≈ 112°C.

### EXEMPLE 27 : 2-butyl 4-(méthylthio) 1-((2'-((((propylamino) carbonyl) amino) sulfonyl) (1,1'-biphényl) 4-yl) méthyl) 1H-imidazole 5-carboxamide.

F ≈ 90°C.
rf = 0,45 (AcOEt).

### EXEMPLE 28 : 4'-((5-(2-(méthylsulfinyl) acétyl) 4-(méthylthio) 2-propyl 1H-imidazol 1-yl) méthyl) N-(((phénylméthyl) amino) carbonyl) (1,1'-biphényl) 2-sulfonamide.

F = 100°C.
rf = 0,5 (CH₂Cl₂-MeOH 95-5).

### EXEMPLE 29 : 4'-((2-butyl 4-(méthylthio) 5-((phénylsulfonyl) acétyl) 1H-imidazol 1-yl) méthyl) N-(((phénylméthyl) amino) carbonyl) (1,1'-biphényl) 2-sulfonamide.

F = 185°C.

### EXEMPLE 30 : 4'-((5-(((4-méthoxyphényl) sulfonyl) acétyl) 4-(méthylthio) 2-propyl 1H-imidazol-1-yl) méthyl) N-(((phénylméthyl) amino) carbonyl) (1,1'-biphényl) 2-sulfonamide.

F = 158°C.

### EXEMPLE 31 : 4'-((5-(((4-méthylphényl) sulfinyl) acétyl) 4-(méthylthio) 2-propyl 1H-imidazol-1-yl) méthyl) N-(((phénylméthyl) amino) carbonyl) (1,1'-biphényl) 2-sulfonamide.

F ≈ 115°C.

### EXEMPLE 32 : (S) 4'-((5-(((4-méthylphényl) sulfinyl) acétyl) 4-(méthylthio) 2-propyl 1H-imidazol-1-yl) méthyl) N-(((phénylméthyl) amino) carbonyl) (1,1'-biphényl) 2-sulfonamide.

F = 109°C (c = 0,5 CHCl₃).
rf = 0,40 (AcOEt-hexane 80-20).

### EXEMPLE 34 : 4'-((4-(méthylthio) 5-((phénylsulfinyl) acétyl) 2-propyl 1H-imidazol-1-yl) méthyl) N-(((2-thiénylméthyl) amino) carbonyl) (1,1'-biphényl) 2-sulfonamide.

F = 200°C.
rf = 0,37 (CH₂Cl₂-AcOEt 60-40).

### EXEMPLE 35 : 4'-((2-butyl 5-(2-(méthylsulfonyl) acétyl) 4-(méthylthio) 1H-imidazol-1-yl) méthyl) N-((propylamino) carbonyl) (1,1'-biphényl) 2-sulfonamide.

F = 159°C.
rf = 0,17 (CH₂Cl₂-AcOEt 80-20).

### EXEMPLE 36 : 4'-((2-butyl 4-(méthylthio) 5-(2-(phénylsulfinyl) acétyl) 1H-imidazol-1-yl) méthyl) N-(((phénylméthyl) amino) carbonyl) (1,1'-biphényl) 2-sulfonamide.

F = 130°C.

### EXEMPLE 37 : 4'-((2-butyl 5-(2-(méthylsulfonyl) acétyl) 4-(méthylthio) 1H-imidazol-1-yl) méthyl) N-(((phénylméthyl) amino) carbonyl) (1,1'-biphényl) 2-sulfonamide.

F = 116°C.
rf = 0,43 (CH₂Cl₂-MeOH 96-4).

### EXEMPLE 40 : 4'-((2-butyl 4-(méthylthio) 5-(2-phénylsulfonyl) acétyl) 1H-imidazol 1-yl) méthyl) N-(((phénylméthyl) amino) carbonyl) (1,1'-biphényl) 2-sulfonamide.

F = 191°C.
rf = 0,16 (AcOEt-cyclohexane 5-5).

### EXEMPLE 41 : 4'-((2-butyl 5-(2-((4-fluorophényl) sulfonyl) acétyl) 4-(méthylthio) 1H-imidazol 1-yl) méthyl) N-(((phénylméthyl) amino) carbonyl) (1,1'-biphényl) 2-sulfonamide.

F =129°C.
rf = 0,3 (AcOEt-cyclohexane 5-5).

### EXEMPLE 42 : 4'-((2-butyl 4-((difluorométhyl) thio) 5-((phénylsulfinyl) acétyl) 1H-imidazol 1-yl) méthyl) N-(((phénylméthyl) amino) carbonyl) (1,1'-biphényl) 2-sulfonamide.

F ≈ 95-100°C.

### EXEMPLE 43 : 4'-((2-butyl 4-((difluorométhyl) thio) 5-((phénylsulfonyl) acétyl) 1H-imidazol 1-yl) méthyl) N-(((phénylméthyl) amino) carbonyl) (1,1'-biphényl) 2-sulfonamide.

F ≈ 190°C.

### EXEMPLE 44 : 4'-((2-éthyl 5-((méthylsulfonyl) acétyl) 4-méthylthio) 1H-imidazol 1-yl) méthyl) N-(((phénylméthyl) amino) carbonyl) (1,1'-biphényl) 2-sulfonamide.

F = 180°C.
rf # 0,37 (CH₂Cl₂-AcOEt-MeOH 6-4-0,5).

### EXEMPLE 45 : 4'-((2-éthyl 4-(méthylthio) 5-((phénylsulfinyl) acétyl) 1H-imidazol 1-yl) méthyl) N-(((phénylméthyl) amino) carbonyl) (1,1'-biphényl) 2-sulfonamide.

F = 228°C.
rf # 0,3 (AcOEt-hexane 80-20).

### EXEMPLE 46 : (±) N-(((cyclopentylméthyl) amino) carbonyl) 4'-((4-(méthylthio) 5-((phénylsulfinyl) acétyl) 2-propyl 1H-imidazol 1-yl) méthyl) (1,1'-biphényl) 2-sulfonamide.

F ≈ 106°C.
rf = 0,5 (CH₂Cl₂-AcOEt 6-4).

### EXEMPLE 47 : (±) N-(4'-((4-(méthylthio) 5-((phénylsulfinyl) acétyl)2-propyl 1H-imidazol 1-yl) méthyl) (1,1'-biphényl-2-yl) sulfonyl) cyclopentanepropanamide.

F ≈ 95°C.
rf = 0,7 (CH₂Cl₂-AcOEt 6-4).

### EXEMPLE 48 : (±) N-(((cyclohexylméthyl) amino) carbonyl) 4'-((4-(méthylthio) 5-((phénylsulfinyl) acétyl) 2-propyl 1H-imidazol 1-yl) méthyl) (1,1'-biphényl) 2-sulfonamide.

F ≈ 90/95°C.
rf = 0,4 (CH₂Cl₂-AcOEt 8-2).

### EXEMPLE 51 : N-(((cyclohexylméthyl) amino) carbonyl) 4'-((2-éthylthio) 5-(2-(méthylsulfinyl) acétyl) 4-(méthylthio) 1H-imidazol 1-yl) méthyl) (1,1'-biphényl) 2-sulfonamide.

F ≈ 180°C.

### EXEMPLE 52 : 2-butyl 4-(méthylthio) N-pentyl 1-((2'-(((((phénylméthyl) amino) carbonyl) amino) sulfonyl) (1,1'-biphényl) 4-yl) méthyl) 1H-imidazol 5-carboxamide.

F = 138°C.
rf = 0,25 (AcOEt-cyclohexane 5-5).

### EXEMPLE 55 : 2-éthyl 4-(méthylthio) 1-((2'-(((((phénylméthyl) amino) carbonyl) amino) sulfonyl) (1,1'-biphényl) 4-yl) méthyl) 1H-imidazol 5-carboxamide.

F = 140°C.
rf # 0,22 (AcOEt-CH₂Cl₂-MeOH 6-4-0,5).

### EXEMPLE 56 : 2-butyl 4-(méthylthio) bêta-oxo 1-((2'-((((phénylméthoxy) carbonyl) amino) sulfonyl) (1,1'-biphényl) 4-yl) méthyl) 1H-imidazol 5-propanoate d'éthyle (sel de potassium).

F = 130°C.
rf # 0,5 (CH₂Cl₂-AcOEt 60-40).

### EXEMPLE 57 : 2-butyl 4-(méthylthio) bêta-oxo 1-((2'-((((phénylméthyl) carbonyl) amino) sulfonyl) (1,1'-biphényl) 4-yl) méthyl) 1H-imidazol 5-propanoate d'éthyle.

F = 110°C.
rf # 0,38 (CH₂Cl₂-MeOH 98-2).

En opérant comme à l'exemple 15 stade B, à partir de l'ester approprié, on a préparé les produits suivants :

### EXEMPLE 60 : Acide 4-(butylthio) alpha-oxo 1-((2'-(((((phénylméthyl) amino) carbonyl) amino) sulfonyl) (1,1'-biphényl) 4-yl) méthyl) 2-propyl 1H-imidazol 5-acétique.

F = 192°C.
rf = 0,36 (CH₂Cl₂-MeOH 80-20).

### EXEMPLE 61 : Acide 1-(((2'-(((((cyclohexylméthyl) amino) carbonyl) amino) sulfonyl) (1,1'-biphényl) 4-yl) méthyl) 2-éthyl 4-(méthylthio) alpha-oxo 1H-imidazol 5-acétique.

F = 190°C.
rf # 0,14 (CH₂Cl₂-MeOH 90-10).

Les produits suivants ont été obtenus selon des conditions opératoires indiquées ci-dessus.

### EXEMPLE 64 : 4'-((4-(méthylthio) 5-(2-(phénylthio) acétyl) 2-propyl 1H-imidazol 1-yl) méthyl) N-(((phénylméthyl) amino) carbonyl) (1,1'-biphenyl) 2-sulfonamide.

F = 178°C.
rf = 0,55 (CHCl₃-MeOH 95-5).

### EXEMPLE 66 de composition pharmaceutique.

On a préparé des comprimés répondant à la formule suivante :

| | |
|---|---|
| Produit de l'exemple 15 | 50 mg |
| Excipient pour un comprimé terminé à | 200 mg |
| (détail de l'excipient : lactose, talc, amidon, stéarate de magnésium). | |

### RESULTATS PHARMACOLOGIOUES

### 1 - Test sur le récepteur AT₁ de l'angiotensine II

On utilise une préparation membranaire fraîche obtenue à partir de foie de rat. Le tissu est broyé au polytron dans un tampon Tris 50 mM pH 7,4, le broyage est suivi de 3 centrifugations à 30 000 g 15' avec reprises intermédiaires des culots dans le tampon Tris pH 7,4.

Les derniers culots sont remis en suspension dans un tampon d'incubation (Tris 20 mM, NaCl 135 mM, KCl 10 mM, glucose S mM, MgCl₂ 135 mM, PMSF 0,3 mM, bacitracine 0,1 mM, lysozyme 0,1 %).

On répartit des fractions aliquotées de 1 ml dans des tubes en verre et ajoute de la ¹²⁵ I angiotensine II (25 000 DPM/tube) et le produit à étudier. (Le produit est d'abord testé à 3 x 10⁻⁵ M en triple). Lorsque le produit testé déplace de plus de 50 % la radioactivité liée spécifiquement au récepteur, il est testé à nouveau selon une gamme de 7 concentrations afin de déterminer la concentration qui inhibe de 50 % la radioactivité liée spécifiquement au récepteur. On détermine ainsi la concentration inhibitrice 50 %).

La liaison non spécifique est déterminée par addition du produit de l'exemple 94 du brevet européen 0253310, à 10⁻⁵M (en triple). On incube à 25°C pendant 150 minutes, remet au bain-marie à 0°C, 5 minutes, filtre sous vide, rince au tampon Tris pH 7,4 et compte la radioactivité en présence de scintillant solide.

Le résultat est exprimé directement en concentration inhibitrice 50 % (CI₅₀), c'est-à-dire en concentration de produit étudié, exprimée en nM, nécessaire pour déplacer 50 % de la radioactivité spécifique fixée sur le récepteur étudié.

### Résultats :

| Produit de l'exemple | Récepteur AT₁ CI₅₀ en nanomoles |
|---|---|
| 7 | 0,7 |
| 9 | 0,3 |
| 10 | 0,18 |
| 12 | 0,2 |
| 13 | 0,24 |
| 15 | 0,2 |
| 20 | 0,08 |
| 21 | 0,08 |
| 34 | 0,05 |
| 46 | 0,4 |
| 47 | 0,09 |

### 2) Test sur le récepteur AT₂ de l'angiotensine II

On utilise une préparation membranaire fraîche obtenue à partir d'utérus de lapine, prétraitées 4 jours auparavent par 50 µg d'estradiol administré par voie percutanée. Le tissu est broyé au polytron dans un tampon Tris 50 mM, pH 7,4 et le broyage est suivi de 3 centrifugations à 30 000 g 15 minutes, avec reprises intermédiaires des culots dans le tampon Tris pH 7,4.

Les derniers culots sont remis en suspension dans un tampon d'incubation (Tris 20 mM, NaCl 135 mM, KCl 10 mM, glucose 5 mM, MgCl₂, 6H₂O 10 mM, PMSF 0,3 mM, bacitracine 0,1 mM, lysocyme 0,1 ‰, pH 7,4).

L'homogénat obtenu est mis à préincuber 20 minutes à 25°C en présence de dithiotreitol 10 mM, puis ramené à 0°-4°C.

On répartit des fractions aliquotées de 1 ml dans des tubes en verre et ajoute de la ¹²⁵I angiotensine II (25 000 DPM/tube) et le produit à étudier. Le produit est d'abord testé à 3.10⁻⁵M en triple. Lorsque le produit testé déplace de plus de 50 % la radioactivité liée spécifiquement au récepteur, il est testé à nouveau selon une gamme de 7 concentrations afin de déterminer la concentration qui inhibe de 50 % la radioactivité liée spécifiquement au récepteur. On détermine ainsi la concentration inhibitrice 50 %.

La liaison spécifique est déterminée par addition de EXP 655 (= PD 123-177 de Warner-Lambert) à 10⁻⁵M en triple. On incube à 25°C pendant 150 minutes, remet au bain-marie à 0°C, 5 minutes, filtre sous vide, rince au tampon Tris pH 7,4 et compte la radioactivité en présence de scintillant solide.

Le résultat est exprimé directement en concentration inhibitrice 50 % (CI₅₀), c'est-à-dire en concentration de. produit étudié, exprimée en nM, nécessaire pour déplacer 50 % de la radioactivité spécifique fixée sur le récepteur étudié.

### Résultats :

| Produit de l'exemple | Récepteur AT₂ CI₅₀ en nanomoles |
|---|---|
| 7 | 6,8 |
| 9 | 15,0 |
| 10 | 38,0 |
| 12 | 83,0 |
| 13 | 24,0 |
| 15 | 2,0 |
| 20 | 0,8 |
| 21 | 1,0 |
| 34 | 2,2 |
| 46 | 2,5 |
| 47 | 2,09 |

### 3 - Test d'activité antagoniste de l'angiotensine II chez le rat démédullé

Des rats mâles Sprague-Dawley (250 à 350 g) sont anesthésiés par une injection intra-péritonéale de pentobarbital sodique (60 mg/kg). La pression artérielle diastolique est enregistrée grâce à un cathéter (PE50) hépariné introduit dans la carotide gauche de l'animal, et relié à un calculateur de pression (Gould, Pressure Processor) par l'intermédiaire d'un capteur de pression Gould.

Un cathéter est introduit dans la jugulaire droite de l'animal afin de permettre l'injection des molécules à étudier.

L'animal est placé sous respiration assistée. Une section bilatérale des nerfs vagues est effectuée. Le rat est alors démédullé.

Après une période de stabilisation suffisante, l'étude de l'antagonisme des molécules vis-à-vis de l'angiotensine II (Hypertensine, CIBA) est abordée de la façon suivante :
1 - Trois injections consécutives d'angiotensine II (0,75 microgrammes/kg) espacées de 15 minutes permettent d'obtenir une réponse pressive reproductible et stable.
2 - Tout en gardant une périodicité de 15 minutes pour l'administration d'angiotensine II, les molécules (0,01 à 10 mg/kg) sont injectées 5 minutes avant l'angiotensine II.
   Les effets presseurs de l'angiotensine II en présence de l'antagoniste sont exprimés en pourcentage des effets presseurs de l'angiotensine II administrée seule. La dose inhibitrice à 50 % (DI₅₀) de l'effet étudié est ainsi déterminée.
   Chaque animal est considéré comme son propre témoin.

### Résultats :

| Produit de l'exemple | DI₅₀ en mg/kg | |
|---|---|---|
| | IV | PO |
| 9 | 0,06 | 0,7 |
| 13 | 0,12 | |
| 20 | 0,04 | 0,8 |
| 21 | 0,08 | |

## Revendications

1. Produits de formule (I) : 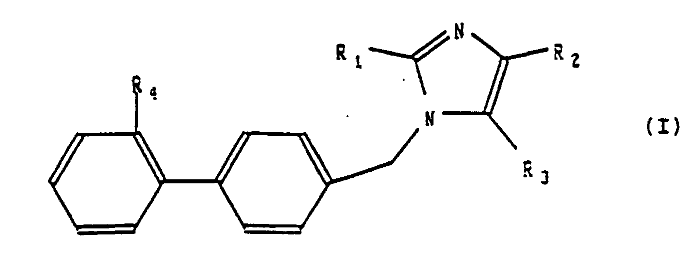 dans laquelle
R₁ représente un radical alkyle ou alkylthio, linéaire ou ramifié renfermant au plus 4 atomes de carbone ,
R₂ représente soit un radical alkylthio linéaire ou ramifié renfermant au plus 6 atomes de carbone et éventuellement substitué par un ou plusieurs atomes d'halogène, soit un radical 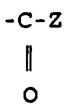 dans
lequel Z représente un radical carboxy libre, salifié par une base minérale ou organique ou estérifié par un radical alkyle linéaire ou ramifié renfermant au plus 6 atomes de carbone,
R₃ représente le radical 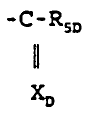 dans lequel
X_{D} représente un atome d'oxygène ou un radical 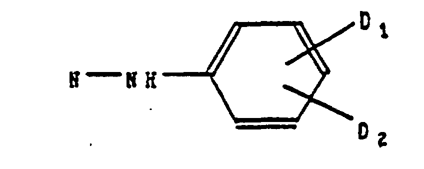 dans lequel D₁ et D₂, identiques ou différents, représentent un atome d'halogène ou un radical nitro,
et R_{5D} représente
i) le radical -CHY₁Y₂ dans lequel
Y₁ représente un atome d'hydrogène ou d'halogène
et Y₂ représente un radical carboxy libre, salifié par une base minérale ou organique, ou estérifié par un radical alkyle linéaire ou ramifié renfermant au plus 6 atomes de carbone ; les radicaux alkylthio, alkylsulfonyle et alkylsulfinyle, linéaires ou ramifiés renfermant au plus 6 atomes de carbone, ou les radicaux phénylthio, phénylsulfonyle ou phénylsulfinyle éventuellement substitués par un ou plusieurs atomes d'halogéne ou radicaux alkyle ou alcoxy linéaires ou ramifiés renfermant au plus 6 atomes de carbone,
ii) le radical 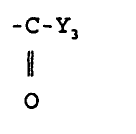 dans lequel Y₃ représente un radical hydroxyle, éventuellement salifié, alcoxy ou alkylthio, linéaire ou ramifié renfermant au plus 6 atomes de carbone,
iii) lorsque X_{D} représente un atome d'oxygène, R_{5D} représente un radical amino éventuellement substitué par un radical tétrazolyle ou par un ou deux radicaux alkyle, linéaires ou ramifiés renfermant au plus 6 atomes de carbone, eux-mêmes éventuellement substitués par un ou plusieurs radicaux phényle ou cyclohexyle,
R₄ représente le radical -SO₂-NH₂,
-SO₂-N=CH-N(CH₃)₂, 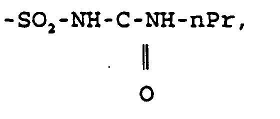 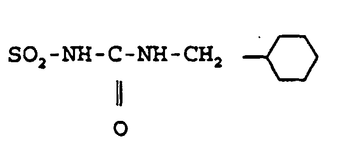 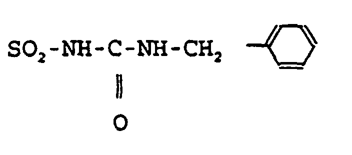 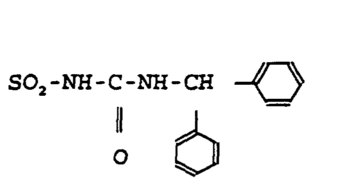 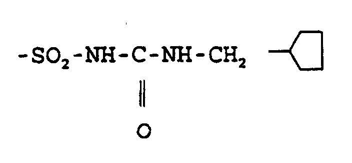 -SO₂-NH-CO₂Et, -SO₂-NH-CO₂H, SO₂-NH-CO₂Pr, 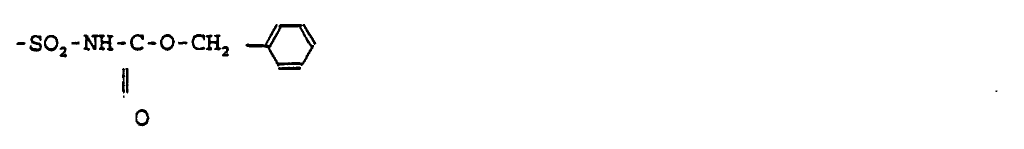 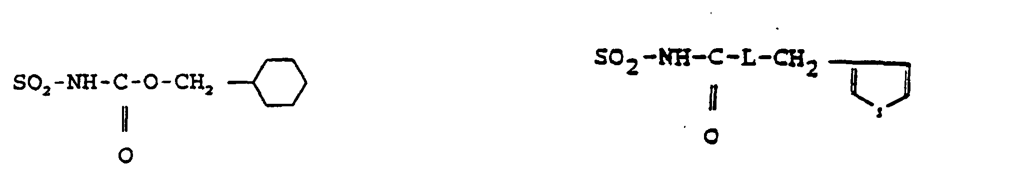 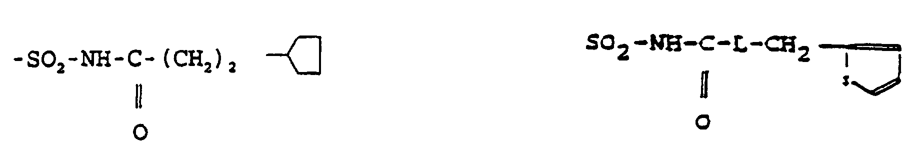 avec L représente -O- ou -NH-
étant entendu que les atomes de soufre dans les produits de formule (I) peuvent éventuellement être oxydés sous forme de sulfone ou de sulfoxyde,
lesdits produits de formule (I) étant sous toutes les formes isomères racémiques, énantiomères et diastéréoisomères possibles ainsi que les sels d'addition avec les acides minéraux et organiques ou avec les basés minérales et organiques desdits produits de formule (I).

2. Les produits tels que définis à la revendication 1, répondant aux formules suivantes :
- acide 2-butyl 1-((2'-(((((cyclohexylméthyl) amino) carbonyl) amino) sulfonyl) (1,1'-biphényl) 4-yl) méthyl) 4-méthylthio alpha-oxo-1H-imidazole 5-acétique,
- 2-butyl 4-(méthylthio) béta-oxo-1-((2'-(((((propylamino) carbonyl) amino) sulfonyl) (1,1'-biphényl) 4-yl) méthyl) 1H-imidazole 5-propanoate d'éthyle,
- 2-butyl 4-(méthylthio) 1-((2'-(((((propylamino) carbonyl) amino) sulfonyl) (1,1'-biphényl) 4-yl) méthyl) N-(1H-tétrazol-5-yl) 1H-imidazole 5-carboxamide,
- acide 2-butyl 4-(méthylthio) alpha-oxo-1-((2'-(((((phénylméthyl) amino) carbonyl) amino) sulfonyl) (1,1'-biphényl) 4-yl) méthyl) 1H-imidazole 5-acétique,
- 4'-((2-butyl 5-(2-(méthylsulfinyl) acétyl) 4-(méthylthio) 1H-imidazol-1-yl) méthyl) N-(((phénylméthyl) amino) carbonyl) (1,1'-biphényl) 2-sulfonamide,
- 4-(méthylthio) 1-((2'-(((((phénylméthyl) amino) carbonyl) amino) sulfonyl) (1,1'-biphényl) 4-yl) méthyl) 2-propyl-N-(2H-tétrazol 5-yl) 1H-imidazole 5-carboxamide,
- 4'-((4-(méthylthio) 5- (2- (phénylsulfinyl) acétyl) 2-propyl 1H-imidazol-1-yl) méthyl N-(((phénylméthyl) amino) carbonyl) (1,1'-biphényl) 2-sulfonamide,
- 2-butyl 4-(méthylthio) beta oxo 1 ((2' (((((phénylméthyl) amino) carbonyl) amino) sulfonyl) (1,1'-biphényl) 4-yl) méthyl) 1H-imidazol-5-propanoate d'éthyle,
- 4'-((2-butyl 5- (2- ((4-fluorophényl) sulfonyl) acétyl) 4-(méthylthio) 1H-imidazol 1-yl) méthyl) N-(((phénylméthyl) amino) carbonyl) (1,1'-biphényl) 2-sulfonamide,
- 4'-((4-(méthylthio) 5-((phénylsulfinyl) acétyl) 2-propyl 1H-imidazol-1-yl) méthyl) N-(((2-thiénylméthyl) amino) carbonyl) (1,1'-biphényl) 2-sulfonamide,
- (±) N-(((cyclopentylméthyl) amino) carbonyl) 4'-((4-(méthylthio) 5-((phénylsulfinyl) acétyl) 2-propyl 1H-imidazol-1-yl) méthyl) (1,1'-biphényl) 2-sulfonamide,
- (±) N- (4'-((4- (méthylthio) 5 ((phenylsulfinyl) acétyl) 2-propyl 1H-imidazol 1-yl) méthyl) (1,1'-biphényl-2-yl) sulfonyl) cyclopentanepropanamide.

3. Procédé de préparation des produits tels que définis à la revendication 1, **caractérisé en ce que** :
soit l'on soumet un composé de formule (II): 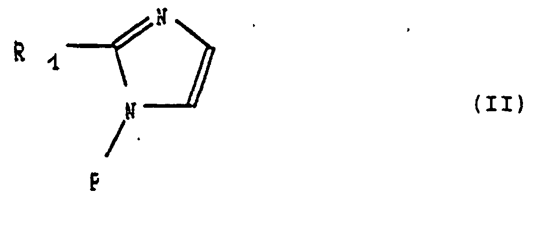 dans laquelle R₁ a la signification indiquée à la revendication 1, et P représente un groupement protecteur de l'atome d'azote,
à une réaction d'halogénation pour obtenir un composé de formule (III) : 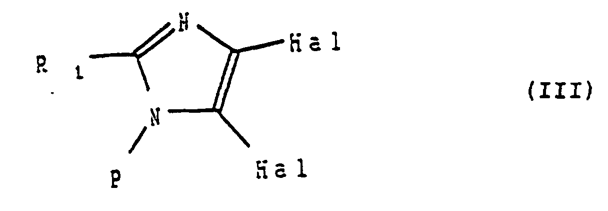 dans laquelle R₁ et P ont les significations indiquées ci-dessus et Hal représente un atome d'halogène, que l'on soumet à une réaction d'échange halogène-métal sur l'un des atomes d'halogène puis à une réaction avec un composé de formule (IV_{c}), (IV_{d}) ou (IVₑ) : 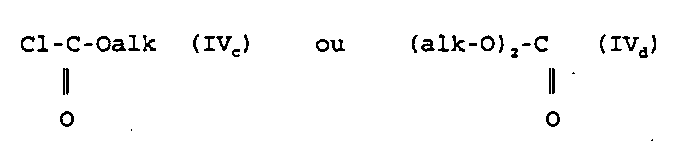 ou 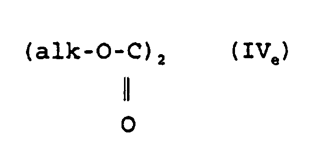 dans lesquelles alk représente un radical alkyle renfermant au plus 4 atomes de carbone,
pour obtenir le composé de formule (V) : 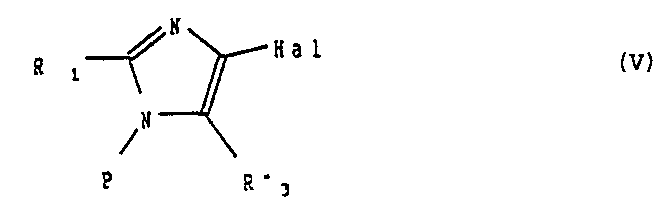 dans laquelle R₁, P et Hal ont les significations indiquées ci-dessus et R"₃ représente K-O-alk avec alk tel que défini ci-dessus et K représente le radical 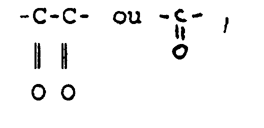 composé de formule (V) que l'on peut soumettre à une réaction d'échange halogène-métal sur l'atome d'halogène puis à une réaction avec un composé de formule (IV'ₐ), (IV'_{b}), (IV'_{c}), (IV'_{d}) ou (IV'ₑ) :
(-S-R")₂ (IV'ₐ)
ou
MeSO₂SR" (IV'_{b}) 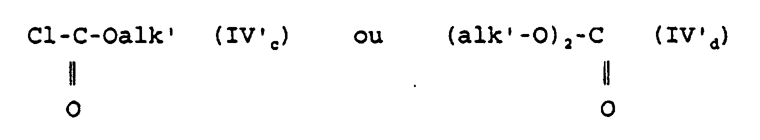
ou 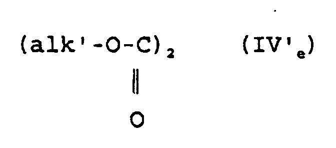 dans lesquelles R" représente un radical alkyle linéaire ou ramifié renfermant au plus 6 atomes de carbone, et alk' identique ou différent de alk, représente un radical alkyle renfermant au plus 4 atomes de carbone, pour obtenir le composé de formule (VII) : 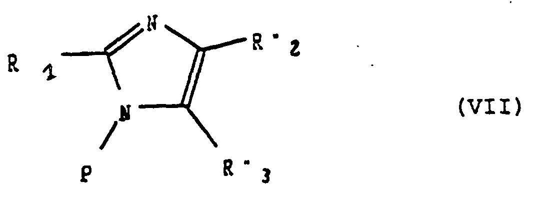 dans laquelle R₁, R"₃ et P ont les significations indiquées ci-dessus, et R"₂ représente -S-R" ou -K-Oalk' dans lesquelles R", alk' et K ont les significations indiquées ci-dessus,
produit de formule (VII) dont on libére la fonction amine bloquée par P tel que défini ci-dessus, puis fait réagir avec un composé de formule (VIII) : 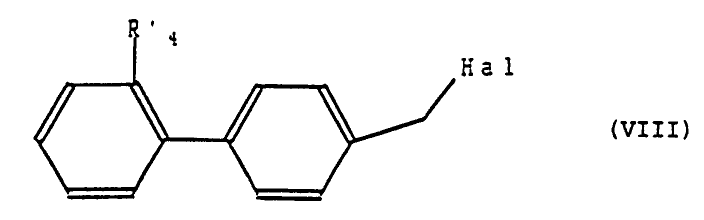 dans laquelle R'₄ a la signification indiquée à la revendication 1 pour R₄, dans laquelle les éventuelles fonctions réactives sont éventuellement protégées par des groupements protecteurs et Hal représente un atome d'halogène pour obtenir un produit de formule (I₁) : 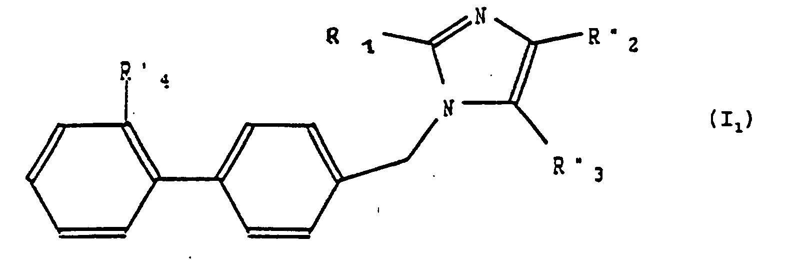 dans laquelle R₁, R"₂, R"₃ et R'₄ ont les significations indiquées ci-dessus,
soit l'on soumet un composé de formule (IX) : 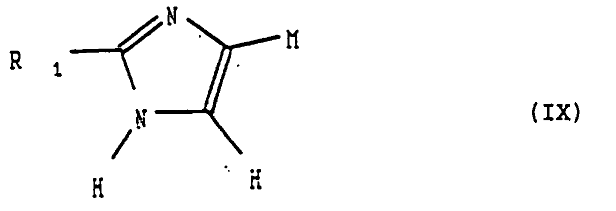 dans laquelle R₁ a la signification indiquée ci-dessus et M représente un atome d'hydrogène ou le radical R'₂ qui a la signification indiquée ci-dessus pour R₂, dans laquelle les éventuelles fonctions réactives sont éventuellement protégées par des groupements protecteurs,
à une réaction avec le composé de formule (VIII) telle que définie ci-dessus, pour obtenir un produit de formule (X) : 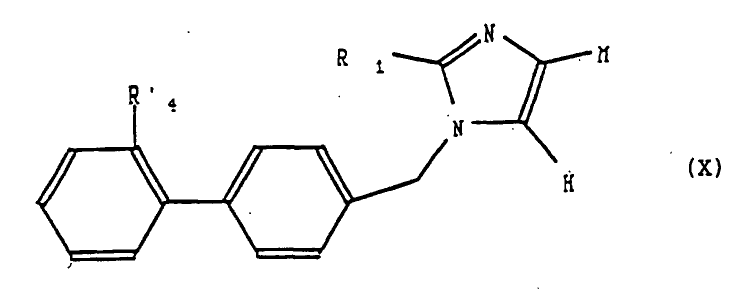 dans laquelle R₁, M et R'4 ont les significations indiquées ci-dessus,
produit de formule (X) que lorsque M représente un atome d'hydrogène, l'on peut soumettre à une réaction d'halogénation pour obtenir le produit de formule (XIV) : 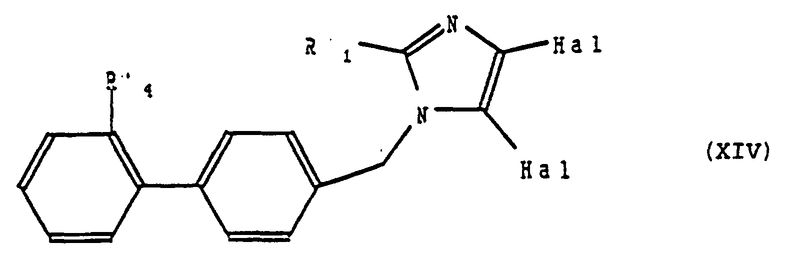 dans laquelle R₁, R'₄ et Hal ont les significations indiquées ci-dessus, que l'on peut soumettre à une réaction d'échange halogène métal puis à l'action d'un composé de formule (IV_{c}), (IV_{d}) ou (IVₑ) telle que définie ci-dessus pour obtenir le produit de formule (XXI): 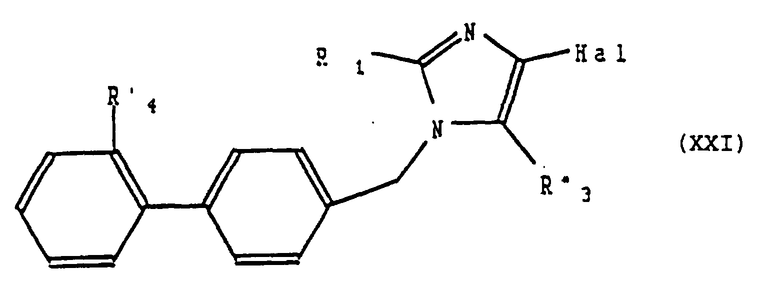 dans laquelle R₁, R'₄, Hal et R"₃ ont les significations indiquées ci-dessus, produit de formule (XXI) que l'on peut soumettre à une réaction d'échange halogène métal puis à l'action d'un composé de formule (IV'ₐ), (IV'_{b}), (IV'_{c}), (IV'_{d}) ou (IV'ₑ) telle que définie ci-dessus, pour obtenir un produit de formule (I₁) : 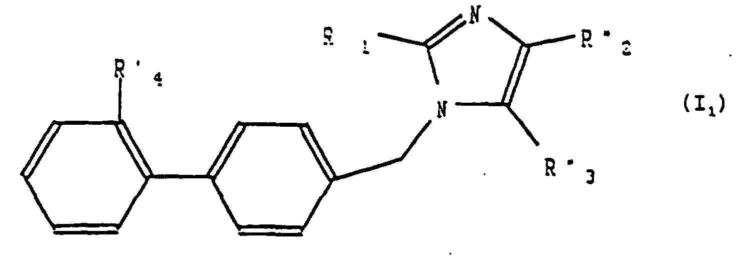 dans laquelle R₁, R'₄, R"₂ et R"₃ ont les significations indiquées ci-dessus,
soit l'on soumet un composé de formule (XX) : 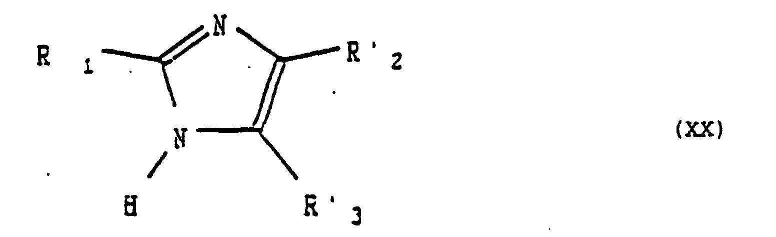 dans laquelle R₁ et R'₂ ont les significations indiquées ci-dessus et R'₃ a la signification indiquée à la revendication 1 pour R₃ dans laquelle les éventuelles fonctions réactives sont éventuellement protégées par des groupements protecteurs, à une réaction avec le composé de formule (VIII) telle que définie ci-dessus, pour obtenir un produit de formule (I') : 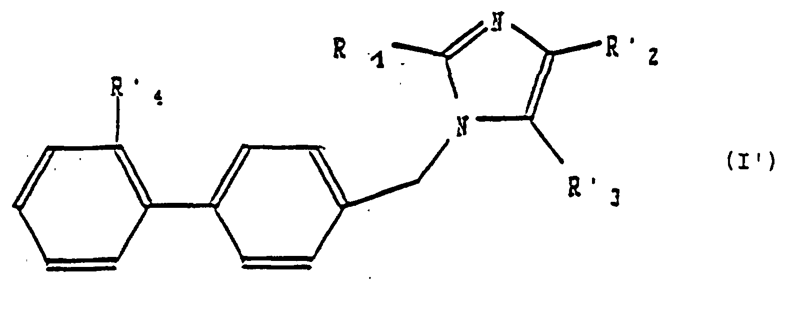 dans laquelle R₁, R'₂, R'₃ et R'₄ ont les significations indiquées ci-dessus,
produits de formules (I₁) et (I') qui peuvent être des produits tel que définis à la revendication 1 et que, pour obtenir des ou d'autres produits tels que définis à la revendication 1, l'on peut soumettre, si désiré et si nécessaire, à l'une ou plusieurs des réactions de transformations suivantes, dans un ordre quelconque :
a) estérification de fonction acide,
b) saponification de fonction ester en fonction acide,
c) transformation de fonction ester en fonction acyle,
d) transformation de la fonction cyano en fonction acide,
e) transformation de fonction acide en fonction amide,
f) réduction de la fonction carboxy en fonction alcool,
g) transformation de fonction alcoxy en fonction hydroxyle, ou encore de fonction hydroxyle en fonction alcoxy,
h) oxydation de fonction alcool en fonction aldéhyde, acide ou cétone,
i) transformation du radical formyle en radical carbamoyle,
j) transformation du radical carbamoyle en radical nitrile,
k) transformation de radical nitrile en tétrazolyle,
l) oxydation de groupement alkylthio ou arylthio en sulfoxyde ou sulfone correspondant,
r) transformation d'un carbamate en urée et notamment d'un sulfonylcarbamate en sulfonylurée,
s) élimination des groupements protecteurs que peuvent porter les fonctions réactives protégées,
t) salification par un acide minéral ou organique ou par une base pour obtenir le sel correspondant,
u) dédoublement des formes racémiques en produits dédoublés, lesdits produits tels que définis à la revendication 1 ainsi obtenus étant sous toutes les formes isomères possibles racémiques, énantiomères et diastéréoisomères.

4. A titre de médicaments, les produits tels que définis à la revendication 1, ces produits étant sous toutes les formes isomères possibles racémiques, énantiomères et diastéréoisomères, ainsi que les sels d'addition avec les acides minéraux et organiques ou avec les bases minérales et organiques pharmaceutiquement acceptables de ces produits.

5. A titre de médicaments, les produits tels que définis à la revendication 2, ainsi que les sels d'addition avec les acides minéraux et organiques ou avec les bases minérales et organiques pharmaceutiquement acceptables de ces produits.

6. Les compositions pharmaceutiques contenant à titre de principe actif, l'un au moins des médicaments tels que définis à l'une quelconque des revendications 4 ou 5.

7. Utilisation des produits tels que définis à la revendication 1 ou 2, pour la préparation de compositions pharmaceutiques destinées au traitement d'affections résultant d'une stimulation anormale des récepteurs AT₁ et AT₂ de l'Angiotensine II.

8. Utilisation des produits tels que définis à la revendication 1 ou 2, pour la préparation de compositions pharmaceutiques destinées au traitement de l'hypertension artérielle, du post-infarctus et de l'insuffisance cardiaque et des resténoses post-angioplastie.

9. Utilisation des produits tels que définis à la revendication 1 ou 2, pour la préparation de compositions pharmaceutiques destinées au traitement de l'insuffisance rénale.

10. Utilisation des produits tels que définis à la revendication 1 ou 2, pour la préparation de compositions pharmaceutiques destinées au traitement et à la prévention des désordres cardiovasculaires et en particulier microcirculatoires associés au diabète.

## Claims

1. Products of formula (I) : 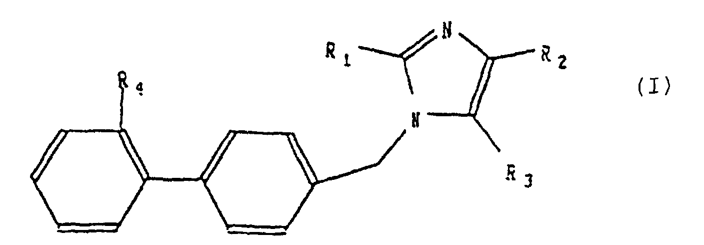 in which
R₁ represents a linear or branched alkyl or alkylthio radical containing at most 4 carbon atoms,
R₂ represents either a linear or branched alkylthio radical containing at most 6 carbon atoms and optionally substituted by one or more halogen atoms, i.e. a 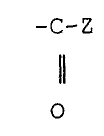 radical
in which Z represents a free carboxy radical, salified by a mineral or organic base or esterified by a linear or branched alkyl radical containing at most 6 carbon atoms,
R₃ represents the 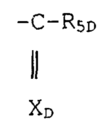 radical in which
X_{D} represents an oxygen atom or a 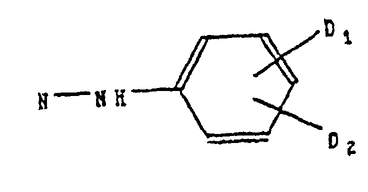 radical in which D₁ and D₂, identical or different, represent a halogen atom or a nitro radical,
and R_{5D} represents
**i)** the -CHY₁Y₂ radical in which
Y₁ represents a hydrogen or halogen atom
and Y₂ represents a free carboxy radical, salified by a mineral or organic base, or esterified by a linear or branched alkyl radical containing at most 6 carbon atoms; the linear or branched alkylthio, alkylsulphonyl and alkylsulphinyl radicals containing at most 6 carbon atoms, or the phenylthio, phenylsulphonyl or phenylsulphinyl radicals optionally substituted by one or more halogen atoms or linear or branched alkyl or alkoxy radicals containing at most 6 carbon atoms,
**ii)** the 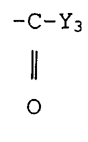 radical
in which Y₃ represents optionally salified, a linear or branched alkoxy or alkylthio hydroxyl radical containing at most 6 carbon atoms,
**iii)** when X_{D} represents an oxygen atom, R_{5D} represents an amino radical optionally substituted by a tetrazolyl radical or by one or two linear or branched alkyl radicals containing at most 6 carbon atoms, themselves optionally substituted by one or more phenyl or cyclohexyl radicals, R₄ represents the -SO₂-NH₂, -SO₂-N=CH-N(CH₃)₂, 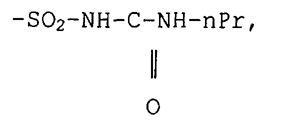 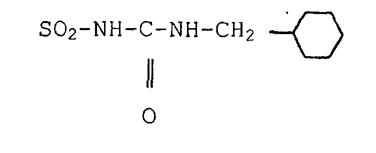 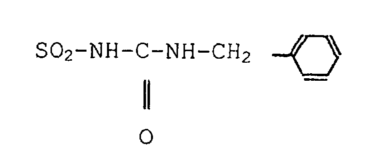 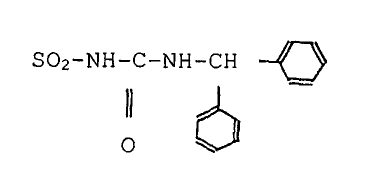 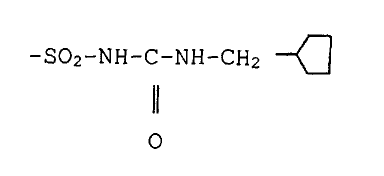 -SO₂-NH-CO₂Et, -SO₂-NH-CO₂H, SO₂-NH-CO₂Pr, 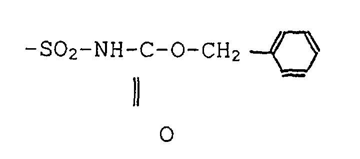 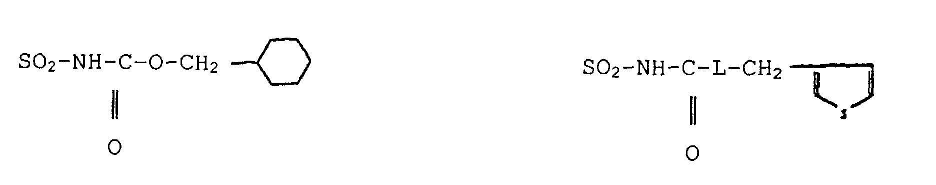 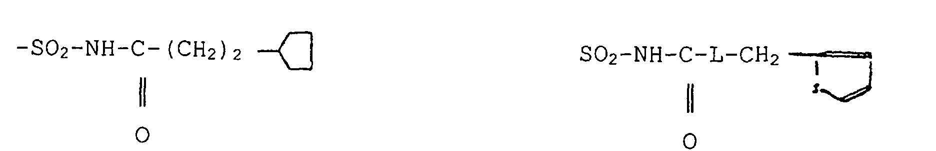 radical, where L represents -O- or -NH- it being understood that the sulphur atoms in the products of formula (I) can optionally be oxidized in the form of sulphonyl or sulphoxide,
said products of formula (I) being in all the possible racemic, enantiomeric and diastereoisomeric isomer forms as well as the addition salts with mineral and organic acids or with mineral and organic bases of said products of formula (I).

2. The products as defined in claim 1, corresponding to the following formulae:
- 2-butyl 1-((2'-(((((cyclohexylmethyl) amino) carbonyl) amino) sulphonyl) (1,1'-biphenyl) 4-yl) methyl) 4-methylthio alpha-oxo-1H-imidazole 5-acetic acid,
- ethyl 2-butyl 4-(methylthio) beta-oxo-1-((2'-(((((propylamino) carbonyl) amino) sulphonyl) (1,1'-biphenyl) 4-yl) methyl) 1H-imidazole 5-propanoate,
- 2-butyl 4-(methylthio) 1-((2'-(((((propylamino) carbonyl) amino) sulphonyl) (1,1'-biphenyl) 4-yl) methyl) N-(1H-tetrazol-5-yl) 1H-imidazole 5-carboxamide,
- 2-butyl 4-(methylthio) alpha-oxo-1-((2'-(((((phenylmethyl) amino) carbonyl) amino) sulphonyl) (1,1'-biphenyl) 4-yl) methyl) 1H-imidazole 5-acetic acid,
- 4'-((2-butyl 5-(2-(methylsulphinyl) acetyl) 4-(methylthio) 1H-imidazol-1-yl) methyl) N-(((phenylmethyl) amino) carbonyl) (1,1'-biphenyl) 2-sulphonamide,
- 4-(methylthio) 1-((2'-(((((phenylmethyl) amino) carbonyl) amino) sulphonyl) (1,1'-biphenyl) 4-yl) methyl) 2-propyl-N-(2H-tetrazol 5-yl) 1H-imidazole 5-carboxamide,
- 4'-((4-(methylthio) 5-(2-(phenylsulphinyl) acetyl) 2-propyl 1H-imidazol-1-yl) methyl N-(((phenylmethyl) amino) carbonyl) (1,1'-biphenyl) 2-sulphonamide,
- ethyl 2-butyl 4-(methylthio) beta oxo 1 ((2' (((((phenylmethyl) amino) carbonyl) amino) sulphonyl) (1,1'-biphenyl) 4-yl) methyl) 1H-imidazol-5-propanoate,
- 4'-((2-butyl 5-(2-((4-fluorophenyl) sulphonyl) acetyl) 4-(methylthio) 1H-imidazol 1-yl) methyl) N-(((phenylmethyl) amino) carbonyl) (1,1'-biphenyl) 2-sulphonamide,
- 4'-((4-(methylthio) 5-((phenylsulphinyl) acetyl) 2-propyl 1H-imidazol-1-yl) methyl) N-(((2-thienylmethyl) amino) carbonyl) (1,1'-biphenyl) 2-sulphonamide,
- (±) N-(((cyclopentylmethyl) amino) carbonyl) 4'-((4-(methylthio) 5-((phenylsulphinyl) acetyl) 2-propyl 1H-imidazol-1-yl) methyl) (1,1'-biphenyl) 2-sulphonamide,
- (±) N- (4'-((4- (methylthio) 5 ((phenylsulphinyl) acetyl) 2-propyl 1H-imidazol 1-yl) methyl) (1,1'-biphenyl-2-yl) sulphonyl) cyclopentanepropanamide.

3. Process for the preparation of the products as defined in claim 1, **characterized in that**:
either a compound of formula (II): 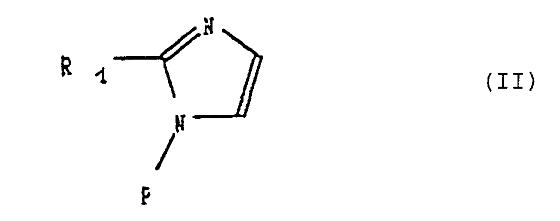 in which R₁ has the meaning indicated in claim 1, and P represents a protective group of the nitrogen atom is subjected,
to a halogenation reaction in order to obtain a compound of formula (III): 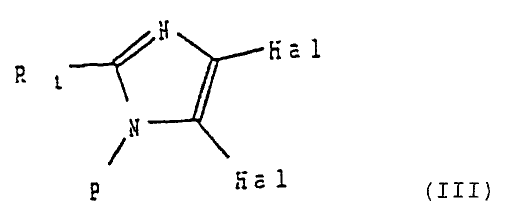 in which R₁ and P have the meanings indicated above and Hal represents a halogen atom, which is subjected to a halogen-metal exchange reaction on one of the halogen atoms then to a reaction with a compound of formula (IV_{c}), (IV_{d}) or (IVₑ): 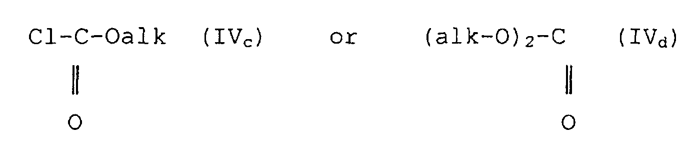 or 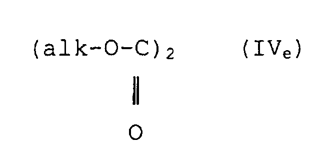 in which alk represents an alkyl radical containing at most 4 carbon atoms,
in order to obtain the compound of formula (V): 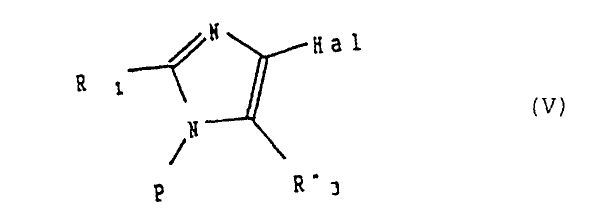 in which R₁, P and Hal have the meanings indicated above and
R"₃ represents K-O-alk with alk as defined above and K represents the 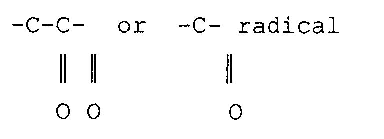 which compound of formula (V) can be subjected to a halogen-metal exchange reaction on the halogen atom then to a reaction with a compound of formula (IV'ₐ), (IV'_{b}), (IV'_{c}), (IV'_{d}) or (IV'ₑ):
(-S-R")₂ (IV'ₐ)
or
MeSO₂SR" (IV'_{b}) 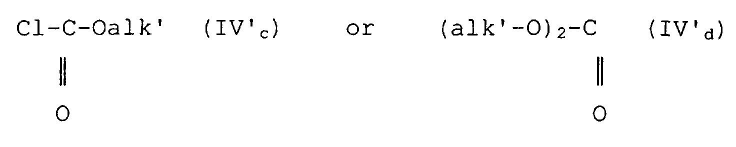
or 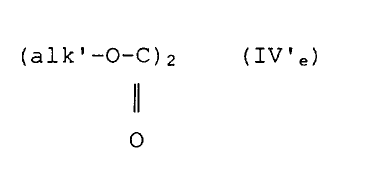 in which R" represents a linear or branched alkyl radical containing at most 6 carbon atoms and alk' identical to or different from alk, represents an alkyl radical containing at most 4 carbon atoms, in order to obtain the compound of formula (VII): 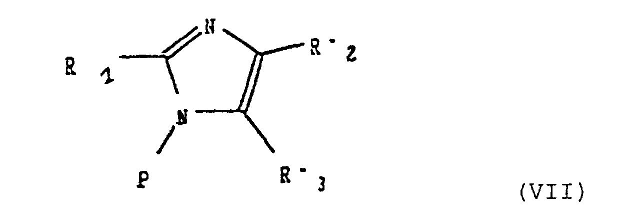 in which R₁, R₃" and P have the meanings indicated above, and
R"₂ represents -S-R" or -K-Oalk' in which R", alk' and K have the meanings indicated above,
product of formula (VII) of which the amine function blocked by P as defined above is liberated, then reacted with a compound of formula (VIII): 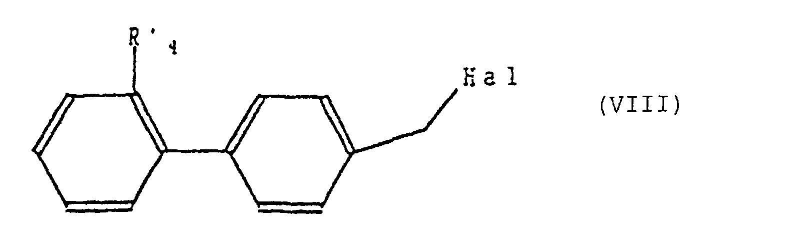 in which R'₄ has the meaning indicated in claim 1 for R₄, in which the optional reactive functions are optionally protected by protective groups and Hal represents a halogen atom in orde: to obtain a product of formula (I₁) : 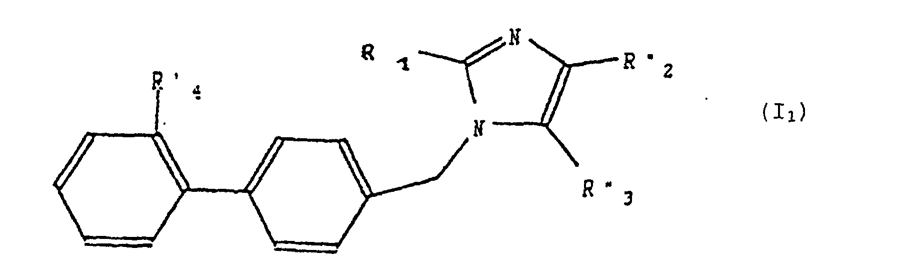 in which R₁, R"₂, R"₃ and R'₄ have the meanings indicated above, or a compound of formula (IX): 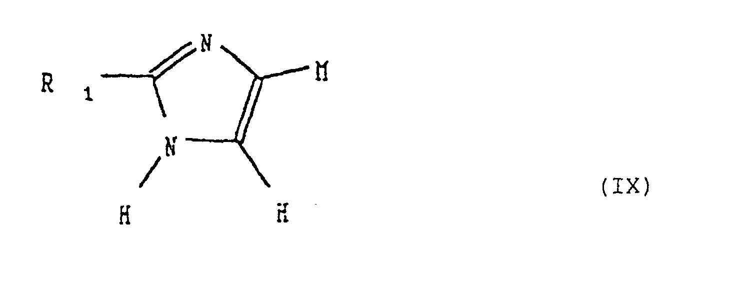 in which R₁ has the meaning indicated above and M represents a hydrogen atom or radical the R'₂ which has the meaning indicated above for R₂, in which the optional reactive functions are optionally protected by protective groups, is subjected to a reaction with the compound of formula (VIII) as defined above, in order to obtain a product of formula (X): 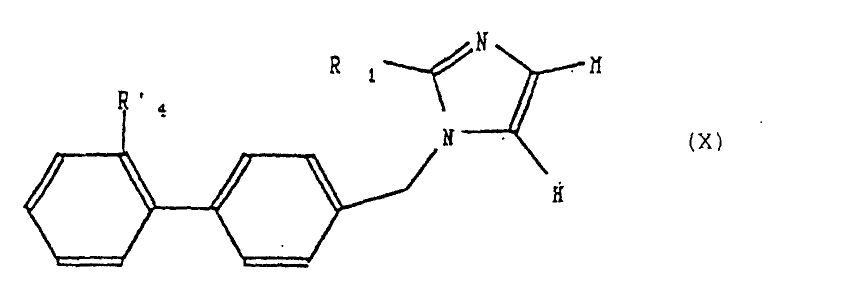 in which R₁, M and R□4 have the meanings indicated above, which product of formula (X) when M represents a hydrogen atom, can be subjected to a halogenation reaction in order to obtain the product of formula (XIV): 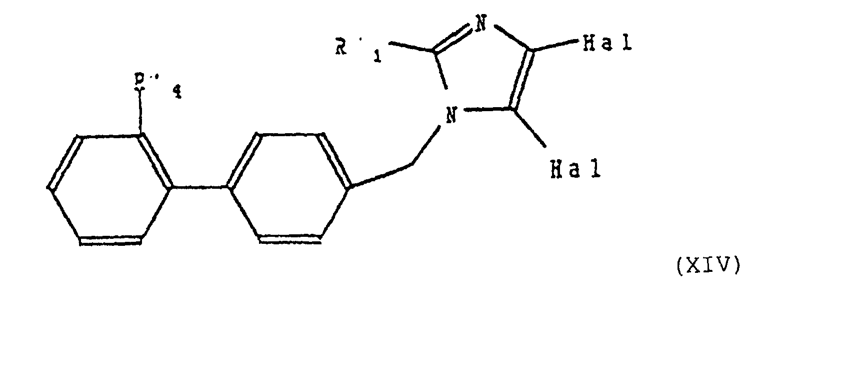 in which R₁, R'₄ and Hal have the meanings indicated above, which can be subjected to a halogen metal exchange reaction, then to the action of a compound of formula (IV_{c}), (IV_{d}) or (IVₑ) as defined above in order to obtain the product of formula (XXI): 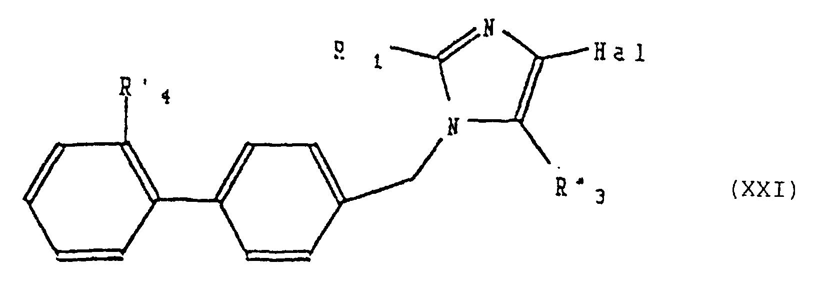 in which R₁, R'₄, Hal and R"₃ have the meanings indicated above, which product of formula (XXI) can be subjected to a halogen metal exchange reaction, then to the action of a compound of formula (IV'ₐ), (IV'_{b}), (IV'_{c}), (IV'_{d}) or (IV'ₑ) as defined above, in order to obtain a product of formula (I₁): 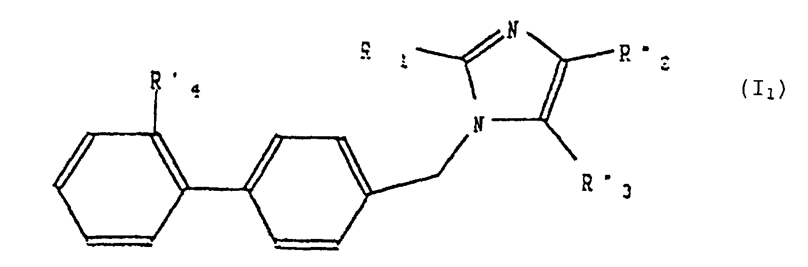 in which R₁, R'₄, R"₂ and R"₃ have the meanings indicated above, or a compound of formula (XX): 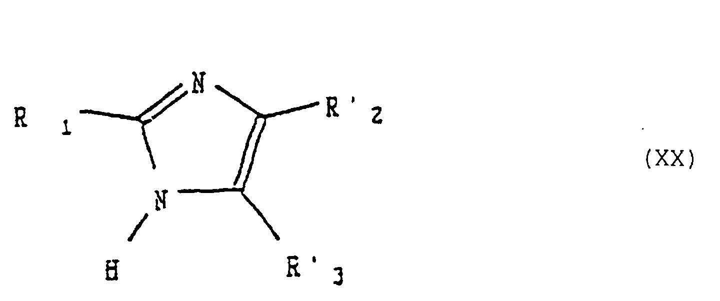 in which R₁ and R'₂ have the meanings indicated above and R'₃ has the meaning indicated in claim 1 for R₃ in which the optional reactive functions are optionally protected by protective groups, is subjected to a reaction with the compound of formula (VIII) as defined above, in order to obtain a product of formula (I'): 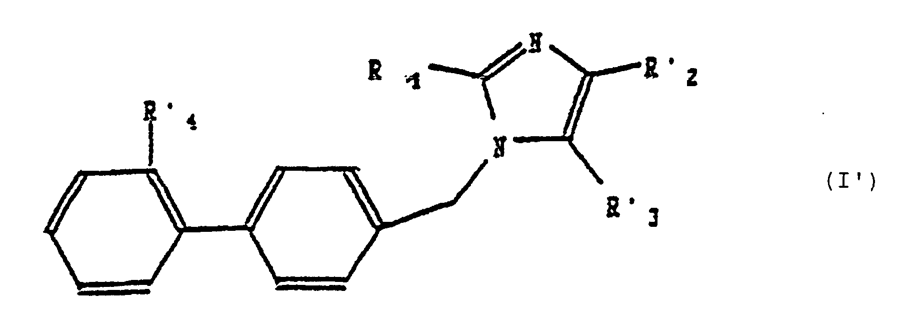 in which R₁, R'₂, R'₃ and R'₄ have the meanings indicated above, which products of formulae (I₁) and (I') can be products as defined in claim 1 and which, in order to obtain products or other products as defined in claim 1, can be subjected, if desired and if necessary, to one or more of the following conversion reactions, in any order:
a) esterification of the acid function,
b) saponification of the ester function to an acid function,
c) conversion of the ester function to an acyl function,
d) conversion of the cyano function to an acid function,
e) conversion of the acid function to an amide function,
f) reduction of the carboxy function to an alcohol function,
g) conversion of the alkoxy function to a hydroxyl function, or also the hydroxyl function to an alkoxy function,
h) oxidization of the alcohol function to an aldehyde, acid or ketone function,
i) conversion of the formyl radical to a carbamoyl radical,
j) conversion of the carbamoyl radical to a nitrile radical,
k) conversion of the nitrile radical to a tetrazolyl,
l) oxidation of the alkylthio or arylthio group to a corresponding sulphoxide or sulphone,
r) conversion of a carbamate to a urea and in particular of a sulphonylcarbamate to a sulphonylurea,
s) elimination of the protective groups which can be carried by the protected reactive functions,
t) salification by a mineral or organic acid or by a base in order to obtain the corresponding salt,
u) resolution of the racemic forms to resolved products,
said products as defined in claim 1 thus obtained being in all possible racemic, enantiomeric and diastereoisomeric isomer forms.

4. As medicaments, the products as defined in claim 1, these products being in all possible racemic, enantiomeric and diastereoisomeric isomer forms, as well as the addition salts with pharmaceutically acceptable mineral and organic acids or mineral and organic bases of these products.

5. As medicaments, the products as defined in claim 2, as well as the addition salts with pharmaceutically acceptable mineral and organic acids or mineral and organic bases of these products.

6. The pharmaceutical compositions containing as active ingredient, at least one of the medicaments as defined in any one of claims 4 or 5.

7. Use of products as defined in claim 1 or 2, for the preparation of pharmaceutical compositions intended for the treatment of illnesses resulting from an abnormal stimulation of the AT₁ and AT₂ receptors of Angiotensin II.

8. Use of products as defined in claim 1 or 2, for the preparation of pharmaceutical compositions intended for the treatment of arterial hypertension, post-infarctus and cardiac insufficiancy and post-angioplasty recurrence of stenosis.

9. Use of products as defined in claim 1 or 2, for the preparation of pharmaceutical compositions intended for the treatment of renal insufficiency.

10. Use of products as defined in claim 1 or 2, for the preparation of pharmaceutical compositions intended for the treatment and the prevention of cardiovascular and in particular microcirculatory disorders associated with diabetes.

## Patentansprüche

1. Produkte der Formel (I): 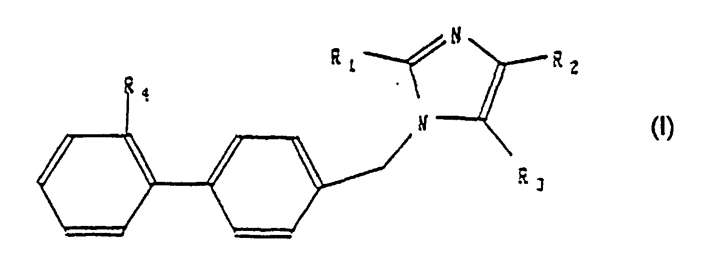 worin
R₁ einen linearen oder verzweigten Alkyl- oder Alkylthiorest mit höchstens 4 Kohlenstoffatomen darstellt,
R₂ entweder darstellt einen linearen oder verzweigten Alkylthiorest mit höchstens 6 Kohlenstoffatomen, gegebenenfalls substituiert mit einem oder mehreren Halogenatomen, oder einen Rest 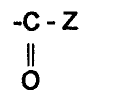 worin Z darstellt einen Carboxyrest in freier Form, in Form eines Salzes mit einer mineralischen oder organischen Base oder in Form eines Esters mit einem linearen oder verzweigten Alkylrest mit höchstens 6 Kohlenstoffatomen,
R₃ den Rest 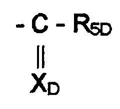 darstellt, worin
X_{D} darstellt ein Sauerstoffatom oder einen Rest 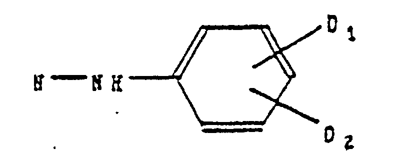 worin D₁ und D₂, die gleich oder verschieden sind, ein Halogenatom oder einen Nitrorest darstellen,
und R_{5D} darstellt
i) den Rest -CHY₁Y₂, worin
Y₁ ein Wasserstoff- oder Halogenatom darstellt
und Y₂ darstellt einen Carboxyrest in freier Form, in Form eines Salzes mit einer mineralischen oder organischen Base oder in Form eines Esters mit einem linearen oder verzweigten Alkylrest mit höchstens 6 Kohlenstoffatomen;
die linearen oder verzweigten Alkylthio-, Alkylsulfonyl- und Alkylsulfinylreste mit höchstens 6 Kohlenstoffatomen,
oder die Phenylthio-, Phenylsulfonyl- oder Phenylsulfinylreste, gegebenenfalls substituiert mit einem oder mehreren Halogenatomen oder linearen oder verzweigten Alkyl- oder Alkoxyresten mit höchstens 6 Kohlenstoffatomen,
ii) den Rest 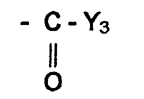 worin Y₃ einen Hydroxyrest, gegebenenfalls in Salzform, einen linearen oder verzweigten Alkoxy- oder Alkylthiorest mit höchstens 6 Kohlenstoffatomen darstellt,
iii) wenn X_{D} ein Sauerstoffatom darstellt, stellt R_{5D} einen Aminorest dar, gegebenenfalls substituiert mit einem Tetrazolylrest oder mit einem oder zwei linearen oder verzweigten Alkylresten mit höchstens 6 Kohlenstoffatomen, selbst gegebenenfalls substituiert mit einem oder mehreren Phenyl- oder Cyclohexylresten,
R₄ darstellt den Rest -SO₂-NH₂,
-SO₂-N=CH-N(CH₃)₂, 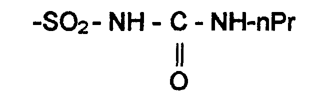 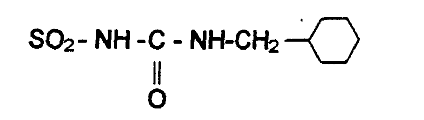 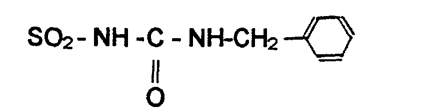 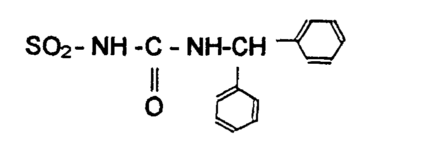 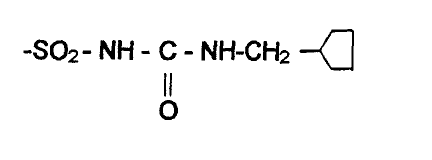 -SO₂-NH-CO₂Et, -SO₂-NH-CO₂H, SO₂-NH-CO₂Pr, 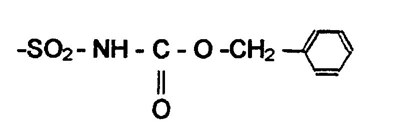 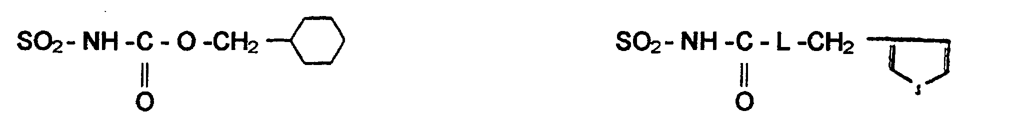 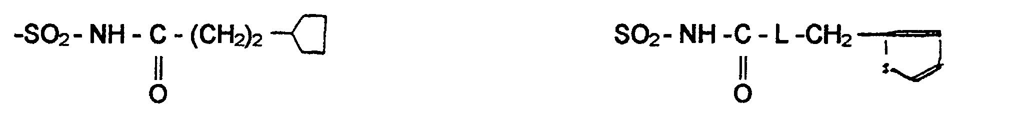 mit L, das -O- oder -NH- darstellt,
wobei es sich versteht, dass die Schwefelatome in den Produkten der Formel (I) gegebenenfalls in Form von Sulfon oder Sulfoxid oxidiert sein können,
wobei besagte Produkte der Formel (I) in allen möglichen racemischen, enantiomeren und diastereoisomeren Isomerformen vorliegen, sowie die Additionssalze besagter Produkte der Formel (I) mit den mineralischen und organischen Säuren oder mit den mineralischen oder organischen Basen.

2. Die Produkte, wie in Anspruch 1 definiert, die den folgenden Formeln entsprechen:
- 2-Butyl-1-((2'-(((((Cyclohexylmethyl)amino)carbonyl)amino)sulfonyl)(1,1'biphenyl)-4-yl)methyl)-4-methylthio-alpha-oxo-1H-imidazol-5-essigsäure,
- 2-Butyl-4-(methylthio)-beta-oxo-1-((2'-(((((propylamino)carbonyl)amino)sulfonyl)-(1,1'-biphenyl)-4-yl)methyl)-1H-imidazol-5-propionsäure-ethylester,
- 2-Butyl-4-(methylthio)-1-((2'-(((((propylamino)carbonyl)amino)sulfonyl)(1,1'biphenyl)-4-yl)methyl)-N-(1H-tetrazol-5-yl)-1H-imidazol-5-carboxamid,
- 2-Butyl-4-(methylthio)-alpha-oxo-1-((2'-(((((phenylmethyl)amino)carbonyl)amino)-sulfonyl)(1,1'-biphenyl)-4-yl)methyl)-1H-imidazol-5-essigsäure,
- 4'-((2-Butyl-5-(2-(methylsulfinyl)acetyl)-4-(methylthio)-1H-imdidazol-1-yl)methyl)-N-(((phenylmethyl)amino)carbonyl)(1,1'-biphenyl)-2-sulfonamid,
- 4-(Methylthio)-1-((2'-(((((phenylmethyl)amino)carbonyl)amino)sulfonyl)(1,1'biphenyl)-4-yl)methyl)-2-propyl-N-(2H-tetrazol-5-yl)-1H-imidazol-5-carboxamid,
- 4'-((4-(Methylthio)-5-(2-(phenylsulfinyl)acetyl)-2-propyl-1H-imidazol-1-yl)methyl-N-(((phenylmethyl)amino)carbonyl)(1,1'-biphenyl)-2-sulfonamid,
- 2-Butyl-4-(methylthio)-beta-oxo-1-((2'-(((((phenylmethyl)amino)carbonyl)amino)-sulfonyl)(1,1'-biphenyl)-4-yl)methyl)-1H-imidazol-5-propionsäure-ethylester,
- 4'-((2-Butyl-5-(2-((4-fluorphenyl)sulfonyl)acetyl)-4-(methylthio)-1H-imidazol-1-yl)methyl)-N-(((phenylmethyl)amino)carbonyl)(1,1'-biphenyl)-2-sulfonamid,
- 4'-((4-(Methylthio)-5-((phenylsulfinyl)acetyl)-2-propyl-1H-imidazol-1-yl)methyl)-N-(((2-thienylmethyl)amino)carbonyl)(1,1'-biphenyl)-2-sulfonamid,
- (±)-N-(((Cyclopentylmethyl)amino)carbonyl-4'-((4-(methylthio)-5-((phenylsulfinyl)-acetyl)-2-propyl-1H-imidazol-1-yl)methyl)(1,1'-biphenyl)-2-sulfonamid,
- (±)-N-(4'-((4-(Methylthio)-5-((phenylsulfinyl)acetyl)-2-propyl-1H-imidazol-1-yl)methyl)(1,1'-biphenyl-2-yl)sulfonyl)cyclopentanpropanamid.

3. Verfahren zur Herstellung der Produkte, wie in Anspruch 1 definiert, **dadurch gekennzeichnet, dass**:
man entweder eine Verbindung der Formel (II): 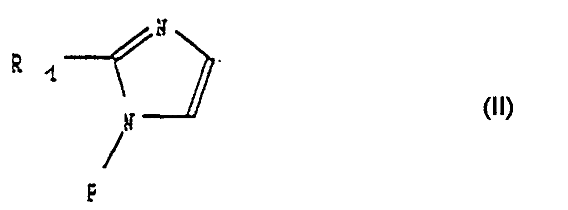 worin R₁ die in Anspruch 1 angegebene Bedeutung hat und P eine Schutzgruppe für das Stickstoffatom darstellt,
einer Halogenierungsreaktion unterzieht, um eine Verbindung der Formel (III) zu erhalten: 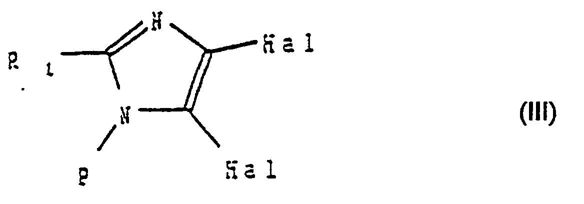 worin R₁ und P die oben angegebenen Bedeutungen haben und Hal ein Halogenatom darstellt, die man einer Halogen-Metall-Austauschreaktion an einem der Halogenatome, dann einer Reaktion mit einer Verbindung der Formel (IV_{c}), (IV_{d}) oder (IVₑ) unterzieht: 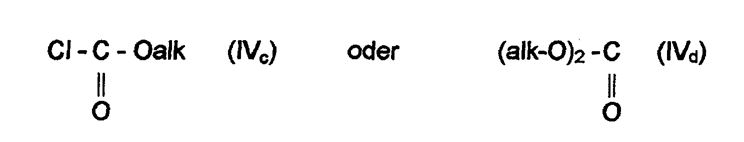 oder 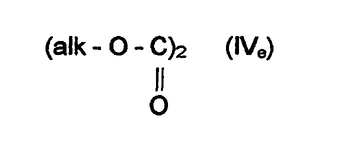 worin alk einen Alkylrest mit höchstens 4 Kohlenstoffatomen darstellt,
um die Verbindung der Formel (V) zu erhalten: 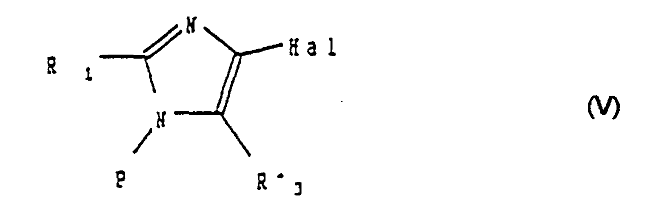 worin R₁, P und Hal die oben angegebenen Bedeutungen haben und R"₃ K-O-alk darstellt mit alk wie oben definiert und K stellt den Rest 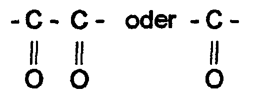 dar, Verbindung der Formel (V), die man einer Halogen-Metall-Austauschreaktion an dem Halogenatom, dann einer Reaktion mit einer Verbindung der Formel (IV'ₐ), (IV'_{b}), (IV'_{c}), (IV'_{d}) oder (IV'ₑ) unterziehen kann:
(-S-R")₂ (IV'ₐ)
oder
MeSO₂SR" (IV'_{b}) 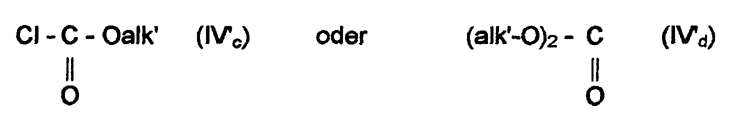
oder 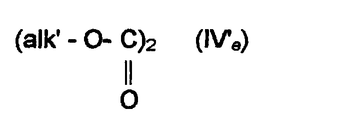 worin R" einen linearen oder verzweigten Alkylrest mit höchstens 6 Kohlenstoffatomen darstellt und alk', gleich oder verschieden von alk, einen Alkylrest mit höchstens 4 Kohlenstoffatomen darstellt, um die Verbindung der Formel (VII) zu erhalten: 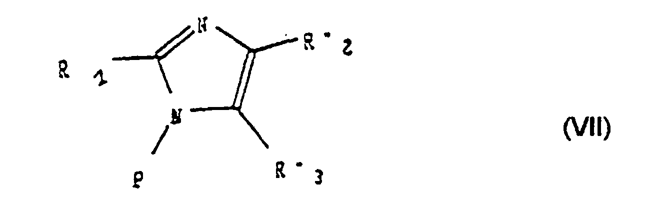 worin R₁, R"₃ und P die oben angegebenen Bedeutungen haben und R"₂ -S-R" oder -K-Oalk' darstellt, worin R", alk' und K die oben angegebenen Bedeutungen haben, Produkt der Formel (VII), bei dem man die durch P, wie oben definiert, blockierte Aminfunktion freisetzt, dann mit einer Verbindung der Formel (VIII) umsetzt: 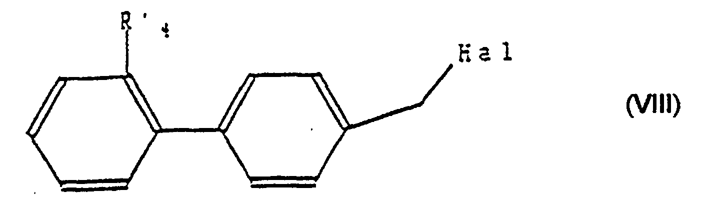 worin R'₄ die in Anspruch 1 für R₄ angegebene Bedeutung hat, worin die eventuellen reaktiven Funktionen gegebenenfalls durch Schutzgruppen geschützt sind und Hal ein Halogenatom darstellt, um ein Produkt der Formel (I₁) zu erhalten: 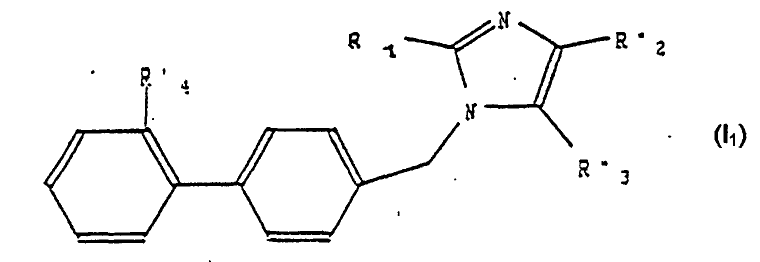 worin R₁, R"₂, R"₃ und R'₄ die oben angegebenen Bedeutungen haben,
oder man eine Verbindung der Formel (IX): 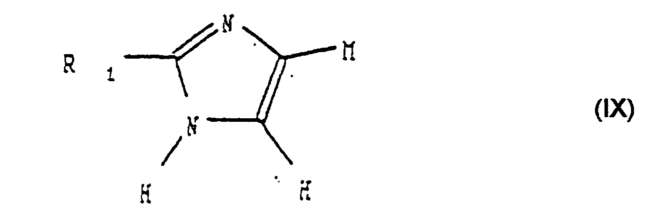 worin R₁ die oben angegebene Bedeutung hat und M ein Wasserstoffatom oder den Rest R'₂ darstellt, der die oben für R₂ angegebene Bedeutung hat, worin die eventuellen reaktiven Funktionen gegebenenfalls durch Schutzgruppen geschützt sind,
einer Reaktion mit der Verbindung der Formel (VIII), wie oben definiert, unterzieht, um ein Produkt der Formel (X) zu erhalten: 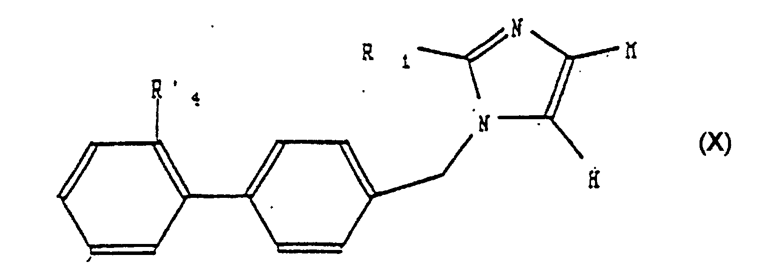 worin R₁, M und R'₄ die oben angegebenen Bedeutungen haben,
Produkt der Formel (X), das man, wenn M ein Wasserstoffatom darstellt, einer Halogenierungsreaktion unterziehen kann, um das Produkt der Formel (XIV) zu erhalten: 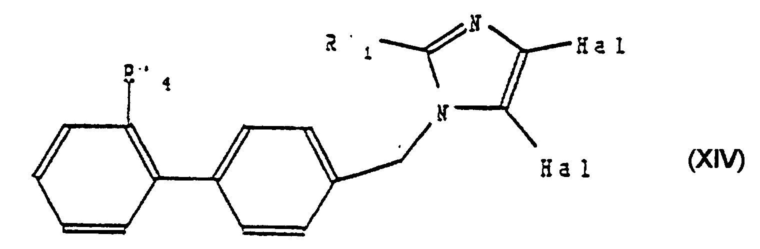 worin R₁, R'₄ und Hal die oben angegebenen Bedeutungen haben, das man einer Halogen-Metall-Austauschreaktion, dann der Einwirkung einer Verbindung der Formel (IV_{c}), (IV_{d}) oder (IVₑ), wie oben definiert, unterziehen kann, um das Produkt der Formel (XXI) zu erhalten: 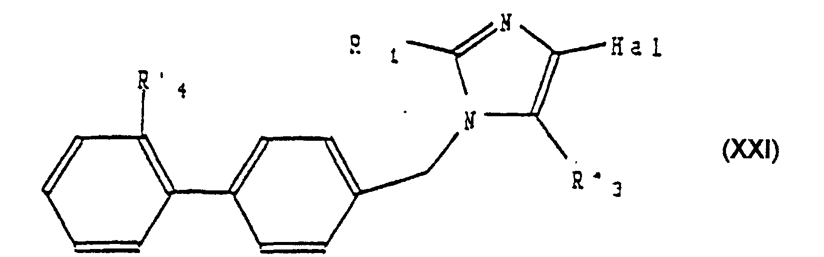 worin R₁, R'₄, Hal und R"₃ die oben angegebenen Bedeutungen haben, Produkt der Formel (XXI), das man einer Halogen-Metall-Austauschreaktion, dann der Einwirkung einer Verbindung der Formel (IV'ₐ), (IV'_{b}), (IV'_{c}), (IV'_{d}) oder (IV'ₑ), wie oben definiert, unterziehen kann, um ein Produkt der Formel (I₁) zu erhalten: 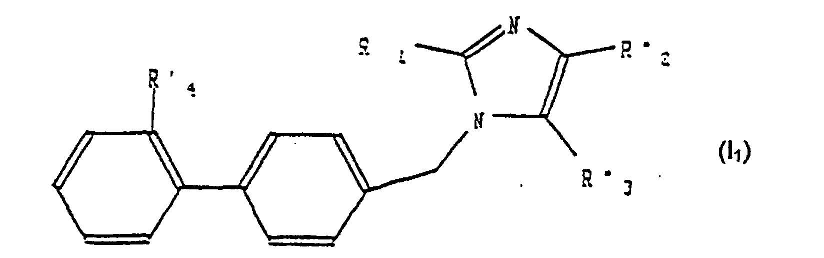 worin R₁, R'₄, R"₂ und R"₃ die oben angegebenen Bedeutungen haben,
oder man eine Verbindung der Formel (XX): 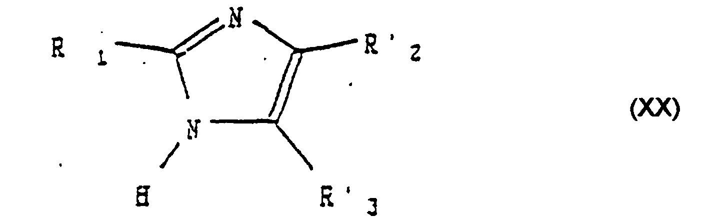 worin R₁ und R'₂ die oben angegebenen Bedeutungen haben und R'₃ die in Anspruch 1 für R₃ angegebene Bedeutung hat, worin die eventuellen reaktiven Funktionen gegebenenfalls durch Schutzgruppen geschützt sind, einer Reaktion mit der Verbindung der Formel (VIII), wie oben definiert, unterzieht, um ein Produkt der Formel (I') zu erhalten: 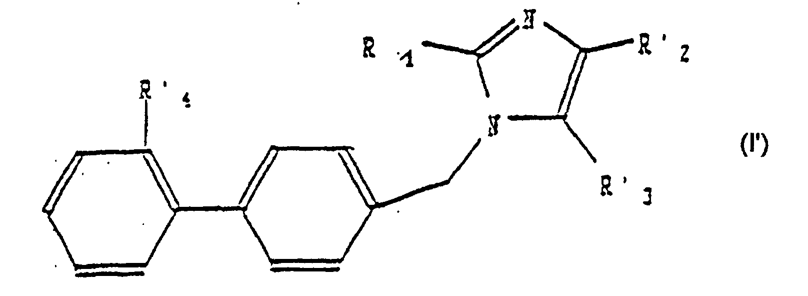 worin R₁, R'₂, R'₃ und R'₄ die oben angegebenen Bedeutungen haben,
Produkte der Formeln (I₁) und (I'), die Produkte, wie in Anspruch 1 definiert, sein können, und die man, um Produkte, wie in Anspruch 1 definiert, oder andere zu erhalten, wenn gewünscht und wenn notwendig, einer oder mehreren der folgenden Unwandlungsreaktion in einer beliebigen Reihenfolge unterziehen kann:
a) Veresterung einer Säurefunktion,
b) Verseifung einer Esterfunktion zu einer Säurefunktion,
c) Umwandlung einer Esterfunktion in eine Acylfunktion,
d) Umwandlung der Cyanofunktion in eine Säurefunktion,
e) Umwandlung einer Säurefunktion in eine Amidfunktion,
f) Reduktion der Carboxyfunktion zu einer Alkoholfunktion,
g) Umwandlung einer Alkoxyfunktion in eine Hydroxylfunktion oder auch einer Hydroxylfunktion in eine Alkoxyfunktion,
h) Oxidation einer Alkoholfunktion zu einer Aldehyd-, Säure- oder Ketonfunktion,
i) Umwandlung des Formylrests in einen Carbamoylrest,
j) Umwandlung des Carbamoylrests in einen Nitrilrest,
k) Umwandlung eines Nitrilrests in Tetrazolyl,
l) Oxidation einer Alkylthio- oder Arylthiogruppe zu einem entsprechenden Sulfoxid oder Sulfon,
r) Umwandlung eines Carbamats in einen Hamstoff und insbesondere eines Sulfonylcarbamats in einen Sulfonylharnstoff,
s) Entfernung der Schutzgruppen, die von den geschützten reaktiven Funktionen getragen werden können,
t) Salzbildung durch eine mineralische oder organische Säure oder durch eine Base, um das entsprechende Salz zu erhalten,
u) Spaltung der racemischen Formen zu aufgetrennten Produkten,
wobei die so erhaltenen besagten Produkte, wie in Anspruch 1 definiert, in allen möglichen racemischen, enantiomeren und diastereoisomeren Isomerformen vorliegen.

4. Als Medikamente die Produkte, wie in Anspruch 1 definiert, wobei diese Produkte in allen möglichen racemischen, enantiomeren und diastereoisomeren Isomerformen vorliegen, sowie die Additionssalze dieser Produkte mit den mineralischen und organischen Säuren oder mit den mineralischen und organischen Basen, die pharmazeutisch annehmbar sind.

5. Als Medikamente die Produkte, wie in Anspruch 2 definiert, sowie die Additionssalze dieser Produkte mit den mineralischen und organischen Säuren oder mit den mineralischen und organischen Basen, die pharmazeutisch annehmbar sind.

6. Die pharmazeutischen Zusammensetzungen, die als Wirkstoff wenigstens eins der Medikamente, wie in einem der Ansprüche 4 oder 5 definiert, enthalten.

7. Verwendung der Produkte, wie in Anspruch 1 oder 2 definiert, für die Herstellung von pharmazeutischen Zusammensetzungen, die für die Behandlung von Erkrankungen bestimmt sind, die sich aus einer anormalen Stimulation der AT₁- und AT₂-Rezeptoren von Angiotensin II ergeben.

8. Verwendung der Produkte, wie in Anspruch 1 oder 2 definiert, für die Herstellung von pharmazeutischen Zusammensetzungen, die für die Behandlung des arteriellen Bluthochdrucks, des Postinfarkts und der Herzinsuffizienz und der wiederkehrenden Verengungen nach Angioplastie bestimmt sind.

9. Verwendung der Produkte, wie in Anspruch 1 oder 2 definiert, für die Herstellung von pharmazeutischen Zusammensetzungen, die für die Behandlung der Niereninsuffizienz bestimmt sind.

10. Verwendung der Produkte, wie in Anspruch 1 oder 2 definiert, für die Herstellung von pharmazeutischen Zusammensetzungen, die für die Behandlung und Vorbeugung von kardiovaskulären Störungen und insbesondere von Mikrozirkulationsstörungen, die mit dem Diabetes verbunden sind, bestimmt sind.
